# EUROPEAN PATENT APPLICATION

(11) **EP 4 834 934 A1**
(43) Date of publication of application: **04.11.2026**
(21) Application number: 24911220.2
(22) Date of filing: 25.12.2024
(51) Int. Cl.: C07K 16/28, A61K 39/395, A61K 47/68, A61P 37/00, A61P 35/00

(54) **ANTI-DLL3 ANTIBODY OR ANTIGEN BINDING FRAGMENT THEREOF, AND USE THEREOF**

(30) Priority: 26.12.2023 CN 202311817477
(71) Applicant: Shanghai Hongcheng Biopharmaceutical Co., Ltd., Shanghai 201315 (CN)
(72) Inventor: XI, Yue, Shanghai 201315 (CN); MIN, Hao, Shanghai 201315 (CN); XU, Jun, Shanghai 201315 (CN); WANG, Yonghui, Shanghai 201315 (CN); WANG, Xingding, Shanghai 201315 (CN); XIAO, Changhui, Shanghai 201315 (CN); HUANG, Fanjuan, Shanghai 201315 (CN)
(74) Representative: Dehns
(86) International application number: PCT/CN2024/142258
(87) International publication number: WO 2025/140296

(57) **Abstract**

The present invention relates to an anti-DLL3 antibody or an antigen-binding fragment thereof, and the use thereof. The anti-DLL3 antibody or antigen-binding fragment thereof provided by the present invention has high affinity for DLL3, and an ADC drug obtained via conjugation exhibits a good anti-tumor therapeutic effect.

## Description

### TECHNICAL FIELD

The present disclosure pertains to the technical field of biomedicine and relates to an anti-DLL3 antibody or an antigen-binding fragment thereof and use thereof. The present disclosure also relates to a nucleic acid molecule encoding the anti-DLL3 antibody or the antigen-binding fragment thereof, a vector comprising the nucleic acid molecule, and a host cell; and a pharmaceutical composition and an antibody-drug conjugate comprising the anti-DLL3 antibody or the antigen-binding fragment thereof. The present disclosure further relates to therapeutic use of the anti-DLL3 antibody or the antigen-binding fragment thereof, as well as the pharmaceutical composition and the antibody-drug conjugate comprising the same.

### BACKGROUND

Small cell lung cancer (SCLC) accounts for about 15% of lung cancers, with more than 240,000 new patients diagnosed annually in China. SCLC is a tumor caused by the combined action of multiple factors, and its pathogenesis is relatively complex. The most significant difference between SCLC and NSCLC lies in the neuroendocrine nature of SCLC cells; that is, tumor cells can receive neuronal signals and secrete hormones into the blood. SCLC is classified into limited stage (LS, without distant metastasis) and extensive stage (ES, with distant metastasis). Among them, ES-SCLC accounts for 2/3 of all cases, with a 5-year survival rate of less than 2%, far lower than the 31% 5-year survival rate of LS-SCLC. Radiotherapy and chemotherapy remain the mainstay of SCLC treatment, with limited alternative drugs available. Although SCLC is highly sensitive to initial treatment, most patients experience recurrence and metastasis due to drug resistance. Therefore, it is particularly critical to actively explore second-line and even third-line treatments for SCLC. Over the past three decades, the life expectancy of patients with small cell lung cancer has not improved, rendering small cell lung cancer defined as an intractable cancer (PMID: 31383005, J Immunother Cancer. 2019 Aug 5; 7(1):205). In recent years, novel immune checkpoint inhibitors (anti-PD-1/PD-L1 antibodies) have been approved by the FDA for SCLC treatment. Nevertheless, certain immune checkpoint inhibitors, such as nivolumab, have failed to demonstrate advantages over chemotherapeutic drugs (PMID: 33539946, Ann Oncol. 2021 May; 32(5):631-641). The therapeutic effect of immune checkpoint inhibitors is largely dependent on immune cell infiltration and the expression levels of PD-L1. Immune checkpoint inhibitors show relatively low efficacy in SCLC; the objective response rate of nivolumab in the second-line treatment of recurrent SCLC is only 13.7% (PMID: 33539946, Ann Oncol. 2021 May; 32(5):631-641; PMID: 33065386, Transl Oncol. 2021 Jan; 14(1):100889). Therefore, it is of great importance to actively explore novel broad-spectrum therapeutic approaches for SCLC.

Delta-like protein ligand 3 (DLL3) is a single-pass transmembrane glycoprotein and a member of the DSL (delta, serrate, lag-2) protein family. It acts as an inhibitory Notch ligand, and its expression is directly correlated with ASCL1, a key neuroendocrine transcription factor. DLL3 is highly expressed in SCLC, while exhibiting low or no expression in normal tissues. In normal tissues, DLL3 is only expressed intracellularly in brain tissue cells rather than on the cell surface (PMID: 33203642, Clin Cancer Res. 2021 Mar 1; 27(5):1526-1537). DLL3 is expressed in 85% of SCLC cases (≥25% DLL3-positive cells), and high-level DLL3 expression is observed in 68% of SCLC cases (≥75% DLL3-positive cells). Accordingly, DLL3 is expected to serve as a novel biomarker and a potential target for small cell lung cancer, and DLL3-targeted therapy may be effective for the majority of SCLC cases (PMID: 26311731, Sci Transl Med. 2015 Aug 26; 7(302):302ra136). Besides SCLC, DLL3 is also highly expressed in other neuroendocrine tumors, such as 76.7% of castration-resistant neuroendocrine prostate cancer, 89% of Merkel cell carcinoma, and 81% of neuroendocrine cervical carcinoma (PMID: 30894499, Sci Transl Med. 2019 Mar 20; 11(484):eaav0891; PMID: 32372416, Oncologist. 2020 Sep; 25(9):810-817; PMID: 33208671, Appl Immunohistochem Mol Morphol. 2021 Apr 1; 29(4):299-304).

All globally investigational drugs targeting the DLL3 target are macromolecular drugs, including one phase III candidate: the DLL3-pyrrolobenzodiazepine (PBD) ADC (Rova-T, AbbVie), the project of which was terminated in 2020. In the phase III TAHOE trial of Rova-T for second-line SCLC treatment, compared with topotecan, the overall survival (OS) of the Rova-T group was inferior to that of the chemotherapeutic drug control group (topotecan group). Rova-T employs highly toxic PBD as a toxin, which limits the administration dose of Rova-T in clinical trials (0.3 mg/kg). Moreover, the clinical toxicity profile of Rova-T is similar to that of other ADCs using PBD as a toxin. This is presumed to be associated with premature linker cleavage in Rova-T, early toxin release prior to internalization, and toxin diffusion from tumor cells to normal cells (PMID: 33607312, J Thorac Oncol. 2021 Sep; 16(9):1547-1558). Two additional drugs targeting DLL3 are currently in phase I clinical trials, including one CAR-T and one CD3×DLL3 bispecific antibody (AMG 757). AMG 757 is developed based on the next-generation bispecific antibody platform HLE BiTE. Structurally, it is constructed by fusing an Fc to tandem scFvs in a BiTE, and the Fc adopts a head-to-tail single-chain structure. AMG 757, used for the second-line treatment of SCLC, is currently in the phase I dose-escalation stage, demonstrating relatively good safety and exhibiting some therapeutic effects (ORR of 16%). In summary, DLL3 is highly expressed in neuroendocrine tumors such as SCLC, and DLL3-targeted drugs demonstrate safety and exhibit some therapeutic effects.

At present, no relevant research on the DLL3 target has been publicly disclosed in China. Therefore, the development of next-generation anti-DLL3 antibodies possesses important scientific and application values.

### SUMMARY OF THE INVENTION

The technical problem to be solved in the present disclosure is to obtain antibodies that specifically bind to DLL3, in particular, antibodies with high affinity for human DLL3, by means of hybridoma screening and humanization technology.

In view of the above technical problem, an objective of the present disclosure is to provide an anti-DLL3 antibody or an antigen-binding fragment thereof that binds to DLL3, and provide use thereof based on the antibody or the fragment thereof. The "fragment" of the antibody molecule described in the present disclosure encompasses various functional fragments of the antibody, such as antigen-binding moieties thereof, e.g., Fab, F(ab')₂, and scFv fragments.

The present disclosure provides the following technical solutions.

In a first aspect, the present disclosure provides a DLL3-targeted antibody or an antigen-binding fragment thereof.

The present disclosure provides a murine anti-DLL3 antibody, a chimeric antibody, and a humanized anti-DLL3 antibody that specifically bind to DLL3. It is expected that the anti-DLL3 antibodies of the present disclosure have low immunogenicity in human subjects, and human subjects have good tolerance to the anti-DLL3 antibodies.

In the present disclosure, mice are immunized with the DLL3-Fc protein; anti-DLL3 specific antibodies screened by hybridoma technology possess higher internalization efficiency and high affinity for both human and cynomolgus monkey DLL3, and ADC drugs obtained via conjugation exhibit good anti-tumor efficacy.

In some embodiments, the present disclosure provides an anti-DLL3 antibody or an antigen-binding fragment thereof that binds to DLL3, which comprises:
1) 3 heavy chain CDRs (HCDR1, HCDR2, and HCDR3) comprised in a heavy chain variable region as shown in SEQ ID NO: 7 and/or 3 light chain CDRs (LCDR1, LCDR2, and LCDR3) comprised in a light chain variable region as shown in SEQ ID NO: 8;
2) 3 heavy chain CDRs (HCDR1, HCDR2, and HCDR3) comprised in a heavy chain variable region as shown in SEQ ID NO: 21 and/or 3 light chain CDRs (LCDR1, LCDR2, and LCDR3) comprised in a light chain variable region as shown in SEQ ID NO: 22;
3) 3 heavy chain CDRs (HCDR1, HCDR2, and HCDR3) comprised in a heavy chain variable region as shown in SEQ ID NO: 35 and/or 3 light chain CDRs (LCDR1, LCDR2, and LCDR3) comprised in a light chain variable region as shown in SEQ ID NO: 36.

Based on the amino acid sequences of the variable regions comprised in the antibodies or fragments thereof provided in the present disclosure, those skilled in the art can routinely identify the CDRs comprised therein. For example, according to the specific embodiments of the present disclosure, the CDRs in the amino acid sequences of the variable regions are defined using the Kabat scheme, the AbM scheme, the Chothia scheme, or the Contact scheme.

In some embodiments, the present disclosure provides an anti-DLL3 antibody or an antigen-binding fragment thereof that binds to DLL3, which comprises heavy chain variable region CDRs (HCDR1, HCDR2, and HCDR3) and/or light chain variable region CDRs (LCDR1, LCDR2, and LCDR3), wherein:
1) the HCDR1 comprises the sequence as shown in SEQ ID NO: 1 or a sequence comprising one or more amino acid substitutions (e.g., conservative substitutions), deletions, or insertions, or any combination thereof relative to SEQ ID NO: 1, or consists of SEQ ID NO: 1; the HCDR2 comprises the sequence as shown in SEQ ID NO: 2 or a sequence comprising one or more amino acid substitutions (e.g., conservative substitutions), deletions, or insertions, or any combination thereof relative to SEQ ID NO: 2, or consists of SEQ ID NO: 2; the HCDR3 comprises the sequence as shown in SEQ ID NO: 3 or a sequence comprising one or more amino acid substitutions (e.g., conservative substitutions), deletions, or insertions, or any combination thereof relative to SEQ ID NO: 3, or consists of SEQ ID NO: 3; the LCDR1 comprises the sequence as shown in SEQ ID NO: 4 or a sequence comprising one or more amino acid substitutions (e.g., conservative substitutions), deletions, or insertions, or any combination thereof relative to SEQ ID NO: 4, or consists of SEQ ID NO: 4; the LCDR2 comprises the sequence as shown in SEQ ID NO: 5 or a sequence comprising one or more amino acid substitutions (e.g., conservative substitutions), deletions, or insertions, or any combination thereof relative to SEQ ID NO: 5, or consists of SEQ ID NO: 5; the LCDR3 comprises the sequence as shown in SEQ ID NO: 6 or a sequence comprising one or more amino acid substitutions (e.g., conservative substitutions), deletions, or insertions, or any combination thereof relative to SEQ ID NO: 6, or consists of SEQ ID NO: 6;
2) the HCDR1 comprises the sequence as shown in SEQ ID NO: 15 or a sequence comprising one or more amino acid substitutions (e.g., conservative substitutions), deletions, or insertions, or any combination thereof relative to SEQ ID NO: 15, or consists of SEQ ID NO: 15; the HCDR2 comprises the sequence as shown in SEQ ID NO: 16 or a sequence comprising one or more amino acid substitutions (e.g., conservative substitutions), deletions, or insertions, or any combination thereof relative to SEQ ID NO: 16, or consists of SEQ ID NO: 16; the HCDR3 comprises the sequence as shown in SEQ ID NO: 17 or a sequence comprising one or more amino acid substitutions (e.g., conservative substitutions), deletions, or insertions, or any combination thereof relative to SEQ ID NO: 17, or consists of SEQ ID NO: 17; the LCDR1 comprises the sequence as shown in SEQ ID NO: 18 or a sequence comprising one or more amino acid substitutions (e.g., conservative substitutions), deletions, or insertions, or any combination thereof relative to SEQ ID NO: 18, or consists of SEQ ID NO: 18; the LCDR2 comprises the sequence as shown in SEQ ID NO: 19 or a sequence comprising one or more amino acid substitutions (e.g., conservative substitutions), deletions, or insertions, or any combination thereof relative to SEQ ID NO: 19, or consists of SEQ ID NO: 19; the LCDR3 comprises the sequence as shown in SEQ ID NO: 20 or a sequence comprising one or more amino acid substitutions (e.g., conservative substitutions), deletions, or insertions, or any combination thereof relative to SEQ ID NO: 20, or consists of SEQ ID NO: 20; or
3) the HCDR1 comprises the sequence as shown in SEQ ID NO: 29 or a sequence comprising one or more amino acid substitutions (e.g., conservative substitutions), deletions, or insertions, or any combination thereof relative to SEQ ID NO: 29, or consists of SEQ ID NO: 29; the HCDR2 comprises the sequence as shown in SEQ ID NO: 30 or a sequence comprising one or more amino acid substitutions (e.g., conservative substitutions), deletions, or insertions, or any combination thereof relative to SEQ ID NO: 30, or consists of SEQ ID NO: 30; the HCDR3 comprises the sequence as shown in SEQ ID NO: 31 or a sequence comprising one or more amino acid substitutions (e.g., conservative substitutions), deletions, or insertions, or any combination thereof relative to SEQ ID NO: 31, or consists of SEQ ID NO: 31; the LCDR1 comprises the sequence as shown in SEQ ID NO: 32 or a sequence comprising one or more amino acid substitutions (e.g., conservative substitutions), deletions, or insertions, or any combination thereof relative to SEQ ID NO: 32, or consists of SEQ ID NO: 32; the LCDR2 comprises the sequence as shown in SEQ ID NO: 33 or a sequence comprising one or more amino acid substitutions (e.g., conservative substitutions), deletions, or insertions, or any combination thereof relative to SEQ ID NO: 33, or consists of SEQ ID NO: 33; the LCDR3 comprises the sequence as shown in SEQ ID NO: 34 or a sequence comprising one or more amino acid substitutions (e.g., conservative substitutions), deletions, or insertions, or any combination thereof relative to SEQ ID NO: 34, or consists of SEQ ID NO: 34.

In some embodiments, the present disclosure provides an anti-DLL3 antibody or an antigen-binding fragment thereof that binds to DLL3, which comprises a heavy chain variable region, wherein:
1) the heavy chain variable region comprises the amino acid sequence as shown in SEQ ID NO: 7, or a sequence comprising one or more amino acid substitutions (e.g., conservative substitutions), deletions, or insertions, or any combination thereof relative to SEQ ID NO: 7, or an amino acid sequence having at least 90%, 91%, 92%, 93%, 94%, 95%, 96%, 97%, 98%, or 99% identity to the amino acid sequence of SEQ ID NO: 7, or consists of SEQ ID NO: 7;
2) the heavy chain variable region comprises the amino acid sequence as shown in SEQ ID NO: 9, or a sequence comprising one or more amino acid substitutions (e.g., conservative substitutions), deletions, or insertions, or any combination thereof relative to SEQ ID NO: 9, or an amino acid sequence having at least 90%, 91%, 92%, 93%, 94%, 95%, 96%, 97%, 98%, or 99% identity to the amino acid sequence of SEQ ID NO: 9, or consists of SEQ ID NO: 9;
3) the heavy chain variable region comprises the amino acid sequence as shown in SEQ ID NO: 10, or a sequence comprising one or more amino acid substitutions (e.g., conservative substitutions), deletions, or insertions, or any combination thereof relative to SEQ ID NO: 10, or an amino acid sequence having at least 90%, 91%, 92%, 93%, 94%, 95%, 96%, 97%, 98%, or 99% identity to the amino acid sequence of SEQ ID NO: 10, or consists of SEQ ID NO: 10;
4) the heavy chain variable region comprises the amino acid sequence as shown in SEQ ID NO: 11, or a sequence comprising one or more amino acid substitutions (e.g., conservative substitutions), deletions, or insertions, or any combination thereof relative to SEQ ID NO: 11, or an amino acid sequence having at least 90%, 91%, 92%, 93%, 94%, 95%, 96%, 97%, 98%, or 99% identity to the amino acid sequence of SEQ ID NO: 11, or consists of SEQ ID NO: 11;
5) the heavy chain variable region comprises the amino acid sequence as shown in SEQ ID NO: 21, or a sequence comprising one or more amino acid substitutions (e.g., conservative substitutions), deletions, or insertions, or any combination thereof relative to SEQ ID NO: 21, or an amino acid sequence having at least 90%, 91%, 92%, 93%, 94%, 95%, 96%, 97%, 98%, or 99% identity to the amino acid sequence of SEQ ID NO: 21, or consists of SEQ ID NO: 21;
6) the heavy chain variable region comprises the amino acid sequence as shown in SEQ ID NO: 23, or a sequence comprising one or more amino acid substitutions (e.g., conservative substitutions), deletions, or insertions, or any combination thereof relative to SEQ ID NO: 23, or an amino acid sequence having at least 90%, 91%, 92%, 93%, 94%, 95%, 96%, 97%, 98%, or 99% identity to the amino acid sequence of SEQ ID NO: 23, or consists of SEQ ID NO: 23;
7) the heavy chain variable region comprises the amino acid sequence as shown in SEQ ID NO: 24, or a sequence comprising one or more amino acid substitutions (e.g., conservative substitutions), deletions, or insertions, or any combination thereof relative to SEQ ID NO: 24, or an amino acid sequence having at least 90%, 91%, 92%, 93%, 94%, 95%, 96%, 97%, 98%, or 99% identity to the amino acid sequence of SEQ ID NO: 24, or consists of SEQ ID NO: 24;
8) the heavy chain variable region comprises the amino acid sequence as shown in SEQ ID NO: 25, or a sequence comprising one or more amino acid substitutions (e.g., conservative substitutions), deletions, or insertions, or any combination thereof relative to SEQ ID NO: 25, or an amino acid sequence having at least 90%, 91%, 92%, 93%, 94%, 95%, 96%, 97%, 98%, or 99% identity to the amino acid sequence of SEQ ID NO: 25, or consists of SEQ ID NO: 25;
9) the heavy chain variable region comprises the amino acid sequence as shown in SEQ ID NO: 26, or a sequence comprising one or more amino acid substitutions (e.g., conservative substitutions), deletions, or insertions, or any combination thereof relative to SEQ ID NO: 26, or an amino acid sequence having at least 90%, 91%, 92%, 93%, 94%, 95%, 96%, 97%, 98%, or 99% identity to the amino acid sequence of SEQ ID NO: 26, or consists of SEQ ID NO: 26;
10) the heavy chain variable region comprises the amino acid sequence as shown in SEQ ID NO: 35, or a sequence comprising one or more amino acid substitutions (e.g., conservative substitutions), deletions, or insertions, or any combination thereof relative to SEQ ID NO: 35, or an amino acid sequence having at least 90%, 91%, 92%, 93%, 94%, 95%, 96%, 97%, 98%, or 99% identity to the amino acid sequence of SEQ ID NO: 35, or consists of SEQ ID NO: 35;
11) the heavy chain variable region comprises the amino acid sequence as shown in SEQ ID NO: 37, or a sequence comprising one or more amino acid substitutions (e.g., conservative substitutions), deletions, or insertions, or any combination thereof relative to SEQ ID NO: 37, or an amino acid sequence having at least 90%, 91%, 92%, 93%, 94%, 95%, 96%, 97%, 98%, or 99% identity to the amino acid sequence of SEQ ID NO: 37, or consists of SEQ ID NO: 37;
12) the heavy chain variable region comprises the amino acid sequence as shown in SEQ ID NO: 38, or a sequence comprising one or more amino acid substitutions (e.g., conservative substitutions), deletions, or insertions, or any combination thereof relative to SEQ ID NO: 38, or an amino acid sequence having at least 90%, 91%, 92%, 93%, 94%, 95%, 96%, 97%, 98%, or 99% identity to the amino acid sequence of SEQ ID NO: 38, or consists of SEQ ID NO: 38;
13) the heavy chain variable region comprises the amino acid sequence as shown in SEQ ID NO: 39, or a sequence comprising one or more amino acid substitutions (e.g., conservative substitutions), deletions, or insertions, or any combination thereof relative to SEQ ID NO: 39, or an amino acid sequence having at least 90%, 91%, 92%, 93%, 94%, 95%, 96%, 97%, 98%, or 99% identity to the amino acid sequence of SEQ ID NO: 39, or consists of SEQ ID NO: 39;
14) the heavy chain variable region comprises the amino acid sequence as shown in SEQ ID NO: 40, or a sequence comprising one or more amino acid substitutions (e.g., conservative substitutions), deletions, or insertions, or any combination thereof relative to SEQ ID NO: 40, or an amino acid sequence having at least 90%, 91%, 92%, 93%, 94%, 95%, 96%, 97%, 98%, or 99% identity to the amino acid sequence of SEQ ID NO: 40, or consists of SEQ ID NO: 40; or
15) the heavy chain variable region comprises the amino acid sequence as shown in SEQ ID NO: 41, or a sequence comprising one or more amino acid substitutions (e.g., conservative substitutions), deletions, or insertions, or any combination thereof relative to SEQ ID NO: 41, or an amino acid sequence having at least 90%, 91%, 92%, 93%, 94%, 95%, 96%, 97%, 98%, or 99% identity to the amino acid sequence of SEQ ID NO: 41, or consists of SEQ ID NO: 41.

In one embodiment, the present disclosure provides an anti-DLL3 antibody or an antigen-binding fragment thereof that binds to DLL3, which comprises a light chain variable region, wherein:
1) the light chain variable region comprises the amino acid sequence as shown in SEQ ID NO: 8, or a sequence comprising one or more amino acid substitutions (e.g., conservative substitutions), deletions, or insertions, or any combination thereof relative to SEQ ID NO: 8, or an amino acid sequence having at least 90%, 91%, 92%, 93%, 94%, 95%, 96%, 97%, 98%, or 99% identity to the amino acid sequence of SEQ ID NO: 8, or consists of SEQ ID NO: 8;
2) the light chain variable region comprises the amino acid sequence as shown in SEQ ID NO: 12, or a sequence comprising one or more amino acid substitutions (e.g., conservative substitutions), deletions, or insertions, or any combination thereof relative to SEQ ID NO: 12, or an amino acid sequence having at least 90%, 91%, 92%, 93%, 94%, 95%, 96%, 97%, 98%, or 99% identity to the amino acid sequence of SEQ ID NO: 12, or consists of SEQ ID NO: 12;
3) the light chain variable region comprises the amino acid sequence as shown in SEQ ID NO: 13, or a sequence comprising one or more amino acid substitutions (e.g., conservative substitutions), deletions, or insertions, or any combination thereof relative to SEQ ID NO: 13, or an amino acid sequence having at least 90%, 91%, 92%, 93%, 94%, 95%, 96%, 97%, 98%, or 99% identity to the amino acid sequence of SEQ ID NO: 13, or consists of SEQ ID NO: 13;
4) the light chain variable region comprises the amino acid sequence as shown in SEQ ID NO: 14, or a sequence comprising one or more amino acid substitutions (e.g., conservative substitutions), deletions, or insertions, or any combination thereof relative to SEQ ID NO: 14, or an amino acid sequence having at least 90%, 91%, 92%, 93%, 94%, 95%, 96%, 97%, 98%, or 99% identity to the amino acid sequence of SEQ ID NO: 14, or consists of SEQ ID NO: 14;
5) the light chain variable region comprises the amino acid sequence as shown in SEQ ID NO: 22, or a sequence comprising one or more amino acid substitutions (e.g., conservative substitutions), deletions, or insertions, or any combination thereof relative to SEQ ID NO: 22, or an amino acid sequence having at least 90%, 91%, 92%, 93%, 94%, 95%, 96%, 97%, 98%, or 99% identity to the amino acid sequence of SEQ ID NO: 22, or consists of SEQ ID NO: 22;
6) the light chain variable region comprises the amino acid sequence as shown in SEQ ID NO: 27, or a sequence comprising one or more amino acid substitutions (e.g., conservative substitutions), deletions, or insertions, or any combination thereof relative to SEQ ID NO: 27, or an amino acid sequence having at least 90%, 91%, 92%, 93%, 94%, 95%, 96%, 97%, 98%, or 99% identity to the amino acid sequence of SEQ ID NO: 27, or consists of SEQ ID NO: 27;
7) the light chain variable region comprises the amino acid sequence as shown in SEQ ID NO: 28, or a sequence comprising one or more amino acid substitutions (e.g., conservative substitutions), deletions, or insertions, or any combination thereof relative to SEQ ID NO: 28, or an amino acid sequence having at least 90%, 91%, 92%, 93%, 94%, 95%, 96%, 97%, 98%, or 99% identity to the amino acid sequence of SEQ ID NO: 28, or consists of SEQ ID NO: 28;
8) the light chain variable region comprises the amino acid sequence as shown in SEQ ID NO: 36, or a sequence comprising one or more amino acid substitutions (e.g., conservative substitutions), deletions, or insertions, or any combination thereof relative to SEQ ID NO: 36, or an amino acid sequence having at least 90%, 91%, 92%, 93%, 94%, 95%, 96%, 97%, 98%, or 99% identity to the amino acid sequence of SEQ ID NO: 36, or consists of SEQ ID NO: 36;
9) the light chain variable region comprises the amino acid sequence as shown in SEQ ID NO: 42, or a sequence comprising one or more amino acid substitutions (e.g., conservative substitutions), deletions, or insertions, or any combination thereof relative to SEQ ID NO: 42, or an amino acid sequence having at least 90%, 91%, 92%, 93%, 94%, 95%, 96%, 97%, 98%, or 99% identity to the amino acid sequence of SEQ ID NO: 42, or consists of SEQ ID NO: 42;
10) the light chain variable region comprises the amino acid sequence as shown in SEQ ID NO: 43, or a sequence comprising one or more amino acid substitutions (e.g., conservative substitutions), deletions, or insertions, or any combination thereof relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consists of SEQ ID NO: 43; or
11) the light chain variable region comprises the amino acid sequence as shown in SEQ ID NO: 44, or a sequence comprising one or more amino acid substitutions (e.g., conservative substitutions), deletions, or insertions, or any combination thereof relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consists of SEQ ID NO: 44.

In some embodiments, the present disclosure provides an anti-DLL3 antibody or an antigen-binding fragment thereof that binds to DLL3, wherein a heavy chain variable region and/or a light chain variable region comprised therein are selected from the following combinations:
1) the heavy chain variable region comprising the amino acid sequence as shown in SEQ ID NO: 7, or a sequence comprising one or more amino acid substitutions (e.g., conservative substitutions), deletions, or insertions, or any combination thereof relative to SEQ ID NO: 7, or an amino acid sequence having at least 90%, 91%, 92%, 93%, 94%, 95%, 96%, 97%, 98%, or 99% identity to the amino acid sequence of SEQ ID NO: 7, or consisting of SEQ ID NO: 7; the light chain variable region comprising the amino acid sequence as shown in SEQ ID NO: 8, or a sequence comprising one or more amino acid substitutions (e.g., conservative substitutions), deletions, or insertions, or any combination thereof relative to SEQ ID NO: 8, or an amino acid sequence having at least 90%, 91%, 92%, 93%, 94%, 95%, 96%, 97%, 98%, or 99% identity to the amino acid sequence of SEQ ID NO: 8, or consisting of SEQ ID NO: 8;
2) the heavy chain variable region comprising the amino acid sequence as shown in SEQ ID NO: 9, or a sequence comprising one or more amino acid substitutions (e.g., conservative substitutions), deletions, or insertions, or any combination thereof relative to SEQ ID NO: 9, or an amino acid sequence having at least 90%, 91%, 92%, 93%, 94%, 95%, 96%, 97%, 98%, or 99% identity to the amino acid sequence of SEQ ID NO: 9, or consisting of SEQ ID NO: 9; the light chain variable region comprising the amino acid sequence as shown in SEQ ID NO: 12, or a sequence comprising one or more amino acid substitutions (e.g., conservative substitutions), deletions, or insertions, or any combination thereof relative to SEQ ID NO: 12, or an amino acid sequence having at least 90%, 91%, 92%, 93%, 94%, 95%, 96%, 97%, 98%, or 99% identity to the amino acid sequence of SEQ ID NO: 12, or consisting of SEQ ID NO: 12;
3) the heavy chain variable region comprising the amino acid sequence as shown in SEQ ID NO: 9, or a sequence comprising one or more amino acid substitutions (e.g., conservative substitutions), deletions, or insertions, or any combination thereof relative to SEQ ID NO: 9, or an amino acid sequence having at least 90%, 91%, 92%, 93%, 94%, 95%, 96%, 97%, 98%, or 99% identity to the amino acid sequence of SEQ ID NO: 9, or consisting of SEQ ID NO: 9; the light chain variable region comprising the amino acid sequence as shown in SEQ ID NO: 13, or a sequence comprising one or more amino acid substitutions (e.g., conservative substitutions), deletions, or insertions, or any combination thereof relative to SEQ ID NO: 13, or an amino acid sequence having at least 90%, 91%, 92%, 93%, 94%, 95%, 96%, 97%, 98%, or 99% identity to the amino acid sequence of SEQ ID NO: 13, or consisting of SEQ ID NO: 13;
4) the heavy chain variable region comprising the amino acid sequence as shown in SEQ ID NO: 9, or a sequence comprising one or more amino acid substitutions (e.g., conservative substitutions), deletions, or insertions, or any combination thereof relative to SEQ ID NO: 9, or an amino acid sequence having at least 90%, 91%, 92%, 93%, 94%, 95%, 96%, 97%, 98%, or 99% identity to the amino acid sequence of SEQ ID NO: 9, or consisting of SEQ ID NO: 9; the light chain variable region comprising the amino acid sequence as shown in SEQ ID NO: 14, or a sequence comprising one or more amino acid substitutions (e.g., conservative substitutions), deletions, or insertions, or any combination thereof relative to SEQ ID NO: 14, or an amino acid sequence having at least 90%, 91%, 92%, 93%, 94%, 95%, 96%, 97%, 98%, or 99% identity to the amino acid sequence of SEQ ID NO: 14, or consisting of SEQ ID NO: 14;
5) the heavy chain variable region comprising the amino acid sequence as shown in SEQ ID NO: 10, or a sequence comprising one or more amino acid substitutions (e.g., conservative substitutions), deletions, or insertions, or any combination thereof relative to SEQ ID NO: 10, or an amino acid sequence having at least 90%, 91%, 92%, 93%, 94%, 95%, 96%, 97%, 98%, or 99% identity to the amino acid sequence of SEQ ID NO: 10, or consisting of SEQ ID NO: 10; the light chain variable region comprising the amino acid sequence as shown in SEQ ID NO: 12, or a sequence comprising one or more amino acid substitutions (e.g., conservative substitutions), deletions, or insertions, or any combination thereof relative to SEQ ID NO: 12, or an amino acid sequence having at least 90%, 91%, 92%, 93%, 94%, 95%, 96%, 97%, 98%, or 99% identity to the amino acid sequence of SEQ ID NO: 12, or consisting of SEQ ID NO: 12;
6) the heavy chain variable region comprising the amino acid sequence as shown in SEQ ID NO: 10, or a sequence comprising one or more amino acid substitutions (e.g., conservative substitutions), deletions, or insertions, or any combination thereof relative to SEQ ID NO: 10, or an amino acid sequence having at least 90%, 91%, 92%, 93%, 94%, 95%, 96%, 97%, 98%, or 99% identity to the amino acid sequence of SEQ ID NO: 10, or consisting of SEQ ID NO: 10; the light chain variable region comprising the amino acid sequence as shown in SEQ ID NO: 13, or a sequence comprising one or more amino acid substitutions (e.g., conservative substitutions), deletions, or insertions, or any combination thereof relative to SEQ ID NO: 13, or an amino acid sequence having at least 90%, 91%, 92%, 93%, 94%, 95%, 96%, 97%, 98%, or 99% identity to the amino acid sequence of SEQ ID NO: 13, or consisting of SEQ ID NO: 13;
7) the heavy chain variable region comprising the amino acid sequence as shown in SEQ ID NO: 10, or a sequence comprising one or more amino acid substitutions (e.g., conservative substitutions), deletions, or insertions, or any combination thereof relative to SEQ ID NO: 10, or an amino acid sequence having at least 90%, 91%, 92%, 93%, 94%, 95%, 96%, 97%, 98%, or 99% identity to the amino acid sequence of SEQ ID NO: 10, or consisting of SEQ ID NO: 10; the light chain variable region comprising the amino acid sequence as shown in SEQ ID NO: 14, or a sequence comprising one or more amino acid substitutions (e.g., conservative substitutions), deletions, or insertions, or any combination thereof relative to SEQ ID NO: 14, or an amino acid sequence having at least 90%, 91%, 92%, 93%, 94%, 95%, 96%, 97%, 98%, or 99% identity to the amino acid sequence of SEQ ID NO: 14, or consisting of SEQ ID NO: 14;
8) the heavy chain variable region comprising the amino acid sequence as shown in SEQ ID NO: 11, or a sequence comprising one or more amino acid substitutions (e.g., conservative substitutions), deletions, or insertions, or any combination thereof relative to SEQ ID NO: 11, or an amino acid sequence having at least 90%, 91%, 92%, 93%, 94%, 95%, 96%, 97%, 98%, or 99% identity to the amino acid sequence of SEQ ID NO: 11, or consisting of SEQ ID NO: 11; the light chain variable region comprising the amino acid sequence as shown in SEQ ID NO: 12, or a sequence comprising one or more amino acid substitutions (e.g., conservative substitutions), deletions, or insertions, or any combination thereof relative to SEQ ID NO: 12, or an amino acid sequence having at least 90%, 91%, 92%, 93%, 94%, 95%, 96%, 97%, 98%, or 99% identity to the amino acid sequence of SEQ ID NO: 12, or consisting of SEQ ID NO: 12;
9) the heavy chain variable region comprising the amino acid sequence as shown in SEQ ID NO: 11, or a sequence comprising one or more amino acid substitutions (e.g., conservative substitutions), deletions, or insertions, or any combination thereof relative to SEQ ID NO: 11, or an amino acid sequence having at least 90%, 91%, 92%, 93%, 94%, 95%, 96%, 97%, 98%, or 99% identity to the amino acid sequence of SEQ ID NO: 11, or consisting of SEQ ID NO: 11; the light chain variable region comprising the amino acid sequence as shown in SEQ ID NO: 13, or a sequence comprising one or more amino acid substitutions (e.g., conservative substitutions), deletions, or insertions, or any combination thereof relative to SEQ ID NO: 13, or an amino acid sequence having at least 90%, 91%, 92%, 93%, 94%, 95%, 96%, 97%, 98%, or 99% identity to the amino acid sequence of SEQ ID NO: 13, or consisting of SEQ ID NO: 13;
10) the heavy chain variable region comprising the amino acid sequence as shown in SEQ ID NO: 11, or a sequence comprising one or more amino acid substitutions (e.g., conservative substitutions), deletions, or insertions, or any combination thereof relative to SEQ ID NO: 11, or an amino acid sequence having at least 90%, 91%, 92%, 93%, 94%, 95%, 96%, 97%, 98%, or 99% identity to the amino acid sequence of SEQ ID NO: 11, or consisting of SEQ ID NO: 11; the light chain variable region comprising the amino acid sequence as shown in SEQ ID NO: 14, or a sequence comprising one or more amino acid substitutions (e.g., conservative substitutions), deletions, or insertions, or any combination thereof relative to SEQ ID NO: 14, or an amino acid sequence having at least 90%, 91%, 92%, 93%, 94%, 95%, 96%, 97%, 98%, or 99% identity to the amino acid sequence of SEQ ID NO: 14, or consisting of SEQ ID NO: 14;
11) the heavy chain variable region comprising the amino acid sequence as shown in SEQ ID NO: 21, or a sequence comprising one or more amino acid substitutions (e.g., conservative substitutions), deletions, or insertions, or any combination thereof relative to SEQ ID NO: 21, or an amino acid sequence having at least 90%, 91%, 92%, 93%, 94%, 95%, 96%, 97%, 98%, or 99% identity to the amino acid sequence of SEQ ID NO: 21, or consisting of SEQ ID NO: 21; the light chain variable region comprising the amino acid sequence as shown in SEQ ID NO: 22, or a sequence comprising one or more amino acid substitutions (e.g., conservative substitutions), deletions, or insertions, or any combination thereof relative to SEQ ID NO: 22, or an amino acid sequence having at least 90%, 91%, 92%, 93%, 94%, 95%, 96%, 97%, 98%, or 99% identity to the amino acid sequence of SEQ ID NO: 22, or consisting of SEQ ID NO: 22;
12) the heavy chain variable region comprising the amino acid sequence as shown in SEQ ID NO: 23, or a sequence comprising one or more amino acid substitutions (e.g., conservative substitutions), deletions, or insertions, or any combination thereof relative to SEQ ID NO: 23, or an amino acid sequence having at least 90%, 91%, 92%, 93%, 94%, 95%, 96%, 97%, 98%, or 99% identity to the amino acid sequence of SEQ ID NO: 23, or consisting of SEQ ID NO: 23; the light chain variable region comprising the amino acid sequence as shown in SEQ ID NO: 28, or a sequence comprising one or more amino acid substitutions (e.g., conservative substitutions), deletions, or insertions, or any combination thereof relative to SEQ ID NO: 28, or an amino acid sequence having at least 90%, 91%, 92%, 93%, 94%, 95%, 96%, 97%, 98%, or 99% identity to the amino acid sequence of SEQ ID NO: 28, or consisting of SEQ ID NO: 28;
13) the heavy chain variable region comprising the amino acid sequence as shown in SEQ ID NO: 35, or a sequence comprising one or more amino acid substitutions (e.g., conservative substitutions), deletions, or insertions, or any combination thereof relative to SEQ ID NO: 35, or an amino acid sequence having at least 90%, 91%, 92%, 93%, 94%, 95%, 96%, 97%, 98%, or 99% identity to the amino acid sequence of SEQ ID NO: 35, or consisting of SEQ ID NO: 35; the light chain variable region comprising the amino acid sequence as shown in SEQ ID NO: 36, or a sequence comprising one or more amino acid substitutions (e.g., conservative substitutions), deletions, or insertions, or any combination thereof relative to SEQ ID NO: 36, or an amino acid sequence having at least 90%, 91%, 92%, 93%, 94%, 95%, 96%, 97%, 98%, or 99% identity to the amino acid sequence of SEQ ID NO: 36, or consisting of SEQ ID NO: 36;
14) the heavy chain variable region comprising the amino acid sequence as shown in SEQ ID NO: 40, or a sequence comprising one or more amino acid substitutions (e.g., conservative substitutions), deletions, or insertions, or any combination thereof relative to SEQ ID NO: 40, or an amino acid sequence having at least 90%, 91%, 92%, 93%, 94%, 95%, 96%, 97%, 98%, or 99% identity to the amino acid sequence of SEQ ID NO: 40, or consisting of SEQ ID NO: 40; the light chain variable region comprising the amino acid sequence as shown in SEQ ID NO: 43, or a sequence comprising one or more amino acid substitutions (e.g., conservative substitutions), deletions, or insertions, or any combination thereof relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consisting of SEQ ID NO: 43;
15) the heavy chain variable region comprising the amino acid sequence as shown in SEQ ID NO: 40, or a sequence comprising one or more amino acid substitutions (e.g., conservative substitutions), deletions, or insertions, or any combination thereof relative to SEQ ID NO: 40, or an amino acid sequence having at least 90%, 91%, 92%, 93%, 94%, 95%, 96%, 97%, 98%, or 99% identity to the amino acid sequence of SEQ ID NO: 40, or consisting of SEQ ID NO: 40; the light chain variable region comprising the amino acid sequence as shown in SEQ ID NO: 44, or a sequence comprising one or more amino acid substitutions (e.g., conservative substitutions), deletions, or insertions, or any combination thereof relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consisting of SEQ ID NO: 44;
16) the heavy chain variable region comprising the amino acid sequence as shown in SEQ ID NO: 41, or a sequence comprising one or more amino acid substitutions (e.g., conservative substitutions), deletions, or insertions, or any combination thereof relative to SEQ ID NO: 41, or an amino acid sequence having at least 90%, 91%, 92%, 93%, 94%, 95%, 96%, 97%, 98%, or 99% identity to the amino acid sequence of SEQ ID NO: 41, or consisting of SEQ ID NO: 41; the light chain variable region comprising the amino acid sequence as shown in SEQ ID NO: 43, or a sequence comprising one or more amino acid substitutions (e.g., conservative substitutions), deletions, or insertions, or any combination thereof relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consisting of SEQ ID NO: 43;
17) the heavy chain variable region comprising the amino acid sequence as shown in SEQ ID NO: 23, or a sequence comprising one or more amino acid substitutions (e.g., conservative substitutions), deletions, or insertions, or any combination thereof relative to SEQ ID NO: 23, or an amino acid sequence having at least 90%, 91%, 92%, 93%, 94%, 95%, 96%, 97%, 98%, or 99% identity to the amino acid sequence of SEQ ID NO: 23, or consisting of SEQ ID NO: 23; the light chain variable region comprising the amino acid sequence as shown in SEQ ID NO: 27, or a sequence comprising one or more amino acid substitutions (e.g., conservative substitutions), deletions, or insertions, or any combination thereof relative to SEQ ID NO: 27, or an amino acid sequence having at least 90%, 91%, 92%, 93%, 94%, 95%, 96%, 97%, 98%, or 99% identity to the amino acid sequence of SEQ ID NO: 27, or consisting of SEQ ID NO: 27;
18) the heavy chain variable region comprising the amino acid sequence as shown in SEQ ID NO: 24, or a sequence comprising one or more amino acid substitutions (e.g., conservative substitutions), deletions, or insertions, or any combination thereof relative to SEQ ID NO: 24, or an amino acid sequence having at least 90%, 91%, 92%, 93%, 94%, 95%, 96%, 97%, 98%, or 99% identity to the amino acid sequence of SEQ ID NO: 24, or consisting of SEQ ID NO: 24; the light chain variable region comprising the amino acid sequence as shown in SEQ ID NO: 27, or a sequence comprising one or more amino acid substitutions (e.g., conservative substitutions), deletions, or insertions, or any combination thereof relative to SEQ ID NO: 27, or an amino acid sequence having at least 90%, 91%, 92%, 93%, 94%, 95%, 96%, 97%, 98%, or 99% identity to the amino acid sequence of SEQ ID NO: 27, or consisting of SEQ ID NO: 27;
19) the heavy chain variable region comprising the amino acid sequence as shown in SEQ ID NO: 24, or a sequence comprising one or more amino acid substitutions (e.g., conservative substitutions), deletions, or insertions, or any combination thereof relative to SEQ ID NO: 24, or an amino acid sequence having at least 90%, 91%, 92%, 93%, 94%, 95%, 96%, 97%, 98%, or 99% identity to the amino acid sequence of SEQ ID NO: 24, or consisting of SEQ ID NO: 24; the light chain variable region comprising the amino acid sequence as shown in SEQ ID NO: 28, or a sequence comprising one or more amino acid substitutions (e.g., conservative substitutions), deletions, or insertions, or any combination thereof relative to SEQ ID NO: 28, or an amino acid sequence having at least 90%, 91%, 92%, 93%, 94%, 95%, 96%, 97%, 98%, or 99% identity to the amino acid sequence of SEQ ID NO: 28, or consisting of SEQ ID NO: 28;
20) the heavy chain variable region comprising the amino acid sequence as shown in SEQ ID NO: 25, or a sequence comprising one or more amino acid substitutions (e.g., conservative substitutions), deletions, or insertions, or any combination thereof relative to SEQ ID NO: 25, or an amino acid sequence having at least 90%, 91%, 92%, 93%, 94%, 95%, 96%, 97%, 98%, or 99% identity to the amino acid sequence of SEQ ID NO: 25, or consisting of SEQ ID NO: 25; the light chain variable region comprising the amino acid sequence as shown in SEQ ID NO: 27, or a sequence comprising one or more amino acid substitutions (e.g., conservative substitutions), deletions, or insertions, or any combination thereof relative to SEQ ID NO: 27, or an amino acid sequence having at least 90%, 91%, 92%, 93%, 94%, 95%, 96%, 97%, 98%, or 99% identity to the amino acid sequence of SEQ ID NO: 27, or consisting of SEQ ID NO: 27;
21) the heavy chain variable region comprising the amino acid sequence as shown in SEQ ID NO: 25, or a sequence comprising one or more amino acid substitutions (e.g., conservative substitutions), deletions, or insertions, or any combination thereof relative to SEQ ID NO: 25, or an amino acid sequence having at least 90%, 91%, 92%, 93%, 94%, 95%, 96%, 97%, 98%, or 99% identity to the amino acid sequence of SEQ ID NO: 25, or consisting of SEQ ID NO: 25; the light chain variable region comprising the amino acid sequence as shown in SEQ ID NO: 28, or a sequence comprising one or more amino acid substitutions (e.g., conservative substitutions), deletions, or insertions, or any combination thereof relative to SEQ ID NO: 28, or an amino acid sequence having at least 90%, 91%, 92%, 93%, 94%, 95%, 96%, 97%, 98%, or 99% identity to the amino acid sequence of SEQ ID NO: 28, or consisting of SEQ ID NO: 28;
22) the heavy chain variable region comprising the amino acid sequence as shown in SEQ ID NO: 26, or a sequence comprising one or more amino acid substitutions (e.g., conservative substitutions), deletions, or insertions, or any combination thereof relative to SEQ ID NO: 26, or an amino acid sequence having at least 90%, 91%, 92%, 93%, 94%, 95%, 96%, 97%, 98%, or 99% identity to the amino acid sequence of SEQ ID NO: 26, or consisting of SEQ ID NO: 26; the light chain variable region comprising the amino acid sequence as shown in SEQ ID NO: 27, or a sequence comprising one or more amino acid substitutions (e.g., conservative substitutions), deletions, or insertions, or any combination thereof relative to SEQ ID NO: 27, or an amino acid sequence having at least 90%, 91%, 92%, 93%, 94%, 95%, 96%, 97%, 98%, or 99% identity to the amino acid sequence of SEQ ID NO: 27, or consisting of SEQ ID NO: 27;
23) the heavy chain variable region comprising the amino acid sequence as shown in SEQ ID NO: 26, or a sequence comprising one or more amino acid substitutions (e.g., conservative substitutions), deletions, or insertions, or any combination thereof relative to SEQ ID NO: 26, or an amino acid sequence having at least 90%, 91%, 92%, 93%, 94%, 95%, 96%, 97%, 98%, or 99% identity to the amino acid sequence of SEQ ID NO: 26, or consisting of SEQ ID NO: 26; the light chain variable region comprising the amino acid sequence as shown in SEQ ID NO: 28, or a sequence comprising one or more amino acid substitutions (e.g., conservative substitutions), deletions, or insertions, or any combination thereof relative to SEQ ID NO: 28, or an amino acid sequence having at least 90%, 91%, 92%, 93%, 94%, 95%, 96%, 97%, 98%, or 99% identity to the amino acid sequence of SEQ ID NO: 28, or consisting of SEQ ID NO: 28;
24) the heavy chain variable region comprising the amino acid sequence as shown in SEQ ID NO: 37, or a sequence comprising one or more amino acid substitutions (e.g., conservative substitutions), deletions, or insertions, or any combination thereof relative to SEQ ID NO: 37, or an amino acid sequence having at least 90%, 91%, 92%, 93%, 94%, 95%, 96%, 97%, 98%, or 99% identity to the amino acid sequence of SEQ ID NO: 37, or consisting of SEQ ID NO: 37; the light chain variable region comprising the amino acid sequence as shown in SEQ ID NO: 42, or a sequence comprising one or more amino acid substitutions (e.g., conservative substitutions), deletions, or insertions, or any combination thereof relative to SEQ ID NO: 42, or an amino acid sequence having at least 90%, 91%, 92%, 93%, 94%, 95%, 96%, 97%, 98%, or 99% identity to the amino acid sequence of SEQ ID NO: 42, or consisting of SEQ ID NO: 42;
25) the heavy chain variable region comprising the amino acid sequence as shown in SEQ ID NO: 37, or a sequence comprising one or more amino acid substitutions (e.g., conservative substitutions), deletions, or insertions, or any combination thereof relative to SEQ ID NO: 37, or an amino acid sequence having at least 90%, 91%, 92%, 93%, 94%, 95%, 96%, 97%, 98%, or 99% identity to the amino acid sequence of SEQ ID NO: 37, or consisting of SEQ ID NO: 37; the light chain variable region comprising the amino acid sequence as shown in SEQ ID NO: 43, or a sequence comprising one or more amino acid substitutions (e.g., conservative substitutions), deletions, or insertions, or any combination thereof relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consisting of SEQ ID NO: 43;
26) the heavy chain variable region comprising the amino acid sequence as shown in SEQ ID NO: 37, or a sequence comprising one or more amino acid substitutions (e.g., conservative substitutions), deletions, or insertions, or any combination thereof relative to SEQ ID NO: 37, or an amino acid sequence having at least 90%, 91%, 92%, 93%, 94%, 95%, 96%, 97%, 98%, or 99% identity to the amino acid sequence of SEQ ID NO: 37, or consisting of SEQ ID NO: 37; the light chain variable region comprising the amino acid sequence as shown in SEQ ID NO: 44, or a sequence comprising one or more amino acid substitutions (e.g., conservative substitutions), deletions, or insertions, or any combination thereof relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consisting of SEQ ID NO: 44;
27) the heavy chain variable region comprising the amino acid sequence as shown in SEQ ID NO: 38, or a sequence comprising one or more amino acid substitutions (e.g., conservative substitutions), deletions, or insertions, or any combination thereof relative to SEQ ID NO: 38, or an amino acid sequence having at least 90%, 91%, 92%, 93%, 94%, 95%, 96%, 97%, 98%, or 99% identity to the amino acid sequence of SEQ ID NO: 38, or consisting of SEQ ID NO: 38; the light chain variable region comprising the amino acid sequence as shown in SEQ ID NO: 42, or a sequence comprising one or more amino acid substitutions (e.g., conservative substitutions), deletions, or insertions, or any combination thereof relative to SEQ ID NO: 42, or an amino acid sequence having at least 90%, 91%, 92%, 93%, 94%, 95%, 96%, 97%, 98%, or 99% identity to the amino acid sequence of SEQ ID NO: 42, or consisting of SEQ ID NO: 42;
28) the heavy chain variable region comprising the amino acid sequence as shown in SEQ ID NO: 38, or a sequence comprising one or more amino acid substitutions (e.g., conservative substitutions), deletions, or insertions, or any combination thereof relative to SEQ ID NO: 38, or an amino acid sequence having at least 90%, 91%, 92%, 93%, 94%, 95%, 96%, 97%, 98%, or 99% identity to the amino acid sequence of SEQ ID NO: 38, or consisting of SEQ ID NO: 38; the light chain variable region comprising the amino acid sequence as shown in SEQ ID NO: 43, or a sequence comprising one or more amino acid substitutions (e.g., conservative substitutions), deletions, or insertions, or any combination thereof relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consisting of SEQ ID NO: 43;
29) the heavy chain variable region comprising the amino acid sequence as shown in SEQ ID NO: 38, or a sequence comprising one or more amino acid substitutions (e.g., conservative substitutions), deletions, or insertions, or any combination thereof relative to SEQ ID NO: 38, or an amino acid sequence having at least 90%, 91%, 92%, 93%, 94%, 95%, 96%, 97%, 98%, or 99% identity to the amino acid sequence of SEQ ID NO: 38, or consisting of SEQ ID NO: 38; the light chain variable region comprising the amino acid sequence as shown in SEQ ID NO: 44, or a sequence comprising one or more amino acid substitutions (e.g., conservative substitutions), deletions, or insertions, or any combination thereof relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consisting of SEQ ID NO: 44;
30) the heavy chain variable region comprising the amino acid sequence as shown in SEQ ID NO: 39, or a sequence comprising one or more amino acid substitutions (e.g., conservative substitutions), deletions, or insertions, or any combination thereof relative to SEQ ID NO: 39, or an amino acid sequence having at least 90%, 91%, 92%, 93%, 94%, 95%, 96%, 97%, 98%, or 99% identity to the amino acid sequence of SEQ ID NO: 39, or consisting of SEQ ID NO: 39; the light chain variable region comprising the amino acid sequence as shown in SEQ ID NO: 42, or a sequence comprising one or more amino acid substitutions (e.g., conservative substitutions), deletions, or insertions, or any combination thereof relative to SEQ ID NO: 42, or an amino acid sequence having at least 90%, 91%, 92%, 93%, 94%, 95%, 96%, 97%, 98%, or 99% identity to the amino acid sequence of SEQ ID NO: 42, or consisting of SEQ ID NO: 42;
31) the heavy chain variable region comprising the amino acid sequence as shown in SEQ ID NO: 39, or a sequence comprising one or more amino acid substitutions (e.g., conservative substitutions), deletions, or insertions, or any combination thereof relative to SEQ ID NO: 39, or an amino acid sequence having at least 90%, 91%, 92%, 93%, 94%, 95%, 96%, 97%, 98%, or 99% identity to the amino acid sequence of SEQ ID NO: 39, or consisting of SEQ ID NO: 39; the light chain variable region comprising the amino acid sequence as shown in SEQ ID NO: 43, or a sequence comprising one or more amino acid substitutions (e.g., conservative substitutions), deletions, or insertions, or any combination thereof relative to SEQ ID NO: 43, or an amino acid sequence having at least 90%, 91%, 92%, 93%, 94%, 95%, 96%, 97%, 98%, or 99% identity to the amino acid sequence of SEQ ID NO: 43, or consisting of SEQ ID NO: 43;
32) the heavy chain variable region comprising the amino acid sequence as shown in SEQ ID NO: 39, or a sequence comprising one or more amino acid substitutions (e.g., conservative substitutions), deletions, or insertions, or any combination thereof relative to SEQ ID NO: 39, or an amino acid sequence having at least 90%, 91%, 92%, 93%, 94%, 95%, 96%, 97%, 98%, or 99% identity to the amino acid sequence of SEQ ID NO: 39, or consisting of SEQ ID NO: 39; the light chain variable region comprising the amino acid sequence as shown in SEQ ID NO: 44, or a sequence comprising one or more amino acid substitutions (e.g., conservative substitutions), deletions, or insertions, or any combination thereof relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consisting of SEQ ID NO: 44;
33) the heavy chain variable region comprising the amino acid sequence as shown in SEQ ID NO: 40, or a sequence comprising one or more amino acid substitutions (e.g., conservative substitutions), deletions, or insertions, or any combination thereof relative to SEQ ID NO: 40, or an amino acid sequence having at least 90%, 91%, 92%, 93%, 94%, 95%, 96%, 97%, 98%, or 99% identity to the amino acid sequence of SEQ ID NO: 40, or consisting of SEQ ID NO: 40; the light chain variable region comprising the amino acid sequence as shown in SEQ ID NO: 42, or a sequence comprising one or more amino acid substitutions (e.g., conservative substitutions), deletions, or insertions, or any combination thereof relative to SEQ ID NO: 42, or an amino acid sequence having at least 90%, 91%, 92%, 93%, 94%, 95%, 96%, 97%, 98%, or 99% identity to the amino acid sequence of SEQ ID NO: 42, or consisting of SEQ ID NO: 42;
34) the heavy chain variable region comprising the amino acid sequence as shown in SEQ ID NO: 41, or a sequence comprising one or more amino acid substitutions (e.g., conservative substitutions), deletions, or insertions, or any combination thereof relative to SEQ ID NO: 41, or an amino acid sequence having at least 90%, 91%, 92%, 93%, 94%, 95%, 96%, 97%, 98%, or 99% identity to the amino acid sequence of SEQ ID NO: 41, or consisting of SEQ ID NO: 41; the light chain variable region comprising the amino acid sequence as shown in SEQ ID NO: 42, or a sequence comprising one or more amino acid substitutions (e.g., conservative substitutions), deletions, or insertions, or any combination thereof relative to SEQ ID NO: 42, or an amino acid sequence having at least 90%, 91%, 92%, 93%, 94%, 95%, 96%, 97%, 98%, or 99% identity to the amino acid sequence of SEQ ID NO: 42, or consisting of SEQ ID NO: 42; and
35) the heavy chain variable region comprising the amino acid sequence as shown in SEQ ID NO: 41, or a sequence comprising one or more amino acid substitutions (e.g., conservative substitutions), deletions, or insertions, or any combination thereof relative to SEQ ID NO: 41, or an amino acid sequence having at least 90%, 91%, 92%, 93%, 94%, 95%, 96%, 97%, 98%, or 99% identity to the amino acid sequence of SEQ ID NO: 41, or consisting of SEQ ID NO: 41; the light chain variable region comprising the amino acid sequence as shown in SEQ ID NO: 44, or a sequence comprising one or more amino acid substitutions (e.g., conservative substitutions), deletions, or insertions, or any combination thereof relative to SEQ ID NO: 44, or an amino acid sequence having at least 90%, 91%, 92%, 93%, 94%, 95%, 96%, 97%, 98%, or 99% identity to the amino acid sequence of SEQ ID NO: 44, or consisting of SEQ ID NO: 44.

In some embodiments, the present disclosure provides an anti-DLL3 antibody or an antigen-binding fragment thereof that binds to DLL3, which comprises a heavy chain variable region and/or a light chain variable region, wherein:
1) the heavy chain variable region comprises or consists of the amino acid sequence as shown in SEQ ID NO: 7, and the light chain variable region comprises or consists of the amino acid sequence as shown in SEQ ID NO: 8;
2) the heavy chain variable region comprises or consists of the amino acid sequence as shown in SEQ ID NO: 9, and the light chain variable region comprises or consists of the amino acid sequence as shown in SEQ ID NO: 12;
3) the heavy chain variable region comprises or consists of the amino acid sequence as shown in SEQ ID NO: 9, and the light chain variable region comprises or consists of the amino acid sequence as shown in SEQ ID NO: 13;
4) the heavy chain variable region comprises or consists of the amino acid sequence as shown in SEQ ID NO: 9, and the light chain variable region comprises or consists of the amino acid sequence as shown in SEQ ID NO: 14;
5) the heavy chain variable region comprises or consists of the amino acid sequence as shown in SEQ ID NO: 10, and the light chain variable region comprises or consists of the amino acid sequence as shown in SEQ ID NO: 12;
6) the heavy chain variable region comprises or consists of the amino acid sequence as shown in SEQ ID NO: 10, and the light chain variable region comprises or consists of the amino acid sequence as shown in SEQ ID NO: 13;
7) the heavy chain variable region comprises or consists of the amino acid sequence as shown in SEQ ID NO: 10, and the light chain variable region comprises or consists of the amino acid sequence as shown in SEQ ID NO: 14;
8) the heavy chain variable region comprises or consists of the amino acid sequence as shown in SEQ ID NO: 11, and the light chain variable region comprises or consists of the amino acid sequence as shown in SEQ ID NO: 12;
9) the heavy chain variable region comprises or consists of the amino acid sequence as shown in SEQ ID NO: 11, and the light chain variable region comprises or consists of the amino acid sequence as shown in SEQ ID NO: 13;
10) the heavy chain variable region comprises or consists of the amino acid sequence as shown in SEQ ID NO: 11, and the light chain variable region comprises or consists of the amino acid sequence as shown in SEQ ID NO: 14;
11) the heavy chain variable region comprises or consists of the amino acid sequence as shown in SEQ ID NO: 21, and the light chain variable region comprises or consists of the amino acid sequence as shown in SEQ ID NO: 22;
12) the heavy chain variable region comprises or consists of the amino acid sequence as shown in SEQ ID NO: 23, and the light chain variable region comprises or consists of the amino acid sequence as shown in SEQ ID NO: 28;
13) the heavy chain variable region comprises or consists of the amino acid sequence as shown in SEQ ID NO: 35, and the light chain variable region comprises or consists of the amino acid sequence as shown in SEQ ID NO: 36;
14) the heavy chain variable region comprises or consists of the amino acid sequence as shown in SEQ ID NO: 40, and the light chain variable region comprises or consists of the amino acid sequence as shown in SEQ ID NO: 43;
15) the heavy chain variable region comprises or consists of the amino acid sequence as shown in SEQ ID NO: 40, and the light chain variable region comprises or consists of the amino acid sequence as shown in SEQ ID NO: 44;
16) the heavy chain variable region comprises or consists of the amino acid sequence as shown in SEQ ID NO: 41, and the light chain variable region comprises or consists of the amino acid sequence as shown in SEQ ID NO: 43;
17) the heavy chain variable region comprises or consists of the amino acid sequence as shown in SEQ ID NO: 23, and the light chain variable region comprises or consists of the amino acid sequence as shown in SEQ ID NO: 27;
18) the heavy chain variable region comprises or consists of the amino acid sequence as shown in SEQ ID NO: 24, and the light chain variable region comprises or consists of the amino acid sequence as shown in SEQ ID NO: 27;
19) the heavy chain variable region comprises or consists of the amino acid sequence as shown in SEQ ID NO: 24, and the light chain variable region comprises or consists of the amino acid sequence as shown in SEQ ID NO: 28;
20) the heavy chain variable region comprises or consists of the amino acid sequence as shown in SEQ ID NO: 25, and the light chain variable region comprises or consists of the amino acid sequence as shown in SEQ ID NO: 27;
21) the heavy chain variable region comprises or consists of the amino acid sequence as shown in SEQ ID NO: 25, and the light chain variable region comprises or consists of the amino acid sequence as shown in SEQ ID NO: 28;
22) the heavy chain variable region comprises or consists of the amino acid sequence as shown in SEQ ID NO: 26, and the light chain variable region comprises or consists of the amino acid sequence as shown in SEQ ID NO: 27;
23) the heavy chain variable region comprises or consists of the amino acid sequence as shown in SEQ ID NO: 26, and the light chain variable region comprises or consists of the amino acid sequence as shown in SEQ ID NO: 28;
24) the heavy chain variable region comprises or consists of the amino acid sequence as shown in SEQ ID NO: 37, and the light chain variable region comprises or consists of the amino acid sequence as shown in SEQ ID NO: 42;
25) the heavy chain variable region comprises or consists of the amino acid sequence as shown in SEQ ID NO: 37, and the light chain variable region comprises or consists of the amino acid sequence as shown in SEQ ID NO: 43;
26) the heavy chain variable region comprises or consists of the amino acid sequence as shown in SEQ ID NO: 37, and the light chain variable region comprises or consists of the amino acid sequence as shown in SEQ ID NO: 44;
27) the heavy chain variable region comprises or consists of the amino acid sequence as shown in SEQ ID NO: 38, and the light chain variable region comprises or consists of the amino acid sequence as shown in SEQ ID NO: 42;
28) the heavy chain variable region comprises or consists of the amino acid sequence as shown in SEQ ID NO: 38, and the light chain variable region comprises or consists of the amino acid sequence as shown in SEQ ID NO: 43;
29) the heavy chain variable region comprises or consists of the amino acid sequence as shown in SEQ ID NO: 38, and the light chain variable region comprises or consists of the amino acid sequence as shown in SEQ ID NO: 44;
30) the heavy chain variable region comprises or consists of the amino acid sequence as shown in SEQ ID NO: 39, and the light chain variable region comprises or consists of the amino acid sequence as shown in SEQ ID NO: 42;
31) the heavy chain variable region comprises or consists of the amino acid sequence as shown in SEQ ID NO: 39, and the light chain variable region comprises or consists of the amino acid sequence as shown in SEQ ID NO: 43;
32) the heavy chain variable region comprises or consists of the amino acid sequence as shown in SEQ ID NO: 39, and the light chain variable region comprises or consists of the amino acid sequence as shown in SEQ ID NO: 44;
33) the heavy chain variable region comprises or consists of the amino acid sequence as shown in SEQ ID NO: 40, and the light chain variable region comprises or consists of the amino acid sequence as shown in SEQ ID NO: 42;
34) the heavy chain variable region comprises or consists of the amino acid sequence as shown in SEQ ID NO: 41, and the light chain variable region comprises or consists of the amino acid sequence as shown in SEQ ID NO: 42; or
35) the heavy chain variable region comprises or consists of the amino acid sequence as shown in SEQ ID NO: 41, and the light chain variable region comprises or consists of the amino acid sequence as shown in SEQ ID NO: 44.

In particular, the antibody or the antigen-binding fragment thereof of the present disclosure comprises at least a heavy chain variable region and a light chain variable region, both of which comprise the CDRs described above and spaced framework regions (FRs), and the domains are arranged as follows: FR1-CDR1-FR2-CDR2-FR3-CDR3-FR4. Further optionally, up to 10% amino acid sequence difference resulting from the "at least 90% identity" may exist in any framework region of the heavy chain variable region or the light chain variable region, or in any domain or sequence other than the heavy chain variable region and the light chain variable region in the antibody or the antigen-binding fragment thereof of the present disclosure. The difference may result from amino acid substitutions, deletions, or insertions at any position.

In some embodiments, the antibody or the antigen-binding fragment thereof further comprises a human or murine constant region, preferably a human or murine heavy chain constant region and/or a human or murine light chain constant region; preferably, the antibody or the antigen-binding fragment thereof comprises a heavy chain constant region of IgG, IgA, IgM, IgD, or IgE and/or a κ or λ light chain constant region.

In some embodiments, the antibody or the antigen-binding fragment thereof further comprises heavy chain constant region sequences and/or light chain constant region sequences derived from consensus sequences of human antibody germlines. In some embodiments, the heavy chain constant region is preferably derived from a constant region sequence of human IgG1, IgG2, IgG3, or IgG4. In a specific embodiment, the heavy chain constant region comprises or consists of the amino acid sequence as shown in SEQ ID NO: 45. In a specific embodiment, the light chain constant region comprises or consists of the amino acid sequence as shown in SEQ ID NO: 46.

It will be appreciated that sequence variants of these constant region domains may also be used, for example, those comprising one or more amino acid modifications, wherein the amino acid positions are identified by the EU index system of Kabat et al. (1991).

In a specific embodiment, the present disclosure provides an anti-DLL3 antibody or an antigen-binding fragment thereof that binds to DLL3, which comprises a heavy chain and/or a light chain, wherein:
1) the heavy chain comprises the amino acid sequence as shown in SEQ ID NO: 47, or a sequence comprising one or more amino acid substitutions (e.g., conservative substitutions), deletions, or insertions, or any combination thereof relative to SEQ ID NO: 47, or an amino acid sequence having at least 90%, 91%, 92%, 93%, 94%, 95%, 96%, 97%, 98%, or 99% identity to the amino acid sequence as shown in SEQ ID NO: 47, or consists of SEQ ID NO: 47; the light chain comprises the amino acid sequence as shown in SEQ ID NO: 48, or a sequence comprising one or more amino acid substitutions (e.g., conservative substitutions), deletions, or insertions, or any combination thereof relative to SEQ ID NO: 48, or an amino acid sequence having at least 90%, 91%, 92%, 93%, 94%, 95%, 96%, 97%, 98%, or 99% identity to the amino acid sequence as shown in SEQ ID NO: 48, or consists of SEQ ID NO: 48;
2) the heavy chain comprises the amino acid sequence as shown in SEQ ID NO: 47, or a sequence comprising one or more amino acid substitutions (e.g., conservative substitutions), deletions, or insertions, or any combination thereof relative to SEQ ID NO: 47, or an amino acid sequence having at least 90%, 91%, 92%, 93%, 94%, 95%, 96%, 97%, 98%, or 99% identity to the amino acid sequence as shown in SEQ ID NO: 47, or consists of SEQ ID NO: 47; the light chain comprises the amino acid sequence as shown in SEQ ID NO: 49, or a sequence comprising one or more amino acid substitutions (e.g., conservative substitutions), deletions, or insertions, or any combination thereof relative to SEQ ID NO: 49, or an amino acid sequence having at least 90%, 91%, 92%, 93%, 94%, 95%, 96%, 97%, 98%, or 99% identity to the amino acid sequence as shown in SEQ ID NO: 49, or consists of SEQ ID NO: 49;
3) the heavy chain comprises the amino acid sequence as shown in SEQ ID NO: 47, or a sequence comprising one or more amino acid substitutions (e.g., conservative substitutions), deletions, or insertions, or any combination thereof relative to SEQ ID NO: 47, or an amino acid sequence having at least 90%, 91%, 92%, 93%, 94%, 95%, 96%, 97%, 98%, or 99% identity to the amino acid sequence as shown in SEQ ID NO: 47, or consists of SEQ ID NO: 47; the light chain comprises the amino acid sequence as shown in SEQ ID NO: 50, or a sequence comprising one or more amino acid substitutions (e.g., conservative substitutions), deletions, or insertions, or any combination thereof relative to SEQ ID NO: 50, or an amino acid sequence having at least 90%, 91%, 92%, 93%, 94%, 95%, 96%, 97%, 98%, or 99% identity to the amino acid sequence as shown in SEQ ID NO: 50, or consists of SEQ ID NO: 50;
4) the heavy chain comprises the amino acid sequence as shown in SEQ ID NO: 51, or a sequence comprising one or more amino acid substitutions (e.g., conservative substitutions), deletions, or insertions, or any combination thereof relative to SEQ ID NO: 51, or an amino acid sequence having at least 90%, 91%, 92%, 93%, 94%, 95%, 96%, 97%, 98%, or 99% identity to the amino acid sequence as shown in SEQ ID NO: 51, or consists of SEQ ID NO: 51; the light chain comprises the amino acid sequence as shown in SEQ ID NO: 48, or a sequence comprising one or more amino acid substitutions (e.g., conservative substitutions), deletions, or insertions, or any combination thereof relative to SEQ ID NO: 48, or an amino acid sequence having at least 90%, 91%, 92%, 93%, 94%, 95%, 96%, 97%, 98%, or 99% identity to the amino acid sequence as shown in SEQ ID NO: 48, or consists of SEQ ID NO: 48;
5) the heavy chain comprises the amino acid sequence as shown in SEQ ID NO: 51, or a sequence comprising one or more amino acid substitutions (e.g., conservative substitutions), deletions, or insertions, or any combination thereof relative to SEQ ID NO: 51, or an amino acid sequence having at least 90%, 91%, 92%, 93%, 94%, 95%, 96%, 97%, 98%, or 99% identity to the amino acid sequence as shown in SEQ ID NO: 51, or consists of SEQ ID NO: 51; the light chain comprises the amino acid sequence as shown in SEQ ID NO: 49, or a sequence comprising one or more amino acid substitutions (e.g., conservative substitutions), deletions, or insertions, or any combination thereof relative to SEQ ID NO: 49, or an amino acid sequence having at least 90%, 91%, 92%, 93%, 94%, 95%, 96%, 97%, 98%, or 99% identity to the amino acid sequence as shown in SEQ ID NO: 49, or consists of SEQ ID NO: 49;
6) the heavy chain comprises the amino acid sequence as shown in SEQ ID NO: 51, or a sequence comprising one or more amino acid substitutions (e.g., conservative substitutions), deletions, or insertions, or any combination thereof relative to SEQ ID NO: 51, or an amino acid sequence having at least 90%, 91%, 92%, 93%, 94%, 95%, 96%, 97%, 98%, or 99% identity to the amino acid sequence as shown in SEQ ID NO: 51, or consists of SEQ ID NO: 51; the light chain comprises the amino acid sequence as shown in SEQ ID NO: 50, or a sequence comprising one or more amino acid substitutions (e.g., conservative substitutions), deletions, or insertions, or any combination thereof relative to SEQ ID NO: 50, or an amino acid sequence having at least 90%, 91%, 92%, 93%, 94%, 95%, 96%, 97%, 98%, or 99% identity to the amino acid sequence as shown in SEQ ID NO: 50, or consists of SEQ ID NO: 50;
7) the heavy chain comprises the amino acid sequence as shown in SEQ ID NO: 52, or a sequence comprising one or more amino acid substitutions (e.g., conservative substitutions), deletions, or insertions, or any combination thereof relative to SEQ ID NO: 52, or an amino acid sequence having at least 90%, 91%, 92%, 93%, 94%, 95%, 96%, 97%, 98%, or 99% identity to the amino acid sequence as shown in SEQ ID NO: 52, or consists of SEQ ID NO: 52; the light chain comprises the amino acid sequence as shown in SEQ ID NO: 48, or a sequence comprising one or more amino acid substitutions (e.g., conservative substitutions), deletions, or insertions, or any combination thereof relative to SEQ ID NO: 48, or an amino acid sequence having at least 90%, 91%, 92%, 93%, 94%, 95%, 96%, 97%, 98%, or 99% identity to the amino acid sequence as shown in SEQ ID NO: 48, or consists of SEQ ID NO: 48;
8) the heavy chain comprises the amino acid sequence as shown in SEQ ID NO: 52, or a sequence comprising one or more amino acid substitutions (e.g., conservative substitutions), deletions, or insertions, or any combination thereof relative to SEQ ID NO: 52, or an amino acid sequence having at least 90%, 91%, 92%, 93%, 94%, 95%, 96%, 97%, 98%, or 99% identity to the amino acid sequence as shown in SEQ ID NO: 52, or consists of SEQ ID NO: 52; the light chain comprises the amino acid sequence as shown in SEQ ID NO: 49, or a sequence comprising one or more amino acid substitutions (e.g., conservative substitutions), deletions, or insertions, or any combination thereof relative to SEQ ID NO: 49, or an amino acid sequence having at least 90%, 91%, 92%, 93%, 94%, 95%, 96%, 97%, 98%, or 99% identity to the amino acid sequence as shown in SEQ ID NO: 49, or consists of SEQ ID NO: 49;
9) the heavy chain comprises the amino acid sequence as shown in SEQ ID NO: 52, or a sequence comprising one or more amino acid substitutions (e.g., conservative substitutions), deletions, or insertions, or any combination thereof relative to SEQ ID NO: 52, or an amino acid sequence having at least 90%, 91%, 92%, 93%, 94%, 95%, 96%, 97%, 98%, or 99% identity to the amino acid sequence as shown in SEQ ID NO: 52, or consists of SEQ ID NO: 52; the light chain comprises the amino acid sequence as shown in SEQ ID NO: 50, or a sequence comprising one or more amino acid substitutions (e.g., conservative substitutions), deletions, or insertions, or any combination thereof relative to SEQ ID NO: 50, or an amino acid sequence having at least 90%, 91%, 92%, 93%, 94%, 95%, 96%, 97%, 98%, or 99% identity to the amino acid sequence as shown in SEQ ID NO: 50, or consists of SEQ ID NO: 50;
10) the heavy chain comprises the amino acid sequence as shown in SEQ ID NO: 53, or a sequence comprising one or more amino acid substitutions (e.g., conservative substitutions), deletions, or insertions, or any combination thereof relative to SEQ ID NO: 53, or an amino acid sequence having at least 90%, 91%, 92%, 93%, 94%, 95%, 96%, 97%, 98%, or 99% identity to the amino acid sequence as shown in SEQ ID NO: 53, or consists of SEQ ID NO: 53; the light chain comprises the amino acid sequence as shown in SEQ ID NO: 54, or a sequence comprising one or more amino acid substitutions (e.g., conservative substitutions), deletions, or insertions, or any combination thereof relative to SEQ ID NO: 54, or an amino acid sequence having at least 90%, 91%, 92%, 93%, 94%, 95%, 96%, 97%, 98%, or 99% identity to the amino acid sequence as shown in SEQ ID NO: 54, or consists of SEQ ID NO: 54;
11) the heavy chain comprises the amino acid sequence as shown in SEQ ID NO: 55, or a sequence comprising one or more amino acid substitutions (e.g., conservative substitutions), deletions, or insertions, or any combination thereof relative to SEQ ID NO: 55, or an amino acid sequence having at least 90%, 91%, 92%, 93%, 94%, 95%, 96%, 97%, 98%, or 99% identity to the amino acid sequence as shown in SEQ ID NO: 55, or consists of SEQ ID NO: 55; the light chain comprises the amino acid sequence as shown in SEQ ID NO: 56, or a sequence comprising one or more amino acid substitutions (e.g., conservative substitutions), deletions, or insertions, or any combination thereof relative to SEQ ID NO: 56, or an amino acid sequence having at least 90%, 91%, 92%, 93%, 94%, 95%, 96%, 97%, 98%, or 99% identity to the amino acid sequence as shown in SEQ ID NO: 56, or consists of SEQ ID NO: 56;
12) the heavy chain comprises the amino acid sequence as shown in SEQ ID NO: 57, or a sequence comprising one or more amino acid substitutions (e.g., conservative substitutions), deletions, or insertions, or any combination thereof relative to SEQ ID NO: 57, or an amino acid sequence having at least 90%, 91%, 92%, 93%, 94%, 95%, 96%, 97%, 98%, or 99% identity to the amino acid sequence as shown in SEQ ID NO: 57, or consists of SEQ ID NO: 57; the light chain comprises the amino acid sequence as shown in SEQ ID NO: 58, or a sequence comprising one or more amino acid substitutions (e.g., conservative substitutions), deletions, or insertions, or any combination thereof relative to SEQ ID NO: 58, or an amino acid sequence having at least 90%, 91%, 92%, 93%, 94%, 95%, 96%, 97%, 98%, or 99% identity to the amino acid sequence as shown in SEQ ID NO: 58, or consists of SEQ ID NO: 58; or
13) the heavy chain comprises the amino acid sequence as shown in SEQ ID NO: 55, or a sequence comprising one or more amino acid substitutions (e.g., conservative substitutions), deletions, or insertions, or any combination thereof relative to SEQ ID NO: 55, or an amino acid sequence having at least 90%, 91%, 92%, 93%, 94%, 95%, 96%, 97%, 98%, or 99% identity to the amino acid sequence as shown in SEQ ID NO: 55, or consists of SEQ ID NO: 55; the light chain comprises the amino acid sequence as shown in SEQ ID NO: 58, or a sequence comprising one or more amino acid substitutions (e.g., conservative substitutions), deletions, or insertions, or any combination thereof relative to SEQ ID NO: 58, or an amino acid sequence having at least 90%, 91%, 92%, 93%, 94%, 95%, 96%, 97%, 98%, or 99% identity to the amino acid sequence as shown in SEQ ID NO: 58, or consists of SEQ ID NO: 58.

In certain embodiments of the antibody according to any one of the foregoing, the antibody or the antigen-binding fragment thereof of the present disclosure is an anti-DLL3 antibody or an antigen-binding fragment thereof; preferably, the antibody is in any form, such as a monoclonal antibody, a single-chain antibody, a bifunctional antibody, a single-domain antibody, a nanobody, a fully or partially humanized antibody, or a chimeric antibody. In a specific embodiment, the antibody is a monoclonal antibody. In a preferred embodiment, the antibody is a chimeric antibody. In another more preferred embodiment, the antibody is a humanized antibody.

In certain embodiments of the antibody according to any one of the foregoing, the antibody is a full-length antibody.

In some embodiments, the anti-DLL3 antibody of the present disclosure is an intact antibody, e.g., an IgG1, IgG2, IgG3, or IgG4 antibody. In another embodiment, the anti-DLL3 antibody of the present disclosure encompasses only an antigen-binding moiety thereof, such as a Fab, Fab', Fab'-SH, (Fab')₂, Fv, scFv, BsFv, dsFv, or (dsFv)₂ fragment.

In some embodiments, the antigen-binding fragment is an antigen-binding fragment of an antibody or a half-antibody, such as a Fab, Fab', Fab'-SH, (Fab')₂, Fv, scFv, BsFv, dsFv, or (dsFv)₂ fragment; more preferably, the antibody is IgG.

In a second aspect, the present disclosure provides a nucleic acid molecule, which encodes any one of the antibodies or the antigen-binding fragments thereof according to the first aspect, or encodes a heavy chain CDR, a light chain CDR, a heavy chain variable region, a light chain variable region, a heavy chain, or a light chain comprised in the antibody or the antigen-binding fragment thereof. In some embodiments, the nucleic acid molecule may be an isolated nucleic acid molecule.

The nucleic acid molecule of the present disclosure can be cloned into a vector, so as to transform or transfect a host cell. Accordingly, in a third aspect, the present disclosure provides a vector, which comprises the nucleic acid molecule according to the second aspect. In one embodiment, the vector is an expression vector, such as a eukaryotic expression vector, a prokaryotic expression vector, an artificial chromosome, or a phage vector.

The vector or nucleic acid molecule of the present disclosure can be used to transform or transfect host cells for purposes such as preservation or antibody expression. Accordingly, in a fourth aspect, the present disclosure provides a host cell, which comprises the vector according to the third aspect or the nucleic acid molecule according to the second aspect, or the host cell is transformed or transfected with the nucleic acid molecule and/or the vector of the present disclosure. The host cell may be any prokaryotic or eukaryotic cell, such as a bacterial cell, an insect cell, a fungal cell, a plant cell, or an animal cell. In some embodiments, the host cell is prokaryotic, such as *Escherichia coli*. In other embodiments, the host cell is eukaryotic, such as a 293 cell, a CHO cell, a yeast cell, or a plant cell. In some embodiments, the host cell is another cell suitable for preparing an antibody or an antigen-binding fragment thereof.

The antibody or the antigen-binding fragment thereof provided in the present disclosure can be obtained using any method known in the art. For example, the heavy chain variable region and/or light chain variable region of the antibody, or the heavy chain and/or light chain of the antibody, may first be obtained from the nucleic acid molecule provided in the present disclosure, and then assembled into an antibody together with any optional other domains of the antibody. Alternatively, in a fifth aspect, the present disclosure provides a method for preparing the anti-DLL3 antibody or the antigen-binding fragment thereof described herein, which comprises expressing the antibody or the antigen-binding fragment thereof in the host cell described herein under conditions suitable for expression of the antibody or the antigen-binding fragment thereof, and recovering the expressed antibody or antigen-binding fragment thereof from the host cell.

In a sixth aspect, the present disclosure provides a conjugate, which comprises the antibody or the antigen-binding fragment thereof of the present disclosure and a conjugation moiety, wherein the conjugation moiety is another molecule; preferably, the conjugation moiety is a radioisotope, a fluorescent substance, a luminescent substance, a colored substance, an enzyme, a chemical drug, or the like.

The antibody or the antigen-binding fragment thereof, the nucleic acid molecule, the vector, the host cell, and/or the conjugate provided in the present disclosure may be comprised in a pharmaceutical composition, more particularly in a pharmaceutical formulation, for various purposes according to actual needs. Accordingly, in a seventh aspect, the present disclosure provides a pharmaceutical composition, which comprises the antibody or the antigen-binding fragment thereof according to the first aspect of the present disclosure, or the nucleic acid molecule, vector, host cell, or conjugate of the present disclosure, and a pharmaceutically acceptable carrier. In some embodiments of the present disclosure, the pharmaceutical composition further comprises a second therapeutic agent and/or an optional pharmaceutical auxiliary material, wherein the second therapeutic agent is selected from a cytokine, an antibody, a chemotherapeutic agent, and a small-molecule drug.

In an eighth aspect, the present disclosure provides an antibody-drug conjugate (ADC), which comprises the antibody or the antigen-binding fragment thereof according to the first aspect of the present disclosure. The antibody-drug conjugate further comprises a cytotoxic drug and a linker, wherein the linker links the antibody or the antigen-binding fragment thereof according to the first aspect of the present disclosure to the cytotoxic drug. The cytotoxic drug is selected from DX8951, MMAE, and Dxd, and the linker is a cleavable linker.

In a ninth aspect, the present disclosure provides use of the anti-DLL3 antibody or the antigen-binding fragment thereof of the present disclosure, or the nucleic acid molecule, vector, host cell, or conjugate of the present disclosure, the pharmaceutical composition of the present disclosure, or the antibody-drug conjugate of the present disclosure in preparing a medicament for treating a disease in a subject.

In one embodiment, any one of the antibodies or the antigen-binding fragments thereof according to the first aspect or the antibody-drug conjugate according to the eighth aspect is used for treating an autoimmune disease or cancer. Further, the disease is a DLL3-positive cancer. In one embodiment, the disease is, for example, a neuroendocrine tumor, lung cancer, prostate cancer, melanoma, gastric cancer, or the like. Preferably, the lung cancer is small cell lung cancer, and the neuroendocrine tumor is castration-resistant neuroendocrine prostate cancer, large cell neuroendocrine carcinoma, gastroenteropancreatic neuroendocrine neoplasm, or the like.

In a tenth aspect, the present disclosure provides a method for preventing and/or treating a disease in a subject, which comprises administering to a subject in need thereof an effective amount of the antibody or the antigen-binding fragment thereof described in the present disclosure, or the nucleic acid molecule, vector, host cell, or conjugate of the present disclosure, the pharmaceutical composition of the present disclosure, and/or the antibody-drug conjugate of the present disclosure. The disease includes a tumor or an autoimmune disease; preferably, the subject is a mammal; more preferably, the subject is a human.

In some embodiments, the disease includes a tumor or an autoimmune disease. Further, the disease is a DLL3-positive autoimmune disease or a DLL3-positive cancer. In one embodiment, the disease is, for example, a neuroendocrine tumor, lung cancer, prostate cancer, melanoma, gastric cancer, or the like. Preferably, the lung cancer is small cell lung cancer, and the neuroendocrine tumor is castration-resistant neuroendocrine prostate cancer, large cell neuroendocrine carcinoma, gastroenteropancreatic neuroendocrine neoplasm, or the like.

In some embodiments, the present disclosure provides the antibody or the antigen-binding fragment thereof disclosed herein or the nucleic acid molecule, vector, host cell, conjugate, pharmaceutical composition, or antibody-drug conjugate of the present disclosure for use as a medicament.

In some embodiments, the present disclosure provides the antibody or the antigen-binding fragment thereof disclosed herein or the nucleic acid molecule, vector, host cell, conjugate, pharmaceutical composition, or antibody-drug conjugate of the present disclosure for use in therapy.

In an eleventh aspect, the present disclosure provides a kit, which comprises the antibody or the antigen-binding fragment thereof or the conjugate of the present disclosure.

In a twelfth aspect, the present disclosure provides a method for detecting the presence or level of DLL3 in a sample, which comprises the steps of contacting the antibody or the antigen-binding fragment thereof or the conjugate of the present disclosure with the sample, and detecting whether the antibody or the antigen-binding fragment thereof or the conjugate forms a complex with DLL3.

The present disclosure is further illustrated by the following drawings and specific embodiments. However, these drawings and specific embodiments should not be considered as limiting the scope of the present disclosure, and changes that are readily conceived by those skilled in the art will be included within the spirit of the present disclosure and the protection scope of the appended claims.

### BRIEF DESCRIPTION OF THE DRAWINGS

FIG. 1 shows the curves for the non-specific binding activity of anti-DLL3 murine monoclonal antibodies to the human DLL1 protein.
FIG. 2 shows the curves for the non-specific binding activity of anti-DLL3 murine monoclonal antibodies to the human DLL4 protein.
FIG. 3 shows the curve for the killing of HEK293-cynoDLL3 cells by a humanized anti-DLL3 antibody-ADC.
FIG. 4 shows the curve for the killing of NCI-H524 cells by a humanized anti-DLL3 antibody-ADC.
FIG. 5 shows the curve for the inhibition of NCI-H524 tumor cell growth by a humanized anti-DLL3 antibody-ADC.
FIG. 6 shows the curve for the rate of change in the body weight of mice in the NCI-H524 tumor model inhibited by a humanized anti-DLL3 antibody-ADC.

### DETAILED DESCRIPTION OF THE INVENTION

All publications, patent applications, patents, and other references mentioned herein are incorporated by reference in their entirety. In addition, the materials, methods, and examples described herein are illustrative only and are not intended to be limiting. Other features, objectives, and advantages of the present disclosure will be apparent from the description and drawings, and from the appended claims. Unless otherwise defined, all technical and scientific terms used herein have the same meaning as commonly understood by those of ordinary skill in the art. For the objectives of the present disclosure, the following terms are defined below.

### Definitions

Unless otherwise specified, the practice of the present disclosure will employ conventional techniques of molecular biology (including recombinant techniques), microbiology, cell biology, biochemistry, and immunology, which are within the skill of the art.

The term "about", when used in conjunction with a numerical value, means encompassing numerical values within a range having a lower limit of 5% less than the specified numerical value and an upper limit of 5% greater than the specified numerical value.

The term "and/or" shall be understood to mean any one of the optional items or any combination of two or more of the optional items.

As used herein, the term "comprise" or "include" means including the stated elements, integers, or steps, but not excluding any other elements, integers, or steps. Herein, when the term "comprise" or "include" is used, it also encompasses the case consisting of the stated elements, integers, or steps, unless otherwise indicated.

The term "antibody" is used herein in its broadest sense, referring to a protein that contains an antigen-binding site.

The term "immunoglobulin" refers to a protein having the structure of a naturally occurring antibody, and may generally be used interchangeably with the term "antibody" in the present application. An IgG immunoglobulin is a heterotetrameric glycoprotein composed of two light chains and two heavy chains linked by disulfide bonds. From the N-terminus to the C-terminus, each immunoglobulin heavy chain has one heavy chain variable region (VH), also referred to as a heavy chain variable domain, followed by three heavy chain constant domains (CH1, CH2, and CH3). Similarly, from the N-terminus to the C-terminus, each immunoglobulin light chain has one light chain variable region (VL), also referred to as a light chain variable domain, followed by one light chain constant domain (CL). In an IgG molecule, the VH-CH1 of the heavy chain is typically paired with the VL-CL of the light chain to form a Fab fragment that specifically binds to an antigen. Accordingly, an IgG immunoglobulin basically consists of two Fab molecules and two dimerized Fc regions linked via an immunoglobulin hinge region. Immunoglobulin heavy chains can be assigned to one of 5 classes based on the type of their constant regions, designated α (IgA), δ (IgD), ε (IgE), γ (IgG), or µ (IgM); certain of these classes can be further divided into subclasses, such as γ1 (IgG1), γ2 (IgG2), γ3 (IgG3), γ4 (IgG4), α1 (IgA1), and α2 (IgA2). Immunoglobulin light chains can also be classified into one of two types based on the amino acid sequence of their constant domains, designated κ and λ.

The terms "DLL3" and "Delta-like ligand 3" as used herein refer to any native DLL3 produced by the expression of DLL3 in cells. Unless otherwise specified, the term encompasses DLL3 from any vertebrate source, such as mammals (e.g., primates (e.g., humans and cynomolgus monkeys) and rodents (e.g., mice and rats)).

"Complementarity determining region", or "CDR region", or "CDR", or "hypervariable region", refers to a region within an antibody variable domain that is highly variable in sequence, forms structurally defined loops ("hypervariable loops"), and/or contains antigen-contacting residues ("antigen-contacting sites"). CDRs are primarily responsible for binding to antigen epitopes.

A variety of schemes are known in the art for identifying CDR sequences within a given VH or VL amino acid sequence: Kabat complementarity determining regions (CDRs) are defined based on sequence variability and are the most commonly used (Kabat et al., Sequences of Proteins of Immunological Interest, 5th ed., Public Health Service, National Institutes of Health, Bethesda, Md. (1991)); Chothia refers to the position of a structural loop (Chothia et al., (1987) J. Mol. Biol. 196:901-917; Chothia et al., (1989) Nature 342:877-883); AbM CDRs represent a compromise between Kabat CDRs and Chothia structural loops and are adopted by the AbM antibody modeling software of Oxford Molecular; "Contact" CDRs are defined based on analysis of available complex crystal structures. The residues corresponding to each of those CDRs under different CDR definition schemes are described as follows.

| CDR | Kabat scheme | AbM scheme | Chothia scheme | Contact scheme |
|---|---|---|---|---|
| CDR1 | L24-L34 | L24-L34 | L26-L32 | L30-L36 |
| CDR2 | L50-L56 | L50-L56 | L50-L52 | L46-L55 |
| CDR3 | L89-L97 | L89-L97 | L91-L96 | L89-L96 |
| CDR1 | H31-H35B | H26-H35B | H26-H32 | H30-H35B |

| (Kabat numbering scheme) | | | | |
|---|---|---|---|---|
| CDR1 | H31-H35 | H26-H35 | H26-H32 | H30-H35 |

| (Chothia numbering scheme) | | | | |
|---|---|---|---|---|
| CDR2 | H50-H65 | H50-H58 | H53-H55 | H47-H58 |
| CDR3 | H95-H102 | H95-H102 | H96-H101 | H93-H101 |
| (Kabat numbering scheme) | | | | |

When it comes to defining an antibody by specific CDR sequences as defined in the present disclosure, the scope of the antibody also encompasses such antibodies whose variable region sequences contain the specific CDR sequences, wherein the claimed CDR boundaries differ from the specific CDR boundaries defined in the present disclosure due to the adoption of a different scheme (e.g., different assignment system rules or combinations thereof).

The boundaries of the CDRs of the antibody of the present disclosure may be manually evaluated and determined according to any scheme in the art or a combination thereof. Unless otherwise specified, the term "CDR" or "CDR sequence" used in the present disclosure encompasses CDR sequences defined by any one of the schemes described above.

The terms "antigen-binding site" and "antigen-binding domain" are used interchangeably, referring to a region of an antibody molecule that actually binds to an antigen. Antigen-binding sites include, but are not limited to, forms such as Fv, a Fab fragment, Fab', Fab'-SH, F(ab')₂, a single-chain antibody molecule (e.g., scFv), and VHH.

The term "variable region" or "variable domain" of an antibody refers to a domain of an antibody heavy chain or light chain that participates in the binding of the antibody to an antigen. Antibody variable regions can be further divided into hypervariable regions (i.e., complementarity determining regions (CDRs)) and relatively conserved regions interspersed among the hypervariable regions (i.e., framework regions (FRs)). In the case of an IgG immunoglobulin, the heavy chain variable region or light chain variable region comprises, sequentially from the N-terminus to the C-terminus, FR1-CDR1-FR2-CDR2-FR3-CDR3-FR4. In the case of a heavy-chain antibody (also referred to herein as a nanobody), such as a heavy-chain antibody from Camelidae, the antigen-binding site consists of a single VH domain (i.e., a "VHH" domain). The VHH of a native heavy-chain antibody shares a similar structure with the heavy chain variable region of a native IgG antibody, namely comprising four conserved framework regions (FRs) and three complementarity determining regions (CDRs).

"Heavy chain constant region domain" or "heavy chain constant region" refers to a constant region domain originated from, obtained from, or derived from an immunoglobulin heavy chain, including heavy chain constant regions CH1, CH2, and CH3, and optionally heavy chain constant region CH4, which are covalently linked sequentially from the N-terminus to the C-terminus. In most cases, the heavy chain constant regions CH1 and CH2 are linked via a heavy chain hinge region; where appropriate, they may also be linked via a flexible linker.

The term "EC₅₀", also known as "half maximal effective concentration", refers to the concentration of a drug, antibody, or toxic agent that induces a response 50% between the baseline and the maximum value after a specified exposure time.

As used herein, the term "binding" or "specific binding" means that the binding is selective for the antigen and can be distinguished from unwanted or non-specific interactions. The ability of an antigen-binding site to bind to a specific antigen can be determined by enzyme-linked immunosorbent assay (ELISA) or conventional binding assays known in the art.

"Affinity" or "binding affinity" refers to the intrinsic binding ability reflecting the interaction between members of a binding pair. The affinity of molecule X for its binding partner Y can be represented by the equilibrium dissociation constant (KD), which is the ratio of the dissociation rate constant to the association rate constant (k_{dis} and kₒₙ, respectively). Binding affinity can be measured by common methods known in the art.

Amino acid mutations can be amino acid substitutions, deletions, insertions, and/or additions. In some embodiments, the amino acid mutation is a substitution of one or more amino acids, such as a single amino acid substitution or a combination of multiple amino acid substitutions. Amino acid deletions and insertions include deletions and insertions at the amino- and/or carboxyl-terminus of a polypeptide sequence, as well as deletions and insertions within the polypeptide sequence. Amino acid substitutions of the present disclosure optionally include conservative substitutions of amino acids.

The "percent (%) identity" of an amino acid sequence refers to the percentage of amino acid residues in a candidate sequence that are identical to the amino acid residues of a specific amino acid sequence as shown in this specification, after aligning the candidate sequence with the specific amino acid sequence as shown in this specification and introducing gaps, if necessary, to achieve the maximum percent sequence identity, without regarding any conservative substitutions as part of the sequence identity. In some embodiments, the present disclosure contemplates variants of the antibody molecules of the present disclosure, and the variants have a substantial degree of identity relative to the antibody molecules and sequences thereof specifically disclosed herein, for example, an identity of at least 80%, 85%, 90%, 95%, 97%, 98%, 99%, or higher. The variants may comprise conservative modifications.

For polypeptide sequences, "conservative modifications" include substitutions, deletions, or additions to a polypeptide sequence that result in the replacement of an amino acid with another chemically similar amino acid. Tables of conservative substitutions providing functionally similar amino acids are well known in the art. Variants having such conservative modifications are additional to, and do not exclude, polymorphic variants, interspecies homologs, and alleles of the present disclosure. The following 8 groups contain amino acids that are conservative substitutions for one another: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M) (see, e.g., Creighton, Proteins (1984)). In some embodiments, the term "conservative sequence modification" is used to refer to an amino acid modification that does not significantly affect or alter the binding characteristics of an antibody comprising the amino acid sequence.

The terms "individual" and "subject" are used interchangeably and refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, the individual is a human.

The term "treatment" refers to clinical intervention intended to alter the natural course of a disease in an individual undergoing treatment. Desired therapeutic effects include, but are not limited to, preventing the onset or recurrence of a disease, alleviating symptoms, reducing any direct or indirect pathological consequences of a disease, preventing metastasis, slowing the rate of disease progression, ameliorating or palliating the disease state, and relieving or improving prognosis. In some embodiments, the antibody molecules of the present disclosure are used to delay the onset or slow the progression of a disease.

The term "prevention" includes the inhibition of the occurrence or development of a disease or condition or symptoms of a specific disease or condition. In some embodiments, subjects with a family history of cancer are candidates for a prophylactic regimen. Generally, in the context of cancer, the term "prevention" refers to the administration of a medicament before the appearance of signs or symptoms of cancer, particularly in subjects at risk of developing cancer.

The term "effective amount" refers to such an amount or dosage of the antibody or composition of the present disclosure that produces an expected effect in a patient in need of treatment or prevention after administration to the patient in a single or multiple doses. The effective amount can be easily determined by an attending physician as a person skilled in the art by considering a variety of factors as follows: such as the mammalian species; body weight, age, and general health status; the specific disease involved; the extent or severity of the disease; response in an individual patient; specific antibody administered; mode of administration; bioavailability characteristics of the administered formulation; selected administration regimen; and use of any concomitant therapy.

The term "therapeutically effective amount" refers to an amount effective to achieve a desired therapeutic outcome at a necessary dose for a necessary period of time. The therapeutically effective amount of an antibody, antibody fragment, or composition may vary according to a variety of factors such as disease state, the age, gender, and body weight of an individual, and the ability of the antibody or antibody portion to elicit a desired response in the individual. The therapeutically effective amount is also such an amount at which any toxic or detrimental effects of the antibody, antibody fragment, or composition are outweighed by the therapeutic benefits thereof. Relative to an untreated subject, the "therapeutically effective amount" preferably inhibits a measurable parameter by at least about 20%, more preferably at least about 40%, even more preferably at least about 50%, 60%, or 70%, and still more preferably at least about 80% or 90%.

The term "prophylactically effective amount" refers to an amount effective to achieve a desired prophylactic outcome at a necessary dose for a necessary period of time. Generally, since prophylactic doses are administered prior to or at an early stage of a disease in a subject, the prophylactically effective amount will be lower than the therapeutically effective amount.

The term "pharmaceutical composition" refers to such a composition that exists in a form permitting the biological activity of the active ingredients contained therein to be effective, and contains no additional ingredients that exert unacceptable toxicity to a subject administered the composition. The pharmaceutical composition includes, but is not limited to: carriers, diluents, adjuvants, excipients, preservatives, fillers, disintegrants, wetting agents, emulsifiers, suspensions, sweeteners, flavoring agents, fragrances, antibacterial agents, antifungal agents, lubricants, dispersants, thermosensitive materials, temperature regulators, adhesives, stabilizers, suspending agents, and the like.

The term "antibody-drug conjugate (ADC)" refers to a construct in which a biologically active small-molecule drug is linked to an antibody via a chemical linker; the antibody serves as a carrier to deliver the small-molecule drug to target cells in a targeted manner.

### Polynucleotides, Vectors, and Hosts

The present disclosure provides a nucleic acid molecule encoding any of the above antibody molecules or antigen-binding fragments thereof. Polynucleotide sequences encoding the antibody molecules or the antigen-binding fragments thereof of the present disclosure may be generated by methods well known in the art, via *de novo* solid-phase DNA synthesis or genetic engineering approaches. Furthermore, the polynucleotide and the nucleic acid of the present disclosure may comprise a segment encoding a secretory signal peptide, which is operably linked to a segment encoding the antibody molecule or the antigen-binding fragment thereof of the present disclosure, so as to direct the secretory expression of the antibody molecule or the antigen-binding fragment thereof of the present disclosure.

The present disclosure also provides a vector comprising the nucleic acid molecule of the present disclosure. In one embodiment, the vector is an expression vector, such as a eukaryotic expression vector or a prokaryotic expression vector. "Expression vector" refers to a vector comprising a recombinant polynucleotide, which comprises an expression control sequence operatively linked to the nucleotide sequence to be expressed. An expression vector contains sufficient cis-acting elements for expression; other elements required for expression may be supplied by a host cell or provided in an *in vitro* expression system. Expression vectors include all those known in the art, including cosmids, plasmids (e.g., naked or contained in liposomes), and viruses (e.g., lentiviruses, retroviruses, adenoviruses, and adeno-associated viruses) into which a recombinant polynucleotide is incorporated.

The present disclosure further provides prokaryotic and eukaryotic host cells comprising the nucleic acid molecule or the vector. Host cells include "transformants" and "transformed cells", which include primary transformed cells and progeny derived therefrom. A host cell may be any type of cellular system that can be used to produce the antibody molecules of the present disclosure, including eukaryotic cells such as mammalian cells, insect cells, and yeast cells, as well as prokaryotic cells such as *Escherichia coli* cells. Host cells include cultured cells, and also cells within transgenic animals, transgenic plants, or cultured plant or animal tissues. Host cells suitable for replicating and supporting the expression of the antibody molecules or the antigen-binding fragments thereof of the present disclosure are well known in the art. Such cells may be transfected or transduced with a specific expression vector, and large quantities of vector-containing cells may be grown for inoculation into large-scale fermenters to obtain sufficient yields of the antibody molecules.

### Pharmaceutical Compositions and Pharmaceutical Formulations

The present disclosure further includes compositions (including pharmaceutical compositions or pharmaceutical formulations) comprising the antibody molecule or the antigen-binding fragment thereof of the present disclosure, and compositions comprising a nucleic acid molecule encoding the antibody molecule or the antigen-binding fragment thereof of the present disclosure. These compositions may also optionally comprise suitable pharmaceutical auxiliary materials, such as pharmaceutically acceptable carriers and pharmaceutical excipients known in the art, including buffer agents.

The pharmaceutical compositions or formulations of the present disclosure may further comprise one or more additional active ingredients, or be used in combination with one or more additional active ingredients. The active ingredients are those required for the specific indication to be treated, and are preferably active ingredients possessing complementary activities that do not adversely affect each other. For example, it is desirable to further provide additional active ingredients for the treatment of autoimmune diseases, which are suitably present in combination in an amount effective for the intended use.

### Treatment Method and Use

As used herein, the terms "individual" and "subject" are used interchangeably and refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, the subject is a human.

In one embodiment, the present disclosure provides a method for treating a disease in a subject in need thereof, which comprises administering to the subject a therapeutically effective amount of the antibody or the antigen-binding fragment thereof of the present disclosure, or the nucleic acid molecule, vector, host cell, or conjugate of the present disclosure, the pharmaceutical composition of the present disclosure, or the antibody-drug conjugate of the present disclosure. In one embodiment, the disease is an autoimmune disease or cancer. Further, the disease is a DLL3-positive cancer. In one embodiment, the disease is, for example, a neuroendocrine tumor, lung cancer, prostate cancer, melanoma, gastric cancer, or the like. Preferably, the lung cancer is small cell lung cancer, and the neuroendocrine tumor is castration-resistant neuroendocrine prostate cancer, large cell neuroendocrine carcinoma, gastroenteropancreatic neuroendocrine neoplasm, or the like.

The antibody molecule or the antigen-binding fragment thereof of the present disclosure (as well as the pharmaceutical composition comprising the same) may be administered by any suitable route, including parenteral administration, intrapulmonary administration, intranasal administration, and, if local treatment is required, intralesional administration. Parenteral infusion includes intramuscular, intravenous, intraarterial, intraperitoneal, or subcutaneous administration. Depending in part on whether administration is for short-term or long-term use, administration may be performed via any suitable route, such as by injection, for example, intravenous or subcutaneous injection. Various administration regimens are encompassed herein, including, but not limited to, single administration or multiple administrations at multiple time points, bolus administration, and pulsed infusion.

In order to prevent or treat a disease, a suitable dosage of the antibody molecule or the antigen-binding fragment thereof of the present disclosure (when used alone or in combination with one or more additional therapeutic agents) will depend on the type of the disease to be treated, the severity and progression of the disease, whether the administration is for prophylactic or therapeutic purposes, prior treatment, the patient's clinical medical history, and the judgment of the attending physician.

The following examples are described to facilitate understanding of the present disclosure. The examples are not intended to be and should not be interpreted in any way as limiting the protection scope of the present disclosure.

### EXAMPLES

Unless otherwise specified, all experimental methods mentioned in the following examples are conventional methods adopting default parameters, procedures, and the like commonly used in the art; all experimental materials used are commercially available products unless otherwise specified. Examples without specified techniques or conditions are performed in accordance with techniques or conditions described in the literature in the art or in accordance with corresponding product instructions. Reagents or instruments without specified manufacturers used herein are conventional products that can be purchased through formal channels.

### Example 1: Preparation of Anti-Human DLL3 Mouse Hybridomas

Mice were immunized with the DLL3-Fc protein (Acro # DL3-H5255), fusion was performed with SP2/0 cells, and positive clones were screened by ELISA.

The specific procedures were as follows:
**Animal immunization:** The DLL3-hFc protein was used as the immunogen. Female Balb/c and SJL mice (supplier: SLAC), aged 6-8 weeks, were immunized with the protein, and the route of injection was intraperitoneal injection. The primary immunization was performed at 50 µg/mouse, and the immunogen was emulsified with Complete Freund's Adjuvant (Sigma, F5581). The booster immunization was performed at 25 µg/mouse, and the immunogen was emulsified with Incomplete Freund's Adjuvant (Sigma, F5506). The time interval between the primary protein immunization and the first booster immunization was generally two weeks, and the time interval between the first and second as well as subsequent booster immunizations was three weeks. 10 days after the final immunization, a final boost was performed by intraperitoneal injection of the DLL3-hFc protein at 25 µg/mouse without adjuvant. 3 days later, the mouse spleens were harvested for cell fusion.

**Cell fusion and selective culture of hybridoma cells:** Mouse splenocytes were electrofused with SP2/0 cells (ATCC No. CRL-1581) at a ratio of 2:1 (BTX electrofusion instrument: ECM2001+). Cells were cultured in a 96-well culture plate with a HAT culture medium (Sigma-Aldrich, Cat. No.: H0262). 10 days later, antibodies in the hybridoma cell supernatant were screened.

**Screening of positive clones**: The cultured cell supernatant was collected and subjected to an ELISA binding assay. Positive wells of the cell culture supernatant were selected and subjected to clonal culture in a 96-well culture plate with an HT culture medium (Sigma-Aldrich, Cat. No.: H0137). 7-10 days later, visible clones appeared, and the supernatant was collected for antibody detection. Observation was performed under an inverted microscope. Positive wells with only a single clone growth were selected for expansion culture and cryopreservation. The supernatant was collected after culture in a serum-free culture medium (SFM, Thermo, 12045076) and purified for subsequent detection.

### ELISA binding assay:

The supernatant, positive control (fused mouse serum or Rovalpituzumab-mIgG), or negative control (HAT culture medium or mIgG) was added at 50 µL/well onto a 96-well flat-bottom assay plate (Corning, 9018) coated with hDLL3-His (Acro, DL3-H52H4) or cynoDLL3-His (Acro, DL3-C52H3). The plate was incubated in an incubator at 37 °C with carbon dioxide for 60 min, and then taken out and washed three times with PBST. A secondary antibody (Anti-Mouse IgG (Fc specific)-Peroxidase antibody produced in goat, Sigma, A0168) was added, and the plate was incubated again in the incubator at 37 °C with carbon dioxide for 30 min, and then taken out and washed three times with PBST. TMB (for hDLL3 detection: Huzhou InnoReagents Co., Ltd., TMB-S-003; for cynoDLL3 detection: Beyotime, P0209) was added at 100 µL/well for color development for 15 min. A stop solution (for hDLL3 detection: sulfuric acid; for cynoDLL3 detection: Solarbio, C1058) was added at 50 µL/well to terminate the reaction. The OD values were read, and the results are shown in Table 1.

**Table 1. OD values of binding between positive clone hybridoma supernatants and hDLL3 as well as cynoDLL3 proteins**

| **Experimental method** | **ELISA** | |
|---|---|---|
| **Sample** | **hDLL3 protein** | **cynoDLL3 protein** |
| 177D10F11 | 0.815 | 0.81 |
| 135A9B10 | 3.46 | 1.294 |
| 137G3D11 | 2.49 | 0.297 |
| Negative control* | 0.063 | 0.048 |
| Positive control# | 1.65 | 0.646 |

| | | |
|---|---|---|
| Note: In Table 1 and other tables of the present disclosure, N/A for ELISA and FACS assay results is defined as no binding activity or poor binding activity, where no valid EC₅₀ value was obtained; N/T stands for not tested. *: HAT culture medium or mIgG; #: mouse antibody serum prior to cell fusion or Rovalpituzumab-mIgG. | | |

### Example 2: Purification and Functional Identification of Anti-Human DLL3 Murine Monoclonal Antibodies

Hybridoma cells with binding activity for the hDLL3-His protein in Table 1 were cultured in a serum-free culture medium. 10 days later, the culture supernatant was collected. Murine monoclonal antibodies were purified using a Protein A column (Bestchrom (Shanghai) Biosciences Co., Ltd., Cat. No.: AA0272) to obtain purified monoclonal antibodies. The binding activity, non-specific binding, ADC killing activity, and internalization efficiency of the monoclonal antibodies were determined.

### 2.1 Binding activity of anti-human DLL3 murine monoclonal antibody for human and cynomolgus monkey DLL3 proteins

The enzyme-linked immunosorbent assay (ELISA) was used to detect the binding activity of an anti-human DLL3 murine monoclonal antibody for the human DLL3 protein and the cynomolgus monkey DLL3 protein, respectively. A high-adsorption 96-well microplate was coated with 1 µg/mL human DLL3-His protein (Acro, DL3-H52H4) or cynomolgus monkey DLL3-His protein (Acro, DL3-C52H3). The plate was blocked with a 1% BSA-PBS blocking solution at 37 °C for 1 h. Antibody concentration gradient diluents (initial concentration: 40 nM, 4-fold serial dilution) were prepared with 1% BSA-PBST (0.05% PBST). The antibody was added to the blocked 96-well microplate at 100 µL/well, and the plate was incubated at 37 °C for 1 h. After 3 washes with 0.05% PBST, an HRP-labeled secondary antibody (Sigma, A0168) diluted with 1% BSA-PBS was added to the microplate at 100 µL/well, and the plate was incubated at 37 °C for 1 h. After 3 washes with PBST, color development was performed with TMB (BD, 555214) and terminated with a stop solution (Solarbio, C1058). The absorbance values at 450 nm and 630 nm were then measured with a microplate reader. The OD450-OD630 values were fitted using a four-parameter logistic (4PL) model, and the EC₅₀ value of each antibody was calculated.

As shown in Table 2, at the molecular level, the binding activity (EC₅₀) of the anti-human DLL3 murine monoclonal antibody 177D10F11 for human and cynomolgus monkey DLL3 proteins was superior to or comparable to that of the positive control antibody Rovalpituzumab-mIgG.

**Table 2. EC₅₀ values for binding of anti-human DLL3 murine monoclonal antibody to human and cynomolgus monkey DLL3 proteins**

| **Sample** | **Human DLL3** | **Cynomolgus monkey DLL3** |
|---|---|---|
| Rovalpituzumab-mIgG | 0.167 | 0.124 |
| 177D10F11 | 0.113 | 0.125 |
| mIgG | N/A | N/A |

| | | |
|---|---|---|
| Note: In Table 2 and other tables of the present disclosure, the EC₅₀ of Rovalpituzumab-mIgG represents the average value of multiple experiments; the unit of all EC₅₀ values is nM. | | |

### 2.2 Binding activity of anti-human DLL3 murine monoclonal antibody to human and cynomolgus monkey DLL3 on cell surface

Flow cytometry (FACS) was used to detect the binding activity of an anti-human DLL3 murine monoclonal antibody for human and cynomolgus monkey DLL3 antigens on the surface of CT26 cells overexpressing human DLL3 (CT26-hDLL3, Kyinno Biotechnology, KC-1129), SHP77 cells (ATCC, CRL-2195), and HEK293T cells overexpressing cynomolgus monkey DLL3 (HEK293-cynoDLL3, Kyinno Biotechnology, KC-0294), respectively. CT26-hDLL3 cells and HEK293-cynoDLL3 cells were obtained by digestion with conventional trypsin (Gibco, 25200072), and SHP77 cells were taken out from a culture flask. The above cells were centrifuged and counted, added to a 96-well U-bottom plate, and centrifuged at 300 g for 5 min, and the supernatant was discarded. 100 µL of an serial dilutions of antibody prepared with an FACS buffer was added, and the mixture was mixed well, followed by incubation at 4 °C for 60 min. After 3 washes with an FACS buffer, the AF647-anti-mIgG (H+L) secondary antibody (Invitrogen, A31571; 1:1000) diluted with an FACS buffer was added at 100 µL/well, and the plate was incubated at 4 °C for 1 h. After 3 washes with an FACS buffer, an FACS buffer was added at 100 µL/well to resuspend the cells. Fluorescence signals were detected by a flow cytometer (BECKMAN COULTER cytoFLEX). The binding activity was fitted using a four-parameter logistic (4PL) mode to calculate the EC₅₀ value of each antibody.

As shown in Table 3, the anti-human DLL3 murine monoclonal antibody 177D10F11 exhibited good binding activity for cells expressing human DLL3 and cells expressing cynomolgus monkey DLL3.

**Table 3. Binding activity of anti-human DLL3 murine monoclonal antibody to human and cynomolgus monkey DLL3 on cell surface**

| **Sample** | **CT26 hDLL3** | **SHP77** | **cynoDLL3 HEK293** |
|---|---|---|---|
| | EC_{50,} nM | EC_{50,} nM | EC_{50,} nM |
| Rovalpituzumab-mIgG | 0.083 | 0.016 | 0.033 |
| 177D10F11 | 0.075 | 0.011 | 0.061 |
| mIgG | N/A | N/A | N/A |

| | | | |
|---|---|---|---|
| Note: In Table 3 and other tables of the present disclosure, N/A for the results of the binding activity of monoclonal antibodies to DLL3 from different sources is defined as no valid EC₅₀ value being obtained by fitting. | | | |

### 2.3 Binding activity of anti-human DLL3 murine monoclonal antibodies to mouse DLL3

The enzyme-linked immunosorbent assay (ELISA) was used to detect the binding activity of anti-human DLL3 murine monoclonal antibodies to the mouse DLL3 protein.

A high-adsorption 96-well microplate was coated with 2 µg/mL mouse DLL3 protein (CUSABIO, CSB-MP006948MO1) and incubated overnight at 4 °C. The next day, the plate was blocked with a 1% BSA-PBS blocking solution at 37 °C for 1 h. Serial dilutions of antibody were prepared with 1% BSA-PBST and were each added to the blocked 96-well microplate at 100 µL/well, followed by incubation at 37 °C for 1 h. After 3 washes with 0.05% PBST, an HRP-labeled secondary antibody (Sigma, A0168) diluted with 1% BSA-PBST was added at 100 µL/well, and the plate was incubated at 37 °C for 1 h. After 3 washes with PBST, color development was performed with TMB (BD, 555214) and terminated with a stop solution (Solarbio, C1058). The absorbance values at 450 nm and 630 nm were then measured with a microplate reader. The OD450-OD630 values were fitted using a four-parameter logistic (4PL) model, and the EC₅₀ value of each antibody was calculated.

As shown in Table 4, the anti-human DLL3 murine monoclonal antibodies 135A9B10 and 177D10F11 exhibited good binding activity for mouse DLL3.

**Table 4. Binding activity of anti-human DLL3 murine monoclonal antibodies to mouse DLL3 antigen**

| **Sample** | **mouse DLL3, EC₅₀, nM** |
|---|---|
| Rovalpituzumab-mIgG | 0.127 |
| 135A9B10 | 0.110 |
| 177D10F11 | 0.139 |
| mIgG | N/A |

### 2.4 Assay on non-specific binding of anti-human DLL3 murine monoclonal antibodies to human DLL1 protein and human DLL4 protein by ELISA

A high-adsorption 96-well microplate was coated with 1 µg/mL human DLL1 protein (Acro, DL1-H52H8) or human DLL4 protein (Acro, DL4-H5227) and incubated at 37 °C for 1 h. After 3 washes with 0.05% PBST, the plate was blocked with a 1% BSA-PBS blocking solution at 37 °C for 1 h. After 3 washes with 0.05% PBST, serial dilutions of antibody (initial concentration: 30 µg/mL, 4-fold dilution) prepared with 1% BSA-PBST were each added at 100 µL/well, and the plate was incubated at 37 °C for 1 h. After 3 washes with 0.05% PBST, an HRP-labeled secondary antibody diluted with 1% BSA-PBST (murine anti-DLL3 monoclonal antibody: Sigma, A0168; positive control anti-DLL1 and anti-DLL4 antibodies: Invitrogen, A16116; diluted at 1:10000) was added at 100 µL/well, and the plate was incubated at 37 °C for 1 h. After 3 washes with PBST, color development was performed with TMB (BD, 555214) and terminated with a stop solution (Solarbio, C1058). The absorbance values at 450 nm were then measured with a microplate reader, and the OD450 values were fitted using a four-parameter logistic (4PL) model.

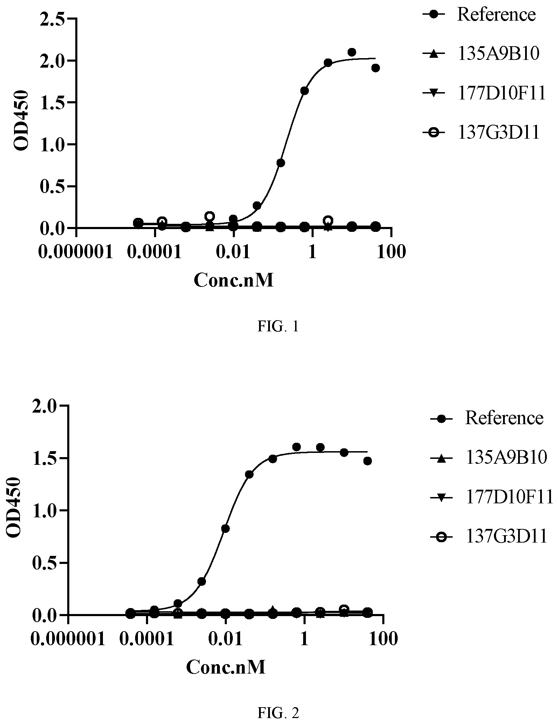

As shown in FIGs. 1 and 2, the anti-human DLL3 murine monoclonal antibodies 177D10F11, 135A9B10, and 137G3D11 exhibited no significant non-specific binding activity to both the human DLL1 protein and the human DLL4 protein.

### 2.5 Cytotoxicity induced by αMFc-CL-DX8951 and anti-human DLL3 murine monoclonal antibodies

αMFc-CL-DX8951 (Moradec, AM-107-DX-50) is an anti-mouse IgG Fc-specific antibody conjugated to DX8951 via a cleavable linker. By recognizing the Fc end of anti-human DLL3 murine antibodies, αMFc-CL-DX8951 induces internalization on DLL3-expressing cells and releases DX8951 intracellularly to kill the cells. Serial dilutions of antibodies and a working solution of the secondary antibody αMFc-CL-DX8951 (4-fold serial dilution, final concentration of 26.4 nM) were prepared with a culture medium. cynoDLL3-HEK293 cells were obtained by digestion with trypsin (Gibco, 25200072), or SHP77 cells were directly pipetted from a culture flask. The cells were centrifuged and adjusted to a cell density of 1E5/mL. 25 µL of each antibody concentration gradient diluent and 50 µL of the cell suspension were added per well to a white clear-bottom 96-well plate. After incubation for 10 min, the secondary antibody working solution was added at 25 µL/well. The 96-well plate was cultured in an incubator at 37 °C with 5% CO₂ for 5-7 days. CTG was added at 50 µL/well, and fluorescence values were read with a microplate reader. The fluorescence values were fitted using four-parameter logistic (4PL) model, and the EC₅₀ value of each antibody was calculated.

As shown in Table 5, the anti-human DLL3 antibody clone 177D10F11 exhibited cell killing activity comparable to or superior to that of the control antibody Rovalpituzumab-mIgG.

**Table 5. EC₅₀ of cytotoxicity induced by αMFc-CL-DX8951 and anti-human DLL3 murine monoclonal antibodies**

| **Sample** | **SHP77** | **HEK293-cynoDLL3** |
|---|---|---|
| | **EC_{50*,*} nM** | **EC_{50,} nM** |
| Rovalpituzumab-mIgG | 0.058 | 0.047 |
| 177D10F11 | 0.042 | 0.031 |
| 135A9B10 | 0.211 | N/T |
| mIgG1 | N/A | N/A |

### 2.6 Detection of internalization function of anti-human DLL3 murine monoclonal antibody

An antibody diluent at 5 µg/mL was prepared with a pre-cooled FACS buffer. hDLL3-CT26 cells were obtained by digestion with trypsin, followed by centrifugation and counting. The cells were taken and transferred into 1.5 mL centrifuge tubes at 1E6/tube, followed by centrifugation. The prepared 5 µg/mL antibody diluent was added, and the plate was incubated at 4 °C in the dark for 1 h. After 3 washes with a pre-cooled FACS buffer, a pre-cooled FACS buffer was added at 1 mL/tube to resuspend the cells. The EP tubes were incubated at 4 °C in the dark. 100 µL of the cell suspension was taken from each EP tube at 0 min, 60 min, 90 min, 105 min, and 115 min, respectively, and added to a 96-well U-bottom plate for incubation at 37 °C. Timing started from the first collection of the cell suspension. After incubation for 120 min, the 96-well plate was taken out and incubated on ice for 5 min. Another 100 µL of the cell suspension was taken from each EP tube and added to the 96-well plate. After 3 washes with a pre-cooled FACS buffer, the AF647 donkey anti-mouse IgG (H+L) secondary antibody (Invitrogen, A31571) was added at 100 µL/well, and the plate was incubated at 4 °C in the dark for 40 min. Fluorescence values were measured with a flow cytometer. The internalization rate was calculated by the formula (G.MFI [0 mins] - G.MFI [time])/G.MFI [0 mins] × 100%.

As shown in Table 6, the internalization capacity of the anti-human DLL3 murine monoclonal antibody 177D10F11 was comparable to that of the positive control antibody Rovalpituzumab-mIgG.

**Table 6. Internalization rates of anti-human DLL3 murine monoclonal antibody in hDLL3-CT26 cells within 5-120 min**

| **Time (mins)** | **5** | **15** | **30** | **60** | **120** |
|---|---|---|---|---|---|
| Rovalpituzumab-mIgG | 10% | 32% | 53% | 68% | 79% |
| 177D10F11 | 13% | 32% | 46% | 71% | 81% |

### 2.7 Assay on affinity of anti-human DLL3 murine monoclonal antibodies

An antibody working solution at 5 µg/mL and gradient diluents of the DLL3-his antigen (initial concentration: 100 nM, 2-fold dilution) were prepared with a buffer (0.05% PBST). Anti-Mouse IgG Fc Capture (AMG) Biosensors (Fortebio, 18-5088) were used to run the binding assay procedure. The parameters were set as: baseline for 1 min, loading for 3 min, baseline 2 for 2 min, association for 3 min, and dissociation for 10 min. Data were analyzed and processed using ForteBio Data Analysis 11.0 software.

As shown in Table 7, the affinity of anti-human DLL3 antibody clones 177D10F11, 137G3D11, and 135A9B10 for the human DLL3 protein was comparable to or superior to that of the control antibody Rovalpituzumab-mIgG.

**Table 7. Affinity of anti-DLL3 murine monoclonal antibodies to human DLL3 protein**

| **Sample** | **KD (M)** |
|---|---|
| Rovalpituzumab-mIgG | 3.00E-10 |
| 177D10F11 | 3.71E-11 |
| 137G3D11 | 2.14E-10 |
| 135A9B10 | 1.40E-10 |

### Example 3: Sequencing of Anti-DLL3 Antibodies and Generation of Chimeric Antibody

### 3.1 Sequencing of anti-DLL3 antibodies and generation of chimeric antibody

Hybridoma clones exhibiting favorable performance in the above activity identification were sequenced, and the sequences are shown in Table 8. The sequenced light chain variable region and heavy chain variable region were respectively constructed onto human constant regions (IgG1/κ, Table 9) for *in vitro* functional identification or *in vivo* pharmacodynamic study. The constructed hIgG1 chimeric antibody was confirmed by sequencing to be consistent with the sequences shown in Table 8 and Table 9.

Nucleic acids encoding the antibodies were transfected into Expi293 cells. Expi293 cells were used for antibody expression, and the antibodies were purified using a Protein A column. The specific procedures were as follows:
Antibody expression in Expi293 cells: Expi293 cells were cultured at 1.5 × 10⁶ cells/mL in a shaker at 37 °C with 8% CO₂ at 120 rpm. The next day, the cell density and viability were measured. The density was 3 × 10⁶ cells/mL, and the viability was higher than 95%. The plasmid comprising the coding nucleic acid was diluted with OPM-293 CD05 Medium (OPM Biosciences, Cat. No.: 81075-001) to a total plasmid concentration of 1 µg/mL. The volume of the culture medium used for plasmid dilution accounted for 1/20 of the transfection volume, and the ratio of light chain to heavy chain was 1:1.5. PEI (1 mg/mL, Polysciences, Cat. No.: 24765-1) was mixed well by inversion and diluted with OPM-293 CD05 Medium. The volume of the culture medium used for PEI dilution accounted for 1/20 of the transfection volume, followed by incubation at room temperature for 5 min. The diluted PEI reagent was added to the diluted plasmid, and the mixture was mixed well, followed by incubation at room temperature for 15 min. The shake flask was cultured in a shaker at 37 °C with 8% CO₂ at 120 rpm for 24 h. 10% OPM-293 ProFeed (OPM Biosciences, Cat. No.: F081918) was added, and the mixture was cultured for another 5-7 days. The supernatant was collected.

Antibody purification by Protein A column: A gasket was placed at the bottom of a gravity column and compressed tightly. Volume of filler suspension = volume of target filler/suspension ratio of filler. The filler was fully vortexed and added to the bottom of the gravity chromatographic column. PBS was added to the gravity chromatographic column until the pH of the outlet was equilibrated to the target pH. A certain volume of the sample was slowly loaded onto the gravity chromatographic column. At least 10 CV of a washing buffer were added, followed by the addition of 5 CV of an elution buffer (10-50 mM NaAc, pH 3.0 to pH 3.5). After incubation for 3-5 min, the eluate was collected. The pH was adjusted to 7.2 with a neutralization buffer (1 M Tris). The protein concentration was determined using Nanodrop. Buffer exchange into PBS was performed using a Millipore ultrafiltration centrifuge tube (50 kDa).

**Table 8. CDR sequences of anti-DLL3 antibodies**

| **Clone No.** | **HCDR1** | **HCDR2** | **HCDR3** | **LCDR1** | **LCDR2** | **LCDR3** |
|---|---|---|---|---|---|---|
| 177D1 0F11 | TYGMS (SEQ ID NO: 1) | WINTYSGVPT YADDFKG (SEQ ID NO: 2) | DPIAMDY (SEQ ID NO: 3) | RASQSIGTDIH (SEQ ID NO: 4) | DASESIS (SEQ ID NO: 5) | QQSNSWPTLT (SEQ ID NO: 6) |
| 135A9 B10 | NYWLH (SEQ ID NO: 15) | MIHPKSDNIK YNEKFKN (SEQ ID NO: 16) | WNLISTVG RYLDV (SEQ ID NO: 17) | KASQNVGTAV G (SEQ ID NO: 18) | STSNRYS (SEQ ID NO: 19) | QQYSSFPLT (SEQ ID NO: 20) |
| 137G3 D11 | DYYMN (SEQ ID NO: 29) | NINPNNGVTT YNQRFKG (SEQ ID NO: 30) | YYYGSSY YVYNSAM DY (SEQ ID NO: 31) | SASSSVSYMH (SEQ ID NO: 32) | DTSKLA S (SEQ ID NO: 33) | HQWSSDPLT (SEQ ID NO: 34) |

The amino acid sequence of the heavy chain variable region (SEQ ID NO: 7) of murine monoclonal antibody 177D10F11 is as follows:

QIQLVQSGPELKKPGETVKISCKASGYTFTTYGMSWVKQAPGKGLKWMGWINTYSGVPTYA
DDFKGRFAFSLETSASTAYLQINNLKNEDTATYFCARDPIAMDYWGQGTSVTVSS

The amino acid sequence of the light chain variable region (SEQ ID NO: 8) of murine monoclonal antibody 177D10F11 is as follows:

DILVTQSPAILSVSPGERVSFSCRASQSIGTDIHWYQQRTNGSPRLLIKDASESISGIPSRFGGSGSGT
DFTLSINSVESEDIADYYCQQSNSWPTLTFGAGTKLELK

The amino acid sequence of the heavy chain variable region (SEQ ID NO: 21) of murine monoclonal antibody 135A9B10 is as follows:

QVQLQQPGTELVKPGASVKLSCKASGYTFTNYWLHWVKQRPGQGLDWIGMIHPKSDNIKY
NEKFKNKATLTVDRSSSTASMQLSSLISEDSAVYYCARWNLISTVGRYLDVWGTGTTVTVSS

The amino acid sequence of the light chain variable region (SEQ ID NO: 22) of murine monoclonal antibody 135A9B10 is as follows:

DIVMTQSQKFMSTTVGDRVSITCKASQNVGTAVGWFQQKPGQSPKLLIYSTSNRYSGVPDRF
TGSGSGTDFTLSINNMQSEDLADYFCQQYSSFPLTFGAGTKLELK

The amino acid sequence of the heavy chain variable region (SEQ ID NO: 35) of murine monoclonal antibody 137G3D11 is as follows:

EVQLQQSGPELVKPGASVKISCKASGFTFTDYYMNWVKQSHGKSLEWIGNINPNNGVTTYN
QRFKGKATLTVDKSSTTACMELRSLTSEDSAVYYCASYYYGSSYYVYNSAMDYWGQGTSVTVSS

The amino acid sequence of the light chain variable region (SEQ ID NO: 36) of murine monoclonal antibody 137G3D11 is as follows:

QIVLTQSPAIMSASPGEKVTMTCSASSSVSYMHWYQQKSGTSPKRWIYDTSKLASGVPARFS
GSGSGTSYSLTLSSMEAEDAATYYCHQWSSDPLTFGAGTKLELK

**Table 9. Constant region sequences of chimeric and humanized antibodies**

| **Sequence name** | **Heavy chain constant region** | **Light chain constant region** |
|---|---|---|
| IgG1/K |  (SEQ ID NO: 45) |  (SEQ ID NO: 46) |

### Example 4: Functional Identification of Chimeric Anti-DLL3 Antibody

### 4.1 Cytotoxicity induced by αHFc-CL-DX8951 and chimeric anti-DLL3 monoclonal antibody

αHFc-CL-DX8951 (Moradec, AH-107-DX-50) is an anti-human IgG Fc-specific antibody conjugated to DX8951 via a cleavable linker. By recognizing the Fc end of a chimeric or humanized anti-DLL3 antibody, αHFc-CL-DX8951 induces internalization on DLL3-expressing cells and releases DX8951 intracellularly to kill the cells. Antibody concentration gradient diluents (initial final concentration: 20 nM, 4-fold serial dilution) and a working solution of the secondary antibody αHFc-CL-DX8951 (final concentration: 26.4 nM, 4-fold serial dilution) were prepared with a culture medium. SHP77 cells were pipetted from a culture flask. The cells were centrifuged and adjusted to a cell density of 1E5/mL. 25 µL of each antibody concentration gradient diluent and 50 µL of the cell suspension were added per well to a white clear-bottom 96-well plate. After incubation for 10 min, the secondary antibody working solution was added at 25 µL/well. The 96-well plate was cultured in a cell incubator at 37 °C with 5% CO₂ for 6 days. CTG was added at 50 µL/well, and fluorescence values were read with a microplate reader. The fluorescence values were fitted using a four-parameter logistic (4PL) model, and the EC₅₀ value of each antibody was calculated.

As shown in Table 10, the cell killing activity of the chimeric antibody ch177D10F11 was comparable to that of the positive control antibody Rovalpituzumab.

**Table 10. EC₅₀ of cytotoxicity induced by αHFc-CL-DX8951 and chimeric anti-DLL3 monoclonal antibody**

| **Sample** | **EC₅₀ nM** |
|---|---|
| Rovalpituzumab | 0.069 |
| ch177D10F11 | 0.100 |
| hIgG1 | N/A |

### 4.2 Humanization of anti-DLL3 antibodies and functional identification of humanized antibodies

CDRs were defined according to the Kabat numbering. Human germline genes with the highest homology to the murine sequences were selected as acceptor frameworks, and the CDRs of the murine sequences were grafted into the human frameworks. Back mutations were performed according to the importance of amino acids; specifically, certain key amino acids in the framework regions after grafting were back-mutated to the corresponding murine amino acids, and several variants were designed for each of the heavy chain and light chain.

### 4.2.1 Humanization of anti-DLL3 antibody 177D10F11

**Table 11. Designed humanized sequences of anti-DLL3 antibody 177D10F11**

| **Heavy/light chain variable region sequence No.** | **Sequence** |
|---|---|
| mAb-013-177D10F11-hVH1 | QVQLVQSGSELKKPGASVKVSCKASGYTFTTYGMSWVRQAPGQGLEWM GWINTYSGVPTYADDFKGRFVFSLDTSVSTAYLQISSLKAEDTAVYYCARD PIAMDYWGQGTLVTVSS (SEQ ID NO: 9) |
| mAb-013-177D10F11-hVH2 | QIQLVQSGSELKKPGASVKVSCKASGYTFTTYGMSWVRQAPGQGLEWM GWINTYSGVPTYADDFKGRFVFSLDTSVSTAYLQISSLKAEDTAVYYCARD PIAMDYWGQGTLVTVSS (SEQ ID NO: 10) |
| mAb-013-177D10F11-hVH3 | QVQLVQSGSELKKPGASVKVSCKASGYTFTTYGMSWVRQAPGQGLEWM GWINTYSGVPTYADDFKGRFVFSLDTSVSTAYLQISSLKAEDTAVYFCARD PIAMDYWGQGTLVTVSS (SEQ ID NO: 11) |
| mAb-013-177D10F11-hVL1 | EIVLTQSPATLSLSPGERATLSCRASQSIGTDIHWYQQKPGQAPRLLIYDAS ESISGIPARFSGSGSGTDFTLTISSLEPEDFAVYYCQQSNSWPTLTFGQGTKL ELK (SEQ ID NO: 12) |
| mAb-013-177D10F11-hVL2 | EIVVTQSPATLSLSPGERATLSCRASQSIGTDIHWYQQKPGQAPRLLIYDAS ESISGIPARFSGSGSGTDFTLTISSLEPEDFAVYYCQQSNSWPTLTFGQGTKL ELK (SEQ ID NO: 13) |
| mAb-013-177D10F11-hVL3 | EIVLTQSPATLSLSPGERATLSCRASQSIGTDIHWYQQKPGQAPRLLIKDAS ESISGIPARFSGSGSGTDFTLTISSLEPEDFAVYYCQQSNSWPTLTFGQGTKL ELK (SEQ ID NO: 14) |

### 4.2.2 Humanization of anti-DLL3 antibody 135A9B10

**Table 12. Designed humanized heavy and light chain sequences of anti-DLL3 antibody 135A9B10**

| **Heavy/light chain variable region sequence No.** | **Sequence** |
|---|---|
| mAb-013-135A9B10-hVH0 | QVQLVQSGAEVKKPGASVKVSCKASGYTFTNYWLHWVRQAPGQGLE WMGMIHPKSDNIKYNEKFKNRVTMTRDTSTSTVYMELSSLRSEDTAVY YCARWNLISTVGRYLDVWGQGTTVTVSS (SEQ ID NO: 23) |
| mAb-013-135A9B10-hVH1 | QVQLVQSGAEVKKPGASVKVSCKASGYTFTNYWLHWVRQAPGQGLE WMGMIHPKSDNIKYNEKFKNRVTMTVDRSTSTVYMELSSLRSEDTAVY YCARWNLISTVGRYLDVWGQGTTVTVSS (SEQ ID NO: 24) |
| mAb-013-135A9B10-hVH2 | QVQLVQSGAEVKKPGASVKVSCKASGYTFTNYWLHWVRQAPGQGLE WMGMIHPKSDNIKYNEKFKNRATLTVDRSTSTVYMELSSLRSEDTAVYY CARWNLISTVGRYLDVWGQGTTVTVSS (SEQ ID NO: 25) |
| mAb-013-135A9B10-hVH3 | QVQLVQSGAEVKKPGASVKVSCKASGYTFTNYWLHWVRQAPGQGLE WIGMIHPKSDNIKYNEKFKNKATLTVDRSTSTAYMELSSLRSEDTAVYYC ARWNLISTVGRYLDVWGQGTTVTVSS (SEQ ID NO: 26) |
| mAb-013-135A9B10-hVL0 | DIQMTQSPSSLSASVGDRVTITCKASQNVGTAVGWYQQKPGKAPKLLIY STSNRYSGVPSRFSGSGSGTDFTLTISSLQPEDFATYYCQQYSSFPLTFGQG TKLELK (SEQ ID NO: 27) |
| mAb-013-135A9B10-hVL1 | DIQMTQSPSSLSASVGDRVTITCKASQNVGTAVGWYQQKPGKAPKLLIY STSNRYSGVPSRFSGSGSGTDFTLTISSLQPEDFATYFCQQYSSFPLTFGQG TKLELK (SEQ ID NO: 28) |

### 4.2.3 Humanization of anti-DLL3 antibody 137G3D11

**Table 13. Designed humanized sequences of anti-DLL3 antibody 137G3D11**

| **Heavy/light chain variable region sequence No.** | **Sequence** |
|---|---|
| mAb-013-137G3D11-hVH0 | QVQLVQSGAEVKKPGASVKVSCKASGYTFTDYYMNWVRQAPGQGLEWM GNINPNNGVTTYNQRFKGRVTMTRDTSTSTVYMELSSLRSEDTAVYYCARY YYGSSYYVYNSAMDYWGQGTTVTVSS (SEQ ID NO: 37) |
| mAb-013-137G3D11-hVH1 | QVQLVQSGAEVKKPGASVKVSCKASGYTFTDYYMNWVRQAPGQGLEWM GNINPNNGVTTYNQRFKGRVTMTVDKSTSTVYMELSSLRSEDTAVYYCAS YYYGSSYYVYNSAMDYWGQGTTVTVSS (SEQ ID NO: 38) |
| mAb-013-137G3D11-hVH2 | QVQLVQSGAEVKKPGASVKVSCKASGFTFTDYYMNWVRQAPGQGLEWIG NINPNNGVTTYNQRFKGRATLTVDKSTSTVYMELSSLRSEDTAVYYCASYY YGSSYYVYNSAMDYWGQGTTVTVSS (SEQ ID NO: 39) |
| mAb-013-137G3D11-hVH3 | QVQLVQSGAEVKKPGASVKVSCKASGFTFTDYYMNWVRQAPGQGLEWM GNINPNNGVTTYNQRFKGRVTMTVDKSTTTAYMELSSLRSEDTAVYYCASY YYGSSYYVYNSAMDYWGQGTTVTVSS (SEQ ID NO: 40) |
| mAb-013-137G3D11-hVH4 | QVQLVQSGAEVKKPGASVKVSCKASGFTFTDYYMNWVRQAPGQGLEWIG NINPNNGVTTYNQRFKGRATLTVDKSTTTAYMELSSLRSEDTAVYYCASYY YGSSYYVYNSAMDYWGQGTTVTVSS (SEQ ID NO: 41) |
| mAb-013-137G3D11-hVL0 | EIVLTQSPATLSLSPGERATLSCSASSSVSYMHWYQQKPGQAPRLLIYDTSKL ASGIPARFSGSGSGTDFTLTISSLEPEDFAVYYCHQWSSDPLTFGQGTKLELK (SEQ ID NO: 42) |
| mAb-013-137G3D11-hVL1 | EIVLTQSPATLSLSPGERATLSCSASSSVSYMHWYQQKPGQAPRRWIYDTSK LASGIPARFSGSGSGTDFTLTISSLEPEDFAVYYCHQWSSDPLTFGQGTKLEL K (SEQ ID NO: 43) |
| mAb-013-137G3D11-hVL2 | EIVLTQSPATLSLSPGERATLSCSASSSVSYMHWYQQKPGQAPRRWIYDTSK LASGVPARFSGSGSGTDYTLTISSLEPEDFAVYYCHQWSSDPLTFGQGTKLE LK (SEQ ID NO: 44) |

**Table 14. Corresponding sequence numbers of humanized antibodies of anti-DLL3 antibodies**

| Name of humanized antibodies | Heavy chain variable region sequence No. | Light chain variable region sequence No. |
|---|---|---|
| hz177D10F11-H1L1 | mAb-013-177D10F11-hVH1 (SEQ ID NO: 9) | mAb-013-177D10F11-hVL1 (SEQ ID NO: 12) |
| hz177D10F11-H1L2 | mAb-013-177D10F11-hVH1 (SEQ ID NO: 9) | mAb-013-177D10F11-hVL2 (SEQ ID NO: 13) |
| hz177D10F11-H1L3 | mAb-013-177D10F11-hVH1 (SEQ ID NO: 9) | mAb-013-177D10F11-hVL3 (SEQ ID NO: 14) |
| hz177D10F11-H2L1 | mAb-013-177D10F11-hVH2 (SEQ ID NO: 10) | mAb-013-177D10F11-hVL1 (SEQ ID NO: 12) |
| hz177D10F11-H2L2 | mAb-013-177D10F11-hVH2 (SEQ ID NO: 10) | mAb-013-177D10F11-hVL2 (SEQ ID NO: 13) |
| hz177D10F11-H2L3 | mAb-013-177D10F11-hVH2 (SEQ ID NO: 10) | mAb-013-177D10F11-hVL3 (SEQ ID NO: 14) |
| hz177D10F11-H3L1 | mAb-013-177D10F11-hVH3 (SEQ ID NO: 11) | mAb-013-177D10F11-hVL1 (SEQ ID NO: 12) |
| hz177D10F11-H3L2 | mAb-013-177D10F11-hVH3 (SEQ ID NO: 11) | mAb-013-177D10F11-hVL2 (SEQ ID NO: 13) |
| hz177D10F11-H3L3 | mAb-013-177D10F11-hVH3 (SEQ ID NO: 11) | mAb-013-177D10F11-hVL3 (SEQ ID NO: 14) |
| hz135A9B10-H0L0 | mAb-013-135A9B10-hVH0 (SEQ ID NO: 23) | mAb-013-135A9B10-hVL0 (SEQ ID NO: 27) |
| hz135A9B10-H0L1 | mAb-013-135A9B10-hVH0 (SEQ ID NO: 23) | mAb-013-135A9B10-hVL1 (SEQ ID NO: 28) |
| hz135A9B10-H1L0 | mAb-013-135A9B10-hVH1 (SEQ ID NO: 24) | mAb-013-135A9B10-hVL0 (SEQ ID NO: 27) |
| hz135A9B10-H1L1 | mAb-013-135A9B10-hVH1 (SEQ ID NO: 24) | mAb-013-135A9B10-hVL1 (SEQ ID NO: 28) |
| hz135A9B10-H2L0 | mAb-013-135A9B10-hVH2 (SEQ ID NO: 25) | mAb-013-135A9B10-hVL0 (SEQ ID NO: 27) |
| hz135A9B10-H2L1 | mAb-013-135A9B10-hVH2 (SEQ ID NO: 25) | mAb-013-135A9B10-hVL1 (SEQ ID NO: 28) |
| hz135A9B10-H3L0 | mAb-013-135A9B10-hVH3 (SEQ ID NO: 26) | mAb-013-135A9B10-hVL0 (SEQ ID NO: 27) |
| hz135A9B10-H3L1 | mAb-013-135A9B10-hVH3 (SEQ ID NO: 26) | mAb-013-135A9B10-hVL1 (SEQ ID NO: 28) |
| hz137G3D11-H0L0 | mAb-013-137G3D11-hVH0 (SEQ ID NO :37) | mAb-013-137G3D11-hVL0 (SEQ ID NO: 42) |
| hz137G3D11-H0L1 | mAb-013-137G3D11-hVH0 (SEQ ID NO: 37) | mAb-013-137G3D11-hVL1 (SEQ ID NO: 43) |
| hz137G3D11-H0L2 | mAb-013-137G3D11-hVH0 (SEQ ID NO :37) | mAb-013-137G3D11-hVL2 (SEQ ID NO: 44) |
| hz137G3D11-H1L0 | mAb-013-137G3D11-hVH1 (SEQ ID NO: 38) | mAb-013-137G3D11-hVL0 (SEQ ID NO: 42) |
| hz137G3D11-H1L1 | mAb-013-137G3D11-hVH1 (SEQ ID NO: 38) | mAb-013-137G3D11-hVL1 (SEQ ID NO: 43) |
| hz137G3D11-H1L2 | mAb-013-137G3D11-hVH1 (SEQ ID NO: 38) | mAb-013-137G3D11-hVL2 (SEQ ID NO: 44) |
| hz137G3D11-H2L0 | mAb-013-137G3D11-hVH2 (SEQ ID NO: 39) | mAb-013-137G3D11-hVL0 (SEQ ID NO: 42) |
| hz137G3D11-H2L1 | mAb-013-137G3D11-hVH2 (SEQ ID NO: 39) | mAb-013-137G3D11-hVL1(SEQ ID NO: 43) |
| hz137G3D11-H2L2 | mAb-013-137G3D11-hVH2 (SEQ ID NO: 39) | mAb-013-137G3D11-hVL2 (SEQ ID NO: 44) |
| hz137G3D11-H3L0 | mAb-013-137G3D11-hVH3 (SEQ ID NO: 40) | mAb-013-137G3D11-hVL0 (SEQ ID NO: 42) |
| hz137G3D11-H3L1 | mAb-013-137G3D11-hVH3 (SEQ ID NO: 40) | mAb-013-137G3D11-hVL1 (SEQ ID NO: 43) |
| hz137G3D11-H3L2 | mAb-013-137G3D11-hVH3 (SEQ ID NO: 40) | mAb-013-137G3D11-hVL2 (SEQ ID NO: 44) |
| hz137G3D11-H4L0 | mAb-013-137G3D11-hVH4 (SEQ ID NO: 41) | mAb-013-137G3D11-hVL0 (SEQ ID NO: 42) |
| hz137G3D11-H4L1 | mAb-013-137G3D11-hVH4 (SEQ ID NO: 41) | mAb-013-137G3D11-hVL1 (SEQ ID NO: 43) |
| hz137G3D11-H4L2 | mAb-013-137G3D11-hVH4 (SEQ ID NO: 41) | mAb-013-137G3D11-hVL2 (SEQ ID NO: 44) |

### 4.3 Expression and purification of humanized anti-DLL3 antibodies

The light chain and heavy chain variable regions of humanized antibodies were respectively constructed onto human constant regions (IgG1/κ, Table 9) for gene synthesis. The consistency with the designed sequences was confirmed by sequencing. Expression was performed in Expi293 cells using the method described in Section 3.1, and purification was performed using a Protein A column.

### Example 5: Binding Activity of Humanized Anti-DLL3 Antibodies

### 5.1 Assay on binding activity of humanized anti-DLL3 antibodies to human DLL3

Flow cytometry was used to detect the binding activity of humanized anti-human DLL3 monoclonal antibodies for human and cynomolgus monkey DLL3 on the surface of SHP77 cells (ATCC, CRL-2195) and HEK293 cells overexpressing cynomolgus monkey DLL3 (Kyinno Biotechnology, KC-0294). HEK293 cells overexpressing cynomolgus monkey DLL3 were obtained by digestion with trypsin, and SHP77 cells were taken from a culture flask and pipetted into single cells. After counting and cell concentration adjustment, the cells were added to a 96-well U-bottom plate. The plate was centrifuged at 300 g for 5 min, and the supernatant was discarded. 100 µL of serially diluted antibody prepared with an FACS buffer was added, and the mixture was mixed well, followed by incubation at 4 °C for 60 min. After 3 washes with an FACS buffer, AF647-anti-hIgG (H+L) (1:1000, Invitrogen, A21445) was added at 100 µL/well, followed by incubation at 4 °C for 1 h. After the 3 washes with an FACS buffer, an FACS buffer was added at 100 µL/well to resuspend the cells. Fluorescence signals were detected by a flow cytometer (BECKMAN COULTER cytoFLEX). The binding activity was fitted using four-parameter logistic (4PL) model to calculate the EC₅₀ value of each antibody.

As shown in Table 15, all tested humanized anti-DLL3 antibodies exhibited good binding activity to human and cynomolgus monkey DLL3 proteins on the cell surface.

**Table 15. Binding activity of humanized anti-DLL3 monoclonal antibodies to human DLL3 protein and human DLL3 protein on surface of SHP77 cells**

| **Sample** | **SHP77** | **HEK293-cynoDLL3** |
|---|---|---|
| | EC_{50,} nM | EC_{50***,***} nM |
| Rovalpituzumab | 0.026 | 0.047 |
| hz177D10F11-H1L1 | 0.043 | 0.043 |
| hz177D10F11-H1L2 | 0.052 | 0.052 |
| hz177D10F11-H1L3 | 0.047 | 0.047 |
| hz177D10F11-H2L1 | 0.031 | 0.031 |
| hz177D10F11-H2L2 | 0.024 | 0.024 |
| hz177D10F11-H2L3 | 0.054 | 0.054 |
| hz177D10F11-H3L1 | 0.039 | 0.039 |
| hz177D10F11-H3L2 | 0.043 | 0.043 |
| hz177D10F11-H3L3 | 0.048 | 0.048 |
| IgG1 | N/A | N/A |

### 5.2 Assay for affinity of humanized anti-DLL3 antibodies

An antibody working solution at 5 µg/mL and serial dilutions of the DLL3-his antigen (initial concentration: 100 nM, 2-fold serial dilution) were prepared with a buffer (0.05% TPBS). Anti-Human IgG Fc Capture (AHG) Biosensors (Fortebio, 18-5068) were used to run the binding kinetic assay. The parameters were set as: baseline for 1 min, loading for 3 min, baseline2 for 2 min, association for 3 min, and dissociation for 10 min. Data were analyzed and processed using ForteBio Data Analysis 11.0 software.

As shown in Table 16, all tested antibodies possessed good affinity to the human DLL3 protein.

**Table 16. Affinity of humanized anti-DLL3 antibodies to human DLL3 protein**

| **Loading Sample ID** | **KD (M)** | **kₒₙ (1/Ms)** | **k_{dis} (1/s)** | **Full R^2** |
|---|---|---|---|---|
| Rovalpituzumab | 1.37E-10 | 5.12E+05 | 7.05E-05 | 0.990 |
| hz177D10F11-H1-L1 | 1.29E-10 | 3.64E+05 | 4.70E-05 | 0.992 |
| hz177D10F11-H2-L1 | 1.40E-10 | 3.72E+05 | 5.20E-05 | 0.992 |
| hz177D10F11-H2-L2 | 1.06E-10 | 3.90E+05 | 4.13E-05 | 0.991 |
| hz135A9B10-H0-L1 | 4.35E-11 | 2.50E+05 | 1.09E-05 | 0.999 |
| hz137G3D11-H3-L2 | 2.37E-11 | 3.93E+05 | 9.31E-06 | 0.996 |
| hz137G3D11-H4-L1 | 4.42E-11 | 3.75E+05 | 1.66E-05 | 0.996 |
| hz137G3D11-H3-L1 | 2.75E-11 | 3.56E+05 | 9.78E-06 | 0.996 |

### Example 6: Conjugation and In Vitro Killing Activity Assay of Humanized Anti-DLL3 Antibody-ADCs

### 6.1 Conjugation of humanized anti-DLL3 antibody-MMAE

A certain amount of the antibody and a PBS buffer were added to achieve an antibody concentration of about 1 mg/mL in the reaction mixture. A certain amount of an EDTA solution was added to achieve an EDTA concentration of about 2 mM in the mixture. A 1 M Tris solution was added to adjust the pH of the reaction system to 6.6-7.5. TCEP was added at 2-6 equivalents for reduction. The mixture was incubated with gentle mixing at 37 °C for 1-3 h. Subsequently, the temperature of the mixer was adjusted to about 4 °C to cool the reaction liquid. Vc-MMAE (MCE, HY-15575) dissolved in DMSO was added at 5-10 equivalents, and the reaction was carried out at 4 °C for 1-2 h. Finally, the reaction liquid was added to a 30 K ultrafiltration tube. Buffer exchange was performed 6-8 times with a 10 mM histidine-hydrochloric acid solution (pH 6.0). The mixture was concentrated to an appropriate volume, loaded onto an equilibrated desalting column, and centrifuged to obtain a purified ADC product. The DAR values were measured, and the measurement result for the DAR value of the ADC sample is shown in Table 17. The structure of the anti-human DLL3 antibody-MMAE conjugate is shown below.

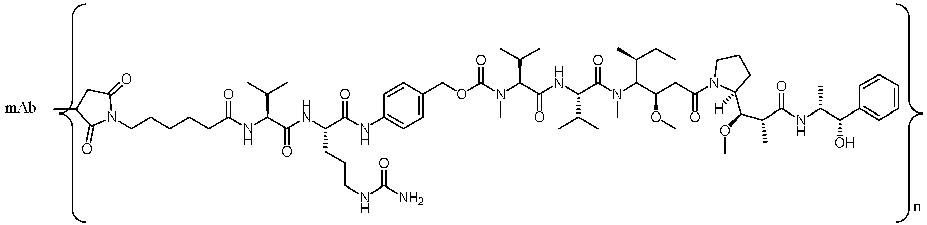

### Structure of humanized anti-DLL3 antibody-MMAE conjugate

### 6.2 Conjugation of humanized anti-DLL3 antibody-Dxd

The antibody was added to a PBS buffer to achieve an antibody reaction concentration of 1.0 mg/mL. 500 mM EDTA (SCRC, 10009617) was added to achieve a final concentration of 2 mM. A TCEP solution (Thermo, 20490) was added to the reaction liquid, and the mixture was mixed well (molar ratio of TCEP:mAb = 6:1) and reacted at 37 °C and 600 rpm for 2 h. After the reaction was completed, the reaction liquid was cooled to 4 °C, and a Deruxtecan solution (MCE, HY-1363E) dissolved in DMSO was added to the reaction liquid. The mixture was mixed well (molar ratio of drug:mAb = 15:1) and reacted at 4 °C and 600 rpm for 1 h. After the reaction was completed, the reaction liquid was added to a 30 K ultrafiltration tube. Buffer exchange was performed 6-8 DV with a 10 mM histidine-hydrochloric acid solution (pH 6.0). The mixture was concentrated to an appropriate volume, loaded onto an equilibrated desalting column, and centrifuged to obtain a purified ADC product. The final concentration was determined by Nanodrop, the purity was detected by SEC-HPLC, and the DAR value was measured by RP-HPLC/HIC-HPLC. The measurement results for the DAR values of the ADC sample are shown in Table 17. The structure of the anti-human DLL3 antibody-Dxd conjugate is shown below.

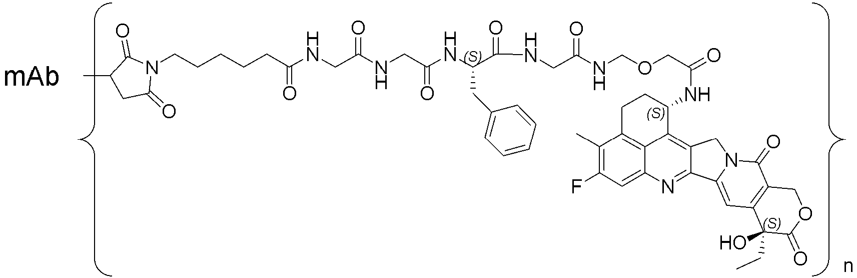

### Structure of anti-human DLL3 antibody-Dxd conjugate

**Table 17. Characterization results of conjugates with anti-human DLL3 antibody to Dxd or MMAE**

| **Sample name** | **DAR value** |
|---|---|
| Rovalpituzumab-Dxd | 5.7 |
| hz177D10F11-H1L1-Dxd | 5.9 |
| hIgG1-Dxd | 6.0 |
| Rovalpituzumab-MMAE | 3.6 |
| hz177D10F11-H1L1-MMAE | 3.4 |
| hIgG1-MMAE | 3.6 |
| Rovalpituzumab-Dxd | 7.6 |
| hz177D10F11-H1L1-Dxd | 7.5 |

### 6.3 Killing activity of anti-DLL3 antibody-ADCs against HEK293-cynoDLL3 cells and NCI-H524 cells

The cell concentration was adjusted, and the cells were seeded into a white opaque plate at 50 µL/well. The plate was cultured overnight in an incubator at 37 °C with 5% CO₂. The anti-DLL3 antibody-ADC, the positive control antibody-ADC, and Isotype hIgG1-ADC serially diluted with a culture medium were added at 50 µL/well, and the plate was cultured in an incubator at 37 °C with 5% CO₂ for 4-6 days. A luciferase substrate solution was added. After mixing, the mixture was reacted at room temperature for 5 min in the dark, and relative light unit (RLU) values were read on the cell culture plate with a microplate reader. Experimental data were analyzed using Graphpad Prism 8.0 software. The logarithm of antibody concentration was set as the x-axis, and the corresponding RLU value was set as the y-axis. A four-parameter logistic (4PL) regression model was selected to fit the dose-response curve of the antibody, and the EC₅₀ values were calculated.

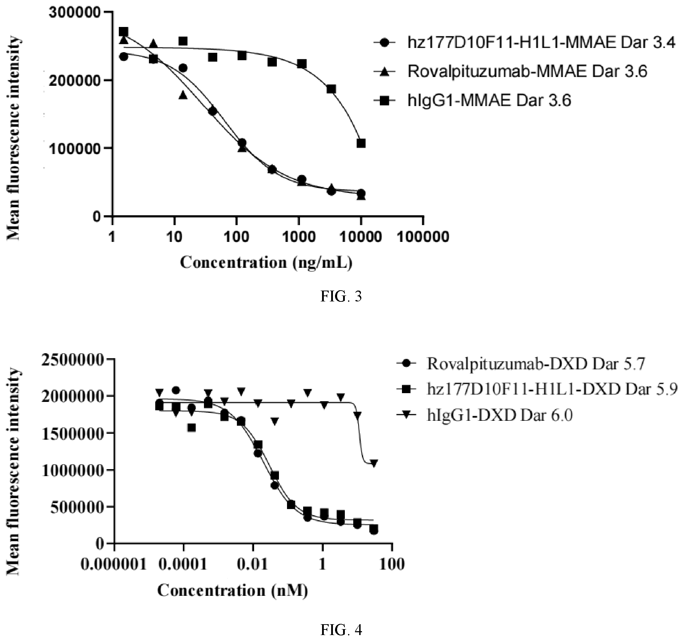

The experimental results shown in FIG. 3, FIG. 4, and Table 18 indicated that compared with Isotype hIgG1-ADC, hz177D10F11-H1L1-ADC exhibited superior cell killing activity, and the killing activity of hz177D10F11-H1L1-ADC was comparable to that of the positive control Rovalpituzumab-ADC.

**Table 18. Killing activity of humanized anti-human DLL3 antibody-ADCs against HEK293-cynoDLL3 and NCI-H524 cells**

| **Sample** | **Cell line** | **IC50** |
|---|---|---|
| Rovalpituzumab- Dxd Dar 5.7 | NCI-H524 | 0.020 nM |
| hz177D10F11-H1L1- Dxd Dar 5.9 | NCI-H524 | 0.029 nM |
| hIgG1-Dxd Dar 6.0 | NCI-H524 | N/A |
| Rovalpituzumab- MMAE Dar 3.6 | HEK293-cynoDLL3 | 28.4 ng/mL |
| hz177D10F11-H1L1- MMAE Dar 3.4 | HEK293-cynoDLL3 | 64.2 ng/mL |
| hIgG1- MMAE Dar 3.6 | HEK293-cynoDLL3 | N/A |

### Example 7: Assay on In Vivo Killing Ability of Anti-Human DLL3 Antibody-ADC

This example aims to investigate the tumor growth inhibition of the anti-human DLL3 antibody-ADC in a mouse NCI-H524 model.

### 7.1 Preparation of conjugate of anti-human DLL3 antibody to Dxd

The procedures are described in Section 6.2 "Conjugation of humanized anti-human DLL3 antibody-Dxd".

### 7.2 Inhibition of NCI-H524 (human non-small cell lung cancer) cell growth by anti-human DLL3 antibody-ADC in immunodeficient mouse (Balb/c Nude) model

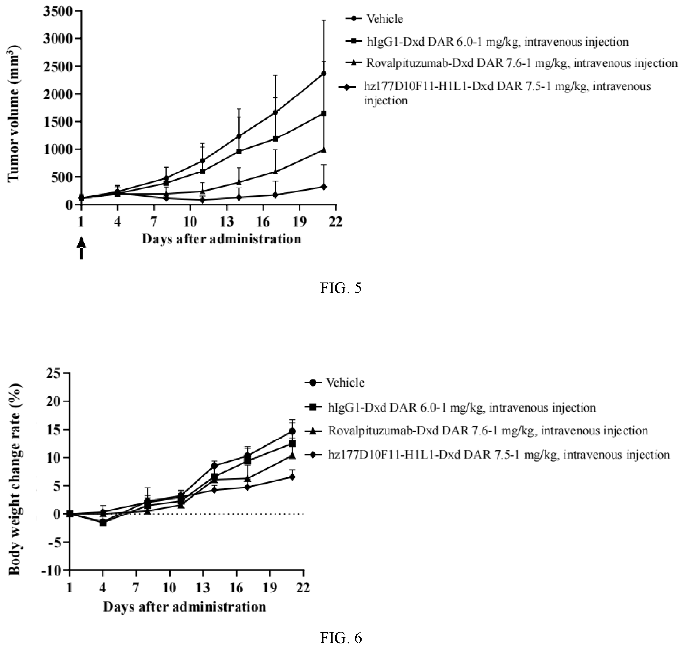

An NCI-H524 human non-small cell lung cancer mouse model was established in immunodeficient mice (Balb/c Nude, BALB/cNj-Foxn1nu/Gpt, Jiangsu GemPharmatech Co., Ltd., Strain Code: D000521), and the anti-tumor efficacy of different molecules of the anti-human DLL3 antibody-ADC was determined in this mouse tumor model. NCI-H524 cells were diluted with a serum-free culture medium (RPMI 1640), and a corresponding volume of Matrigel (Cat. No.: 354234, BD) was added and mixed well with the cell solution (RPMI 1640 culture medium:Matrigel = 7:3). Then, the cells were inoculated subcutaneously into the right flank of Balb/c Nude mice at 5 × 10⁶ cells/mouse. When the mean tumor volume reached about 150 mm³, the mice were grouped, with 5 mice in each group. Vehicle control (histidine-hydrochloric acid buffer), hIgG1-Dxd (DAR 6.0, 1 mg/kg), Rovalpituzumab-Dxd (DAR 7.6, 1 mg/kg), and hz177D10F11-H1L1-Dxd (DAR 7.5, 1 mg/kg) were respectively administered via tail vein injection (i.v.) at an administration volume of 10 mL/kg. After administration, the mice were fed normally, and the survival status of the mice was observed. The body weight and tumor volume of the mice were recorded every Tuesday and Friday for 3 weeks. FIG. 5 shows the curve for the growth inhibition of NCI-H524 tumor cells by the anti-human DLL3 antibody-ADC, and FIG. 6 shows the curve for the rate of change in the body weight of mice in the NCI-H524 tumor model inhibited by the anti-human DLL3 antibody-ADC. As shown in FIGs. 5 and 6, starting from Day 8 after the administration, the tumor volume in the vehicle control group was 474.9±100.3 mm³, while the tumor volumes in the Rovalpituzumab-Dxd (DAR 7.6, 1 mg/kg) group and the hz177D10F11-H1L1-Dxd (DAR 7.5, 1 mg/kg) group were 196.1±60.9 mm³ and 115.5±26.6 mm³, respectively, with TGI_{TV} (tumor volume inhibition rate) values being 58.7% and 75.7%, respectively. Subsequently, on Days 11, 14, 17, and 21, the TGI_{TV} values for the Rovalpituzumab-Dxd (DAR 7.6, 1 mg/kg) group were 69.8%, 67.7%, 64.4%, and 58.2%, respectively. The TGI_{TV} values for the hz177D10F11-H1L1-Dxd (DAR 7.5, 1 mg/kg) group on the same days were 89.4%, 89.4%, 89.4%, and 86.4%, respectively. During the experimental observation, tumor-bearing mice exhibited good tolerance to the test samples, with no body weight loss. Under single administration at 1 mg/kg, hz177D10F11-H1L1-Dxd (DAR 7.6) exhibited a significant effect of inhibiting tumor growth.

Apparently, the above examples are merely illustrative for clarity and are not intended to limit the embodiments. Other changes or variations in different forms can be made by those of ordinary skill in the art on the basis of the above description. It is neither necessary nor possible to provide an exhaustive list of all embodiments herein. Obvious changes or variations derived therefrom still fall within the protection scope of the present disclosure.

### Sequence information

### CDR, heavy chain variable region, and light chain variable region sequences:

| **No.** | **Name** | **Sequence** |
|---|---|---|
| SEQ ID NO: 1 | 177D10F11 HCDR1 | TYGMS |
| SEQ ID NO: 2 | 177D10F11 HCDR2 | WINTYSGVPTYADDFKG |
| SEQ ID NO: 3 | 177D10F11 HCDR3 | DPIAMDY |
| SEQ ID NO: 4 | 177D10F11 LCDR1 | RASQSIGTDIH |
| SEQ ID NO: 5 | 177D10F11 LCDR2 | DASESIS |
| SEQ ID NO: 6 | 177D10F11 LCDR3 | QQSNSWPTLT |
| SEQ ID NO: 7 | 177D10F11 VH (murine) | QIQLVQSGPELKKPGETVKISCKASGYTFTTYGMSWVK QAPGKGLKWMGWINTYSGVPTYADDFKGRFAFSLETS ASTAYLQINNLKNEDTATYFCARDPIAMDYWGQGTSVT VSS |
| SEQ ID NO: 8 | 177D10F11 VL (murine) | DILVTQSPAILSVSPGERVSFSCRASQSIGTDIHWYQQRT NGSPRLLIKDASESISGIPSRFGGSGSGTDFTLSINSVESE DIADYYCQQSNSWPTLTFGAGTKLELK |
| SEQ ID NO: 9 | mAb-013-177D10F11-hVH1 (humanized) | QVQLVQSGSELKKPGASVKVSCKASGYTFTTYGMSWV RQAPGQGLEWMGWINTYSGVPTYADDFKGRFVFSLDT SVSTAYLQISSLKAEDTAVYYCARDPIAMDYWGQGTLV TVSS |
| SEQ ID NO: 10 | mAb-013-177D10F11-hVH2 (humanized) | QIQLVQSGSELKKPGASVKVSCKASGYTFTTYGMSWV RQAPGQGLEWMGWINTYSGVPTYADDFKGRFVFSLDT SVSTAYLQISSLKAEDTAVYYCARDPIAMDYWGQGTLV TVSS |
| SEQ ID NO: 11 | mAb-013-177D10F11-hVH3 (humanized) | QVQLVQSGSELKKPGASVKVSCKASGYTFTTYGMSWV RQAPGQGLEWMGWINTYSGVPTYADDFKGRFVFSLDT SVSTAYLQISSLKAEDTAVYFCARDPIAMDYWGQGTLV TVSS |
| SEQ ID NO: 12 | mAb-013-177D10F11-hVL1 (humanized) | EIVLTQSPATLSLSPGERATLSCRASQSIGTDIHWYQQKP GQAPRLLIYDASESISGIPARFSGSGSGTDFTLTISSLEPED FAVYYCQQSNSWPTLTFGQGTKLELK |
| SEQ ID NO: 13 | mAb-013-177D10F11-hVL2 (humanized) | EIVVTQSPATLSLSPGERATLSCRASQSIGTDIHWYQQKP GQAPRLLIYDASESISGIPARFSGSGSGTDFTLTISSLEPED |
| | | FAVYYCQQSNSWPTLTFGQGTKLELK |
| SEQ ID NO: 14 | mAb-013-177D10F11-hVL3 (humanized) | EIVLTQSPATLSLSPGERATLSCRASQSIGTDIHWYQQKP GQAPRLLIKDASESISGIPARFSGSGSGTDFTLTISSLEPED FAVYYCQQSNSWPTLTFGQGTKLELK |
| SEQ ID NO: 15 | 135A9B10 HCDR1 | NYWLH |
| SEQ ID NO: 16 | 135A9B10 HCDR2 | MIHPKSDNIKYNEKFKN |
| SEQ ID NO: 17 | 135A9B10 HCDR3 | WNLISTVGRYLDV |
| SEQ ID NO: 18 | 135A9B10 LCDR1 | KASQNVGTAVG |
| SEQ ID NO: 19 | 135A9B10 LCDR2 | STSNRYS |
| SEQ ID NO: 20 | 135A9B10 LCDR3 | QQYSSFPLT |
| SEQ ID NO: 21 | 135A9B10 VH (murine) | QVQLQQPGTELVKPGASVKLSCKASGYTFTNYWLHWV KQRPGQGLDWIGMIHPKSDNIKYNEKFKNKATLTVDRS SSTASMQLSSLISEDSAVYYCARWNLISTVGRYLDVWG TGTTVTVSS |
| SEQ ID NO: 22 | 135A9B10 VL (murine) | DIVMTQSQKFMSTTVGDRVSITCKASQNVGTAVGWFQ QKPGQSPKLLIYSTSNRYSGVPDRFTGSGSGTDFTLSINN MQSEDLADYFCQQYSSFPLTFGAGTKLELK |
| SEQ ID NO: 23 | mAb-013-135A9B10-hVH0 (humanized) | QVQLVQSGAEVKKPGASVKVSCKASGYTFTNYWLHW VRQAPGQGLEWMGMIHPKSDNIKYNEKFKNRVTMTRD TSTSTVYMELSSLRSEDTAVYYCARWNLISTVGRYLDV WGQGTTVTVSS |
| SEQ ID NO: 24 | mAb-013-135A9B10-hVH1 (humanized) | QVQLVQSGAEVKKPGASVKVSCKASGYTFTNYWLHW VRQAPGQGLEWMGMIHPKSDNIKYNEKFKNRVTMTV DRSTSTVYMELSSLRSEDTAVYYCARWNLISTVGRYLD VWGQGTTVTVSS |
| SEQ ID NO: 25 | mAb-013-135A9B10-hVH2 (humanized) | QVQLVQSGAEVKKPGASVKVSCKASGYTFTNYWLHW VRQAPGQGLEWMGMIHPKSDNIKYNEKFKNRATLTVD RSTSTVYMELSSLRSEDTAVYYCARWNLISTVGRYLDV WGQGTTVTVSS |
| SEQ ID NO: 26 | mAb-013-135A9B10-hVH3 (humanized) | QVQLVQSGAEVKKPGASVKVSCKASGYTFTNYWLHW VRQAPGQGLEWIGMIHPKSDNIKYNEKFKNKATLTVDR STSTAYMELSSLRSEDTAVYYCARWNLISTVGRYLDVW GQGTTVTVSS |
| SEQ ID NO: 27 | mAb-013-135A9B10-hVL0 (humanized) | DIQMTQSPSSLSASVGDRVTITCKASQNVGTAVGWYQQ KPGKAPKLLIYSTSNRYSGVPSRFSGSGSGTDFTLTISSL QPEDFATYYCQQYSSFPLTFGQGTKLELK |
| SEQ ID NO: 28 | mAb-013-135A9B10-hVL1 (humanized) | DIQMTQSPSSLSASVGDRVTITCKASQNVGTAVGWYQQ KPGKAPKLLIYSTSNRYSGVPSRFSGSGSGTDFTLTISSL QPEDFATYFCQQYSSFPLTFGQGTKLELK |
| SEQ ID NO: 29 | 137G3D11 HCDR1 | DYYMN |
| SEQ ID NO: 30 | 137G3D11 HCDR2 | NINPNNGVTTYNQRFKG |
| SEQ ID NO: 31 | 137G3D11 HCDR3 | YYYGSSYYVYNSAMDY |
| SEQ ID NO: 32 | 137G3D11 LCDR1 | SASSSVSYMH |
| SEQ ID NO: 33 | 137G3D11 LCDR2 | DTSKLAS |
| SEQ ID NO: 34 | 137G3D11 LCDR3 | HQWSSDPLT |
| SEQ ID NO: 35 | 137G3D11 VH (murine) | EVQLQQSGPELVKPGASVKISCKASGFTFTDYYMNWV KQSHGKSLEWIGNINPNNGVTTYNQRFKGKATLTVDKS STTACMELRSLTSEDSAVYYCASYYYGSSYYVYNSAMD YWGQGTSVTVSS |
| SEQ ID NO: 36 | 137G3D11 VL (murine) | QIVLTQSPAIMSASPGEKVTMTCSASSSVSYMHWYQQK SGTSPKRWIYDTSKLASGVPARFSGSGSGTSYSLTLSSM EAEDAATYYCHQWSSDPLTFGAGTKLELK |
| SEQ ID NO: 37 | mAb-013-137G3D11-hVH0 (humanized) | QVQLVQSGAEVKKPGASVKVSCKASGYTFTDYYMNW VRQAPGQGLEWMGNINPNNGVTTYNQRFKGRVTMTR DTSTSTVYMELSSLRSEDTAVYYCARYYYGSSYYVYNS AMDYWGQGTTVTVSS |
| SEQ ID NO: 38 | mAb-013-137G3D11-hVH1 (humanized) | QVQLVQSGAEVKKPGASVKVSCKASGYTFTDYYMNW VRQAPGQGLEWMGNINPNNGVTTYNQRFKGRVTMTV DKSTSTVYMELSSLRSEDTAVYYCASYYYGSSYYVYNS AMDYWGQGTTVTVSS |
| SEQ ID NO: 39 | mAb-013-137G3D11-hVH2 (humanized) | QVQLVQSGAEVKKPGASVKVSCKASGFTFTDYYMNW VRQAPGQGLEWIGNINPNNGVTTYNQRFKGRATLTVD KSTSTVYMELSSLRSEDTAVYYCASYYYGSSYYVYNSA MDYWGQGTTVTVSS |
| SEQ ID NO: 40 | mAb-013-137G3D11-hVH3 (humanized) | QVQLVQSGAEVKKPGASVKVSCKASGFTFTDYYMNW VRQAPGQGLEWMGNINPNNGVTTYNQRFKGRVTMTV DKSTTTAYMELSSLRSEDTAVYYCASYYYGSSYYVYNS AMDYWGQGTTVTVSS |
| SEQ ID NO: 41 | mAb-013-137G3D11-hVH4 (humanized) | QVQLVQSGAEVKKPGASVKVSCKASGFTFTDYYMNW VRQAPGQGLEWIGNINPNNGVTTYNQRFKGRATLTVD KSTTTAYMELSSLRSEDTAVYYCASYYYGSSYYVYNSA MDYWGQGTTVTVSS |
| SEQ ID NO: 42 | mAb-013-137G3D11-hVL0 (humanized) | EIVLTQSPATLSLSPGERATLSCSASSSVSYMHWYQQKP GQAPRLLIYDTSKLASGIPARFSGSGSGTDFTLTISSLEPE DFAVYYCHQWSSDPLTFGQGTKLELK |
| SEQ ID NO: 43 | mAb-013-137G3D11-hVL1 (humanized) | EIVLTQSPATLSLSPGERATLSCSASSSVSYMHWYQQKP GQAPRRWIYDTSKLASGIPARFSGSGSGTDFTLTISSLEP EDFAVYYCHQWSSDPLTFGQGTKLELK |
| SEQ ID NO: 44 | mAb-013-137G3D11-hVL2 (humanized) | EIVLTQSPATLSLSPGERATLSCSASSSVSYMHWYQQKP GQAPRRWIYDTSKLASGVPARFSGSGSGTDYTLTISSLEP EDFAVYYCHQWSSDPLTFGQGTKLELK |
| SEQ ID NO: 45 | IgG1/K heavy chain constant region | |
| SEQ ID NO: 46 | IgG1/K light chain constant region | |

### Humanized anti-DLL3 antibody heavy chain and light chain combinations

| | |
|---|---|
| hz177D10F11-H1L1 | Heavy chain: |
| | QVQLVQSGSELKKPGASVKVSCKASGYTFTTYGMSWVRQAPGQGLEWMG WINTYSGVPTYADDFKGRFVFSLDTSVSTAYLQISSLKAEDTAVYYCARDPIA MDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSR WQQGNVFSCSVMHEALHNHYTQKSLSLSPGK (SEQ ID NO: 47) |
| | Light chain: |
| | EIVLTQSPATLSLSPGERATLSCRASQSIGTDIHWYQQKPGQAPRLLIYDASESI SGIPARFSGSGSGTDFTLTISSLEPEDFAVYYCQQSNSWPTLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 48) |
| hz177D10F11-H1L2 | Heavy chain: |
| | QVQLVQSGSELKKPGASVKVSCKASGYTFTTYGMSWVRQAPGQGLEWMG WINTYSGVPTYADDFKGRFVFSLDTSVSTAYLQISSLKAEDTAVYYCARDPIA MDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSR WQQGNVFSCSVMHEALHNHYTQKSLSLSPGK (SEQ ID NO: 47) |
| | Light chain: |
| | EIVVTQSPATLSLSPGERATLSCRASQSIGTDIHWYQQKPGQAPRLLIYDASESI SGIPARFSGSGSGTDFTLTISSLEPEDFAVYYCQQSNSWPTLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 49) |
| hz177D10F11-H1L3 | Heavy chain: |
| | QVQLVQSGSELKKPGASVKVSCKASGYTFTTYGMSWVRQAPGQGLEWMG WINTYSGVPTYADDFKGRFVFSLDTSVSTAYLQISSLKAEDTAVYYCARDPIA MDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSR WQQGNVFSCSVMHEALHNHYTQKSLSLSPGK (SEQ ID NO: 47) |
| | Light chain: |
| | EIVLTQSPATLSLSPGERATLSCRASQSIGTDIHWYQQKPGQAPRLLIKDASESI SGIPARFSGSGSGTDFTLTISSLEPEDFAVYYCQQSNSWPTLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 50) |
| hz177D10F11-H2L1 | Heavy chain: |
| | QIQLVQSGSELKKPGASVKVSCKASGYTFTTYGMSWVRQAPGQGLEWMGW INTYSGVPTYADDFKGRFVFSLDTSVSTAYLQISSLKAEDTAVYYCARDPIAM |
| | DYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVS LTCLVKGFYPSDIAVEWESNGQPENNYKTTPPVLDSDGSFFLYSKLTVDKSRW QQGNVFSCSVMHEALHNHYTQKSLSLSPGK (SEQ ID NO: 51) |
| | Light chain: |
| | EIVLTQSPATLSLSPGERATLSCRASQSIGTDIHWYQQKPGQAPRLLIYDASESI SGIPARFSGSGSGTDFTLTISSLEPEDFAVYYCQQSNSWPTLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 48) |
| hz177D10F11-H2L2 | Heavy chain: |
| | QIQLVQSGSELKKPGASVKVSCKASGYTFTTYGMSWVRQAPGQGLEWMGW INTYSGVPTYADDFKGRFVFSLDTSVSTAYLQISSLKAEDTAVYYCARDPIAM DYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVS LTCLVKGFYPSDIAVEWESNGQPENNYKTTPPVLDSDGSFFLYSKLTVDKSRW QQGNVFSCSVMHEALHNHYTQKSLSLSPGK (SEQ ID NO: 51) |
| | Light chain: |
| | EIVVTQSPATLSLSPGERATLSCRASQSIGTDIHWYQQKPGQAPRLLIYDASESI SGIPARFSGSGSGTDFTLTISSLEPEDFAVYYCQQSNSWPTLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 49) |
| hz177D10F11-H2L3 | Heavy chain: |
| | QIQLVQSGSELKKPGASVKVSCKASGYTFTTYGMSWVRQAPGQGLEWMGW INTYSGVPTYADDFKGRFVFSLDTSVSTAYLQISSLKAEDTAVYYCARDPIAM DYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVS LTCLVKGFYPSDIAVEWESNGQPENNYKTTPPVLDSDGSFFLYSKLTVDKSRW QQGNVFSCSVMHEALHNHYTQKSLSLSPGK (SEQ ID NO: 51) |
| | Light chain: |
| | EIVLTQSPATLSLSPGERATLSCRASQSIGTDIHWYQQKPGQAPRLLIKDASESI SGIPARFSGSGSGTDFTLTISSLEPEDFAVYYCQQSNSWPTLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 50) |
| hz177D10F11-H3L1 | Heavy chain: |
| | QVQLVQSGSELKKPGASVKVSCKASGYTFTTYGMSWVRQAPGQGLEWMG WINTYSGVPTYADDFKGRFVFSLDTSVSTAYLQISSLKAEDTAVYFCARDPIA MDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSR |
| | WQQGNVFSCSVMHEALHNHYTQKSLSLSPGK (SEQ ID NO: 52) |
| | Light chain: |
| | EIVLTQSPATLSLSPGERATLSCRASQSIGTDIHWYQQKPGQAPRLLIYDASESI SGIPARFSGSGSGTDFTLTISSLEPEDFAVYYCQQSNSWPTLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 48) |
| hz177D10F11-H3L2 | Heavy chain: |
| | QVQLVQSGSELKKPGASVKVSCKASGYTFTTYGMSWVRQAPGQGLEWMG WINTYSGVPTYADDFKGRFVFSLDTSVSTAYLQISSLKAEDTAVYFCARDPIA MDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSR WQQGNVFSCSVMHEALHNHYTQKSLSLSPGK (SEQ ID NO: 52) |
| | Light chain: |
| | EIVVTQSPATLSLSPGERATLSCRASQSIGTDIHWYQQKPGQAPRLLIYDASESI SGIPARFSGSGSGTDFTLTISSLEPEDFAVYYCQQSNSWPTLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 49) |
| hz177D10F11-H3L3 | Heavy chain: |
| | QVQLVQSGSELKKPGASVKVSCKASGYTFTTYGMSWVRQAPGQGLEWMG WINTYSGVPTYADDFKGRFVFSLDTSVSTAYLQISSLKAEDTAVYFCARDPIA MDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSR WQQGNVFSCSVMHEALHNHYTQKSLSLSPGK (SEQ ID NO: 52) |
| | Light chain: |
| | EIVLTQSPATLSLSPGERATLSCRASQSIGTDIHWYQQKPGQAPRLLIKDASESI SGIPARFSGSGSGTDFTLTISSLEPEDFAVYYCQQSNSWPTLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 50) |
| hz135A9B10-H0-L1 | Heavy chain: |
| | QVQLVQSGAEVKKPGASVKVSCKASGYTFTNYWLHWVRQAPGQGLEWMG MIHPKSDNIKYNEKFKNRVTMTRDTSTSTVYMELSSLRSEDTAVYYCARWNL ISTVGRYLDVWGQGTTVTVSSASTKGPSVFPLAPSSKSTSGGTAALGCLVKD YFPEPVTVSWNSGALTSGVHTFPAVLQSSGLYSLSSVVTVPSSSLGTQTYICNV NHKPSNTKVDKKVEPKSCDKTHTCPPCPAPELLGGPSVFLFPPKPKDTLMISR TPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKL TVDKSRWQQGNVFSCSVMHEALHNHYTQKSLSLSPGK (SEQ ID NO: 53) |
| | Light chain: |
| | DIQMTQSPSSLSASVGDRVTITCKASQNVGTAVGWYQQKPGKAPKLLIYSTS NRYSGVPSRFSGSGSGTDFTLTISSLQPEDFATYFCQQYSSFPLTFGQGTKLEL KRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR |
| | GEC (SEQ ID NO: 54) |
| hz137G3D11-H3-L2 | Heavy chain: |
| | QVQLVQSGAEVKKPGASVKVSCKASGFTFTDYYMNWVRQAPGQGLEWMG NINPNNGVTTYNQRFKGRVTMTVDKSTTTAYMELSSLRSEDTAVYYCASYY YGSSYYVVYNSAMDYWGQGTTVTVSSASTKGPSVFPLAPSSKSTSGGTAALG CLVKDYFPEPVTVSWNSGALTSGVHTFPAVLQSSGLYSLSSVVTVPSSSLGTQ TYICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFF LYSKLTVDKSRWQQGNVFSCSVMHEALHNHYTQKSLSLSPGK (SEQ ID NO: 55) |
| | Light chain: |
| | EIVLTQSPATLSLSPGERATLSCSASSSVSYMHWYQQKPGQAPRRWIYDTSKL ASGVPARFSGSGSGTDYTLTISSLEPEDFAVYYCHQWSSDPLTFGQGTKLELK RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNR GEC (SEQ ID NO: 56) |
| hz137G3D11-H4-L1 | Heavy chain: |
| | QVQLVQSGAEVKKPGASVKVSCKASGFTFTDYYMNWVRQAPGQGLEWIGN INPNNGVTTYNQRFKGRATLTVDKSTTTAYMELSSLRSEDTAVYYCASYYYG SSYYVYNSAMDYWGQGTTVTVSSASTKGPSVFPLAPSSKSTSGGTAALGCLV KDYFPEPVTVSWNSGALTSGVHTFPAVLQSSGLYSLSSVVTVPSSSLGTQTYIC NVNHKPSNTKVDKKVEPKSCDKTHTCPPCPAPELLGGPSVFLFPPKPKDTLMI SRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYSK LTVDKSRWQQGNVFSCSVMHEALHNHYTQKSLSLSPGK (SEQ ID NO: 57) Light chain: EIVLTQSPATLSLSPGERATLSCSASSSVSYMHWYQQKPGQAPRRWIYDTSKL ASGIPARFSGSGSGTDFTLTISSLEPEDFAVYYCHQWSSDPLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 58) |
| | Light chain: |
| | |
| hz137G3D11-H3-L1 | Heavy chain: |
| | QVQLVQSGAEVKKPGASVKVSCKASGFTFTDYYMNWVRQAPGQGLEWMG NINPNNGVTTYNQRFKGRVTMTVDKSTTTAYMELSSLRSEDTAVYYCASYY YGSSYYVVYNSAMDYWGQGTTVTVSSASTKGPSVFPLAPSSKSTSGGTAALG CLVKDYFPEPVTVSWNSGALTSGVHTFPAVLQSSGLYSLSSVVTVPSSSLGTQ TYICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFF LYSKLTVDKSRWQQGNVFSCSVMHEALHNHYTQKSLSLSPGK (SEQ ID NO: 55) |
| | Light chain: |
| | EIVLTQSPATLSLSPGERATLSCSASSSVSYMHWYQQKPGQAPRRWIYDTSKL ASGIPARFSGSGSGTDFTLTISSLEPEDFAVYYCHQWSSDPLTFGQGTKLELKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO: 58) |

Full-length sequences of Rovalpituzumab and Rovalpituzumab-mIgG

| | |
|---|---|
| Rovalpituzumab | Heavy chain: |
| | QVQLVQSGAEVKKPGASVKVSCKASGYTFTNYGMNWVRQAPGQGLE WMGWINTYTGEPTYADDFKGRVTMTTDTSTSTAYMELRSLRSDDTAVY YCARIGDSSPSDYWGQGTLVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYT QKSLSLSPG (SEQ ID NO: 59) |
| | Light chain: |
| | EIVMTQSPATLSVSPGERATLSCKASQSVSNDVVWYQQKPGQAPRLLIY YASNRYTGIPARFSGSGSGTEFTLTISSLQSEDFAVYYCQQDYTSPWTFGQ GTKLEIKRTVAAPSVFIFPPSDEQLKSGTASVVCLLNNFYPREAKVQWKV DNALQSGNSQESVTEQDSKDSTYSLSSTLTLSKADYEKHKVYACEVTHQ GLSSPVTKSFNRGEC (SEQ ID NO: 60) |
| Rovalpituzumab-mIgG (A murine chimeric antibody was constructed by grafting the publicly reported variable regions of Rovalpituzumab onto murine constant regions) | Heavy chain: |
| | QVQLVQSGAEVKKPGASVKVSCKASGYTFTNYGMNWVRQAPGQGLE WMGWINTYTGEPTYADDFKGRVTMTTDTSTSTAYMELRSLRSDDTAVY YCARIGDSSPSDYWGQGTLVTVSSAKTTAPSVYPLAPVCGDTTGSSVTL GCLVKGYFPEPVTLTWNSGSLSSGVHTFPAVLQSDLYTLSSSVTVTSSTW PSQSITCNVAHPASSTKVDKKIEPRGPTIKPCPPCKCPAPNLLGGPSVFIFP PKIKDVLMISLSPIVTCVVVDVSEDDPDVQISWFVNNVEVHTAQTQTHR EDYNSTLRVVSALPIQHQDWMSGKEFKCKVNNKDLPAPIERTISKPKGS VRAPQVYVLPPPEEEMTKKQVTLTCMVTDFMPEDIYVEWTNNGKTELN YKNTEPVLDSDGSYFMYSKLRVEKKNWVERNSYSCSVVHEGLHNHHT TKSFSRTPGK (SEQ ID NO: 61) |
| | Light chain: |
| | EIVMTQSPATLSVSPGERATLSCKASQSVSNDVVWYQQKPGQAPRLLIY YASNRYTGIPARFSGSGSGTEFTLTISSLQSEDFAVYYCQQDYTSPWTFGQ GTKLEIKRADAAPTVSIFPPSSEQLTSGGASVVCFLNNFYPKDINVKWKI DGSERQNGVLNSWTDQDSKDSTYSMSSTLTLTKDEYERHNSYTCEATH KTSTSPIVKSFNRNEC (SEQ ID NO: 62) |

## Claims

1. An antibody or an antigen-binding fragment thereof that binds to DLL3, comprising heavy chain complementarity determining regions HCDR1, HCDR2, and HCDR3, and/or light chain complementarity determining regions LCDR1, LCDR2, and LCDR3, wherein:
1) the HCDR1 comprises the sequence as shown in SEQ ID NO: 1 or a sequence comprising one or more amino acid substitutions, deletions, or insertions, or any combination thereof relative to SEQ ID NO: 1, or consists of SEQ ID NO: 1; the HCDR2 comprises the sequence as shown in SEQ ID NO: 2 or a sequence comprising one or more amino acid substitutions, deletions, or insertions, or any combination thereof relative to SEQ ID NO: 2, or consists of SEQ ID NO: 2; the HCDR3 comprises the sequence as shown in SEQ ID NO: 3 or a sequence comprising one or more amino acid substitutions, deletions, or insertions, or any combination thereof relative to SEQ ID NO: 3, or consists of SEQ ID NO: 3; the LCDR1 comprises the sequence as shown in SEQ ID NO: 4 or a sequence comprising one or more amino acid substitutions, deletions, or insertions, or any combination thereof relative to SEQ ID NO: 4, or consists of SEQ ID NO: 4; the LCDR2 comprises the sequence as shown in SEQ ID NO: 5 or a sequence comprising one or more amino acid substitutions, deletions, or insertions, or any combination thereof relative to SEQ ID NO: 5, or consists of SEQ ID NO: 5; the LCDR3 comprises the sequence as shown in SEQ ID NO: 6 or a sequence comprising one or more amino acid substitutions, deletions, or insertions, or any combination thereof relative to SEQ ID NO: 6, or consists of SEQ ID NO: 6;
2) the HCDR1 comprises the sequence as shown in SEQ ID NO: 15 or a sequence comprising one or more amino acid substitutions, deletions, or insertions, or any combination thereof relative to SEQ ID NO: 15, or consists of SEQ ID NO: 15; the HCDR2 comprises the sequence as shown in SEQ ID NO: 16 or a sequence comprising one or more amino acid substitutions, deletions, or insertions, or any combination thereof relative to SEQ ID NO: 16, or consists of SEQ ID NO: 16; the HCDR3 comprises the sequence as shown in SEQ ID NO: 17 or a sequence comprising one or more amino acid substitutions, deletions, or insertions, or any combination thereof relative to SEQ ID NO: 17, or consists of SEQ ID NO: 17; the LCDR1 comprises the sequence as shown in SEQ ID NO: 18 or a sequence comprising one or more amino acid substitutions, deletions, or insertions, or any combination thereof relative to SEQ ID NO: 18, or consists of SEQ ID NO: 18; the LCDR2 comprises the sequence as shown in SEQ ID NO: 19 or a sequence comprising one or more amino acid substitutions, deletions, or insertions, or any combination thereof relative to SEQ ID NO: 19, or consists of SEQ ID NO: 19; the LCDR3 comprises the sequence as shown in SEQ ID NO: 20 or a sequence comprising one or more amino acid substitutions, deletions, or insertions, or any combination thereof relative to SEQ ID NO: 20, or consists of SEQ ID NO: 20; or
3) the HCDR1 comprises the sequence as shown in SEQ ID NO: 29 or a sequence comprising one or more amino acid substitutions, deletions, or insertions, or any combination thereof relative to SEQ ID NO: 29, or consists of SEQ ID NO: 29; the HCDR2 comprises the sequence as shown in SEQ ID NO: 30 or a sequence comprising one or more amino acid substitutions, deletions, or insertions, or any combination thereof relative to SEQ ID NO: 30, or consists of SEQ ID NO: 30; the HCDR3 comprises the sequence as shown in SEQ ID NO: 31 or a sequence comprising one or more amino acid substitutions, deletions, or insertions, or any combination thereof relative to SEQ ID NO: 31, or consists of SEQ ID NO: 31; the LCDR1 comprises the sequence as shown in SEQ ID NO: 32 or a sequence comprising one or more amino acid substitutions, deletions, or insertions, or any combination thereof relative to SEQ ID NO: 32, or consists of SEQ ID NO: 32; the LCDR2 comprises the sequence as shown in SEQ ID NO: 33 or a sequence comprising one or more amino acid substitutions, deletions, or insertions, or any combination thereof relative to SEQ ID NO: 33, or consists of SEQ ID NO: 33; the LCDR3 comprises the sequence as shown in SEQ ID NO: 34 or a sequence comprising one or more amino acid substitutions, deletions, or insertions, or any combination thereof relative to SEQ ID NO: 34, or consists of SEQ ID NO: 34.

2. The antibody or the antigen-binding fragment thereof that binds to DLL3 according to claim 1, comprising a heavy chain variable region and/or a light chain variable region, wherein:
1) the heavy chain variable region comprises the amino acid sequence as shown in SEQ ID NO: 7, or a sequence comprising one or more amino acid substitutions, deletions, or insertions, or any combination thereof relative to SEQ ID NO: 7, or an amino acid sequence having at least 90% identity to the amino acid sequence of SEQ ID NO: 7, or consists of SEQ ID NO: 7; and/or the light chain variable region comprises the amino acid sequence as shown in SEQ ID NO: 8, or a sequence comprising one or more amino acid substitutions, deletions, or insertions, or any combination thereof relative to SEQ ID NO: 8, or an amino acid sequence having at least 90% identity to the amino acid sequence of SEQ ID NO: 8, or consists of SEQ ID NO: 8;
2) the heavy chain variable region comprises the amino acid sequence as shown in SEQ ID NO: 9, or a sequence comprising one or more amino acid substitutions, deletions, or insertions, or any combination thereof relative to SEQ ID NO: 9, or an amino acid sequence having at least 90% identity to the amino acid sequence of SEQ ID NO: 9, or consists of SEQ ID NO: 9; and/or the light chain variable region comprises the amino acid sequence as shown in SEQ ID NO: 12, or a sequence comprising one or more amino acid substitutions, deletions, or insertions, or any combination thereof relative to SEQ ID NO: 12, or an amino acid sequence having at least 90% identity to the amino acid sequence of SEQ ID NO: 12, or consists of SEQ ID NO: 12;
3) the heavy chain variable region comprises the amino acid sequence as shown in SEQ ID NO: 9, or a sequence comprising one or more amino acid substitutions, deletions, or insertions, or any combination thereof relative to SEQ ID NO: 9, or an amino acid sequence having at least 90% identity to the amino acid sequence of SEQ ID NO: 9, or consists of SEQ ID NO: 9; and/or the light chain variable region comprises the amino acid sequence as shown in SEQ ID NO: 13, or a sequence comprising one or more amino acid substitutions, deletions, or insertions, or any combination thereof relative to SEQ ID NO: 13, or an amino acid sequence having at least 90% identity to the amino acid sequence of SEQ ID NO: 13, or consists of SEQ ID NO: 13;
4) the heavy chain variable region comprises the amino acid sequence as shown in SEQ ID NO: 9, or a sequence comprising one or more amino acid substitutions, deletions, or insertions, or any combination thereof relative to SEQ ID NO: 9, or an amino acid sequence having at least 90% identity to the amino acid sequence of SEQ ID NO: 9, or consists of SEQ ID NO: 9; and/or the light chain variable region comprises the amino acid sequence as shown in SEQ ID NO: 14, or a sequence comprising one or more amino acid substitutions, deletions, or insertions, or any combination thereof relative to SEQ ID NO: 14, or an amino acid sequence having at least 90% identity to the amino acid sequence of SEQ ID NO: 14, or consists of SEQ ID NO: 14;
5) the heavy chain variable region comprises the amino acid sequence as shown in SEQ ID NO: 10, or a sequence comprising one or more amino acid substitutions, deletions, or insertions, or any combination thereof relative to SEQ ID NO: 10, or an amino acid sequence having at least 90% identity to the amino acid sequence of SEQ ID NO: 10, or consists of SEQ ID NO: 10; and/or the light chain variable region comprises the amino acid sequence as shown in SEQ ID NO: 12, or a sequence comprising one or more amino acid substitutions, deletions, or insertions, or any combination thereof relative to SEQ ID NO: 12, or an amino acid sequence having at least 90% identity to the amino acid sequence of SEQ ID NO: 12, or consists of SEQ ID NO: 12;
6) the heavy chain variable region comprises the amino acid sequence as shown in SEQ ID NO: 10, or a sequence comprising one or more amino acid substitutions, deletions, or insertions, or any combination thereof relative to SEQ ID NO: 10, or an amino acid sequence having at least 90% identity to the amino acid sequence of SEQ ID NO: 10, or consists of SEQ ID NO: 10; and/or the light chain variable region comprises the amino acid sequence as shown in SEQ ID NO: 13, or a sequence comprising one or more amino acid substitutions, deletions, or insertions, or any combination thereof relative to SEQ ID NO: 13, or an amino acid sequence having at least 90% identity to the amino acid sequence of SEQ ID NO: 13, or consists of SEQ ID NO: 13;
7) the heavy chain variable region comprises the amino acid sequence as shown in SEQ ID NO: 10, or a sequence comprising one or more amino acid substitutions, deletions, or insertions, or any combination thereof relative to SEQ ID NO: 10, or an amino acid sequence having at least 90% identity to the amino acid sequence of SEQ ID NO: 10, or consists of SEQ ID NO: 10; and/or the light chain variable region comprises the amino acid sequence as shown in SEQ ID NO: 14, or a sequence comprising one or more amino acid substitutions, deletions, or insertions, or any combination thereof relative to SEQ ID NO: 14, or an amino acid sequence having at least 90% identity to the amino acid sequence of SEQ ID NO: 14, or consists of SEQ ID NO: 14;
8) the heavy chain variable region comprises the amino acid sequence as shown in SEQ ID NO: 11, or a sequence comprising one or more amino acid substitutions, deletions, or insertions, or any combination thereof relative to SEQ ID NO: 11, or an amino acid sequence having at least 90% identity to the amino acid sequence of SEQ ID NO: 11, or consists of SEQ ID NO: 11; and/or the light chain variable region comprises the amino acid sequence as shown in SEQ ID NO: 12, or a sequence comprising one or more amino acid substitutions, deletions, or insertions, or any combination thereof relative to SEQ ID NO: 12, or an amino acid sequence having at least 90% identity to the amino acid sequence of SEQ ID NO: 12, or consists of SEQ ID NO: 12;
9) the heavy chain variable region comprises the amino acid sequence as shown in SEQ ID NO: 11, or a sequence comprising one or more amino acid substitutions, deletions, or insertions, or any combination thereof relative to SEQ ID NO: 11, or an amino acid sequence having at least 90% identity to the amino acid sequence of SEQ ID NO: 11, or consists of SEQ ID NO: 11; and/or the light chain variable region comprises the amino acid sequence as shown in SEQ ID NO: 13, or a sequence comprising one or more amino acid substitutions, deletions, or insertions, or any combination thereof relative to SEQ ID NO: 13, or an amino acid sequence having at least 90% identity to the amino acid sequence of SEQ ID NO: 13, or consists of SEQ ID NO: 13;
10) the heavy chain variable region comprises the amino acid sequence as shown in SEQ ID NO: 11, or a sequence comprising one or more amino acid substitutions, deletions, or insertions, or any combination thereof relative to SEQ ID NO: 11, or an amino acid sequence having at least 90% identity to the amino acid sequence of SEQ ID NO: 11, or consists of SEQ ID NO: 11; and/or the light chain variable region comprises the amino acid sequence as shown in SEQ ID NO: 14, or a sequence comprising one or more amino acid substitutions, deletions, or insertions, or any combination thereof relative to SEQ ID NO: 14, or an amino acid sequence having at least 90% identity to the amino acid sequence of SEQ ID NO: 14, or consists of SEQ ID NO: 14;
11) the heavy chain variable region comprises the amino acid sequence as shown in SEQ ID NO: 21, or a sequence comprising one or more amino acid substitutions, deletions, or insertions, or any combination thereof relative to SEQ ID NO: 21, or an amino acid sequence having at least 90% identity to the amino acid sequence of SEQ ID NO: 21, or consists of SEQ ID NO: 21; and/or the light chain variable region comprises the amino acid sequence as shown in SEQ ID NO: 22, or a sequence comprising one or more amino acid substitutions, deletions, or insertions, or any combination thereof relative to SEQ ID NO: 22, or an amino acid sequence having at least 90% identity to the amino acid sequence of SEQ ID NO: 22, or consists of SEQ ID NO: 22;
12) the heavy chain variable region comprises the amino acid sequence as shown in SEQ ID NO: 23, or a sequence comprising one or more amino acid substitutions, deletions, or insertions, or any combination thereof relative to SEQ ID NO: 23, or an amino acid sequence having at least 90% identity to the amino acid sequence of SEQ ID NO: 23, or consists of SEQ ID NO: 23; and/or the light chain variable region comprises the amino acid sequence as shown in SEQ ID NO: 28, or a sequence comprising one or more amino acid substitutions, deletions, or insertions, or any combination thereof relative to SEQ ID NO: 28, or an amino acid sequence having at least 90% identity to the amino acid sequence of SEQ ID NO: 28, or consists of SEQ ID NO: 28;
13) the heavy chain variable region comprises the amino acid sequence as shown in SEQ ID NO: 35, or a sequence comprising one or more amino acid substitutions, deletions, or insertions, or any combination thereof relative to SEQ ID NO: 35, or an amino acid sequence having at least 90% identity to the amino acid sequence of SEQ ID NO: 35, or consists of SEQ ID NO: 35; and/or the light chain variable region comprises the amino acid sequence as shown in SEQ ID NO: 36, or a sequence comprising one or more amino acid substitutions, deletions, or insertions, or any combination thereof relative to SEQ ID NO: 36, or an amino acid sequence having at least 90% identity to the amino acid sequence of SEQ ID NO: 36, or consists of SEQ ID NO: 36;
14) the heavy chain variable region comprises the amino acid sequence as shown in SEQ ID NO: 40, or a sequence comprising one or more amino acid substitutions, deletions, or insertions, or any combination thereof relative to SEQ ID NO: 40, or an amino acid sequence having at least 90% identity to the amino acid sequence of SEQ ID NO: 40, or consists of SEQ ID NO: 40; and/or the light chain variable region comprises the amino acid sequence as shown in SEQ ID NO: 43, or a sequence comprising one or more amino acid substitutions, deletions, or insertions, or any combination thereof relative to SEQ ID NO: 43, or an amino acid sequence having at least 90% identity to the amino acid sequence of SEQ ID NO: 43, or consists of SEQ ID NO: 43;
15) the heavy chain variable region comprises the amino acid sequence as shown in SEQ ID NO: 40, or a sequence comprising one or more amino acid substitutions, deletions, or insertions, or any combination thereof relative to SEQ ID NO: 40, or an amino acid sequence having at least 90% identity to the amino acid sequence of SEQ ID NO: 40, or consists of SEQ ID NO: 40; and/or the light chain variable region comprises the amino acid sequence as shown in SEQ ID NO: 44, or a sequence comprising one or more amino acid substitutions, deletions, or insertions, or any combination thereof relative to SEQ ID NO: 44, or an amino acid sequence having at least 90% identity to the amino acid sequence of SEQ ID NO: 44, or consists of SEQ ID NO: 44; or
16) the heavy chain variable region comprises the amino acid sequence as shown in SEQ ID NO: 41, or a sequence comprising one or more amino acid substitutions, deletions, or insertions, or any combination thereof relative to SEQ ID NO: 41, or an amino acid sequence having at least 90% identity to the amino acid sequence of SEQ ID NO: 41, or consists of SEQ ID NO: 41; and/or the light chain variable region comprises the amino acid sequence as shown in SEQ ID NO: 43, or a sequence comprising one or more amino acid substitutions, deletions, or insertions, or any combination thereof relative to SEQ ID NO: 43, or an amino acid sequence having at least 90% identity to the amino acid sequence of SEQ ID NO: 43, or consists of SEQ ID NO: 43.

3. The antibody or the antigen-binding fragment thereof that binds to DLL3 according to claim 1 or 2, wherein the antibody is in any form, such as a monoclonal antibody, a single-chain antibody, a bifunctional antibody, a single-domain antibody, a nanobody, a fully or partially humanized antibody, or a chimeric antibody; and/or
the antigen-binding fragment is an antigen-binding fragment of an antibody or a half-antibody, such as a Fab, Fab', Fab'-SH, (Fab')2, Fv, scFv, BsFv, dsFv, or (dsFv)2 fragment.

4. The antibody or the antigen-binding fragment thereof that binds to DLL3 according to any one of claims 1-3, wherein the antibody or the antigen-binding fragment thereof further comprises a human or murine constant region, preferably a human or murine heavy chain constant region and/or a human or murine light chain constant region;
more preferably, the antibody or the antigen-binding fragment thereof comprises a heavy chain constant region of IgG, IgA, IgM, IgD, or IgE and/or a κ or λ light chain constant region.

5. The antibody or the antigen-binding fragment thereof that binds to DLL3 according to any one of claims 1-4, wherein the antibody is a monoclonal antibody, preferably a murine, chimeric, or humanized monoclonal antibody; preferably, a heavy chain constant region of the monoclonal antibody is of the IgG1, IgG2, IgG3, or IgG4 subtype;
more preferably, the heavy chain constant region comprises or consists of the sequence as shown in SEQ ID NO: 45; and/or the light chain constant region comprises or consists of the sequence as shown in SEQ ID NO: 46.

6. The antibody or the antigen-binding fragment thereof that binds to DLL3 according to any one of claims 1-5, comprising a heavy chain and/or a light chain, wherein:
1) the heavy chain comprises the amino acid sequence as shown in SEQ ID NO: 47, or a sequence comprising one or more amino acid substitutions, deletions, or insertions, or any combination thereof relative to SEQ ID NO: 47, or an amino acid sequence having at least 90% identity to the amino acid sequence of SEQ ID NO: 47, or consists of SEQ ID NO: 47; the light chain comprises the amino acid sequence as shown in SEQ ID NO: 48, or a sequence comprising one or more amino acid substitutions, deletions, or insertions, or any combination thereof relative to SEQ ID NO: 48, or an amino acid sequence having at least 90% identity to the amino acid sequence of SEQ ID NO: 48, or consists of SEQ ID NO: 48;
2) the heavy chain comprises the amino acid sequence as shown in SEQ ID NO: 47, or a sequence comprising one or more amino acid substitutions, deletions, or insertions, or any combination thereof relative to SEQ ID NO: 47, or an amino acid sequence having at least 90% identity to the amino acid sequence of SEQ ID NO: 47, or consists of SEQ ID NO: 47; the light chain comprises the amino acid sequence as shown in SEQ ID NO: 49, or a sequence comprising one or more amino acid substitutions, deletions, or insertions, or any combination thereof relative to SEQ ID NO: 49, or an amino acid sequence having at least 90% identity to the amino acid sequence of SEQ ID NO: 49, or consists of SEQ ID NO: 49;
3) the heavy chain comprises the amino acid sequence as shown in SEQ ID NO: 47, or a sequence comprising one or more amino acid substitutions, deletions, or insertions, or any combination thereof relative to SEQ ID NO: 47, or an amino acid sequence having at least 90% identity to the amino acid sequence of SEQ ID NO: 47, or consists of SEQ ID NO: 47; the light chain comprises the amino acid sequence as shown in SEQ ID NO: 50, or a sequence comprising one or more amino acid substitutions, deletions, or insertions, or any combination thereof relative to SEQ ID NO: 50, or an amino acid sequence having at least 90% identity to the amino acid sequence of SEQ ID NO: 50, or consists of SEQ ID NO: 50;
4) the heavy chain comprises the amino acid sequence as shown in SEQ ID NO: 51, or a sequence comprising one or more amino acid substitutions, deletions, or insertions, or any combination thereof relative to SEQ ID NO: 51, or an amino acid sequence having at least 90% identity to the amino acid sequence of SEQ ID NO: 51, or consists of SEQ ID NO: 51; the light chain comprises the amino acid sequence as shown in SEQ ID NO: 48, or a sequence comprising one or more amino acid substitutions, deletions, or insertions, or any combination thereof relative to SEQ ID NO: 48, or an amino acid sequence having at least 90% identity to the amino acid sequence of SEQ ID NO: 48, or consists of SEQ ID NO: 48;
5) the heavy chain comprises the amino acid sequence as shown in SEQ ID NO: 51, or a sequence comprising one or more amino acid substitutions, deletions, or insertions, or any combination thereof relative to SEQ ID NO: 51, or an amino acid sequence having at least 90% identity to the amino acid sequence of SEQ ID NO: 51, or consists of SEQ ID NO: 51; the light chain comprises the amino acid sequence as shown in SEQ ID NO: 49, or a sequence comprising one or more amino acid substitutions, deletions, or insertions, or any combination thereof relative to SEQ ID NO: 49, or an amino acid sequence having at least 90% identity to the amino acid sequence of SEQ ID NO: 49, or consists of SEQ ID NO: 49;
6) the heavy chain comprises the amino acid sequence as shown in SEQ ID NO: 51, or a sequence comprising one or more amino acid substitutions, deletions, or insertions, or any combination thereof relative to SEQ ID NO: 51, or an amino acid sequence having at least 90% identity to the amino acid sequence of SEQ ID NO: 51, or consists of SEQ ID NO: 51; the light chain comprises the amino acid sequence as shown in SEQ ID NO: 50, or a sequence comprising one or more amino acid substitutions, deletions, or insertions, or any combination thereof relative to SEQ ID NO: 50, or an amino acid sequence having at least 90% identity to the amino acid sequence of SEQ ID NO: 50, or consists of SEQ ID NO: 50;
7) the heavy chain comprises the amino acid sequence as shown in SEQ ID NO: 52, or a sequence comprising one or more amino acid substitutions, deletions, or insertions, or any combination thereof relative to SEQ ID NO: 52, or an amino acid sequence having at least 90% identity to the amino acid sequence of SEQ ID NO: 52, or consists of SEQ ID NO: 52; the light chain comprises the amino acid sequence as shown in SEQ ID NO: 48, or a sequence comprising one or more amino acid substitutions, deletions, or insertions, or any combination thereof relative to SEQ ID NO: 48, or an amino acid sequence having at least 90% identity to the amino acid sequence of SEQ ID NO: 48, or consists of SEQ ID NO: 48;
8) the heavy chain comprises the amino acid sequence as shown in SEQ ID NO: 52, or a sequence comprising one or more amino acid substitutions, deletions, or insertions, or any combination thereof relative to SEQ ID NO: 52, or an amino acid sequence having at least 90% identity to the amino acid sequence of SEQ ID NO: 52, or consists of SEQ ID NO: 52; the light chain comprises the amino acid sequence as shown in SEQ ID NO: 49, or a sequence comprising one or more amino acid substitutions, deletions, or insertions, or any combination thereof relative to SEQ ID NO: 49, or an amino acid sequence having at least 90% identity to the amino acid sequence of SEQ ID NO: 49, or consists of SEQ ID NO: 49;
9) the heavy chain comprises the amino acid sequence as shown in SEQ ID NO: 52, or a sequence comprising one or more amino acid substitutions, deletions, or insertions, or any combination thereof relative to SEQ ID NO: 52, or an amino acid sequence having at least 90% identity to the amino acid sequence of SEQ ID NO: 52, or consists of SEQ ID NO: 52; the light chain comprises the amino acid sequence as shown in SEQ ID NO: 50, or a sequence comprising one or more amino acid substitutions, deletions, or insertions, or any combination thereof relative to SEQ ID NO: 50, or an amino acid sequence having at least 90% identity to the amino acid sequence of SEQ ID NO: 50, or consists of SEQ ID NO: 50;
10) the heavy chain comprises the amino acid sequence as shown in SEQ ID NO: 53, or a sequence comprising one or more amino acid substitutions, deletions, or insertions, or any combination thereof relative to SEQ ID NO: 53, or an amino acid sequence having at least 90% identity to the amino acid sequence of SEQ ID NO: 53, or consists of SEQ ID NO: 53; the light chain comprises the amino acid sequence as shown in SEQ ID NO: 54, or a sequence comprising one or more amino acid substitutions, deletions, or insertions, or any combination thereof relative to SEQ ID NO: 54, or an amino acid sequence having at least 90% identity to the amino acid sequence of SEQ ID NO: 54, or consists of SEQ ID NO: 54;
11) the heavy chain comprises the amino acid sequence as shown in SEQ ID NO: 55, or a sequence comprising one or more amino acid substitutions, deletions, or insertions, or any combination thereof relative to SEQ ID NO: 55, or an amino acid sequence having at least 90% identity to the amino acid sequence of SEQ ID NO: 55, or consists of SEQ ID NO: 55; the light chain comprises the amino acid sequence as shown in SEQ ID NO: 56, or a sequence comprising one or more amino acid substitutions, deletions, or insertions, or any combination thereof relative to SEQ ID NO: 56, or an amino acid sequence having at least 90% identity to the amino acid sequence of SEQ ID NO: 56, or consists of SEQ ID NO: 56;
12) the heavy chain comprises the amino acid sequence as shown in SEQ ID NO: 57, or a sequence comprising one or more amino acid substitutions, deletions, or insertions, or any combination thereof relative to SEQ ID NO: 57, or an amino acid sequence having at least 90% identity to the amino acid sequence of SEQ ID NO: 57, or consists of SEQ ID NO: 57; the light chain comprises the amino acid sequence as shown in SEQ ID NO: 58, or a sequence comprising one or more amino acid substitutions, deletions, or insertions, or any combination thereof relative to SEQ ID NO: 58, or an amino acid sequence having at least 90% identity to the amino acid sequence of SEQ ID NO: 58, or consists of SEQ ID NO: 58; or
13) the heavy chain comprises the amino acid sequence as shown in SEQ ID NO: 55, or a sequence comprising one or more amino acid substitutions, deletions, or insertions, or any combination thereof relative to SEQ ID NO: 55, or an amino acid sequence having at least 90% identity to the amino acid sequence of SEQ ID NO: 55, or consists of SEQ ID NO: 55; the light chain comprises the amino acid sequence as shown in SEQ ID NO: 58, or a sequence comprising one or more amino acid substitutions, deletions, or insertions, or any combination thereof relative to SEQ ID NO: 58, or an amino acid sequence having at least 90% identity to the amino acid sequence of SEQ ID NO: 58, or consists of SEQ ID NO: 58.

7. A nucleic acid molecule, wherein the nucleic acid molecule encodes the antibody or the antigen-binding fragment thereof according to any one of claims 1-6, or encodes a heavy chain CDR, a light chain CDR, a heavy chain variable region, a light chain variable region, a heavy chain, or a light chain comprised in the antibody or the antigen-binding fragment thereof.

8. A vector, comprising the nucleic acid molecule according to claim 7.

9. A host cell, comprising the nucleic acid molecule according to claim 7 and/or the vector according to claim 8, wherein alternatively, the host cell is transformed or transfected with the nucleic acid molecule according to claim 7 and/or the vector according to claim 8.

10. The host cell according to claim 9, wherein the host cell is prokaryotic or eukaryotic; preferably, the host cell is selected from a yeast cell, a CHO cell, a 293 cell, a plant cell, and another cell suitable for preparing an antibody or an antigen-binding fragment thereof.

11. A method for preparing the antibody or the antigen-binding fragment thereof that binds to DLL3, comprising culturing a host cell that comprises a nucleic acid molecule encoding the antibody or the antigen-binding fragment thereof according to any one of claims 1-6 under conditions suitable for antibody expression, wherein optionally, the method further comprises recovering the antibody or the antigen-binding fragment thereof from the host cell.

12. A conjugate, comprising the antibody or the antigen-binding fragment thereof according to any one of claims 1-6 and a conjugation moiety, wherein the conjugation moiety is another molecule; preferably, the conjugation moiety is a radioisotope, a fluorescent substance, a luminescent substance, a colored substance, or an enzyme.

13. A pharmaceutical composition, comprising the antibody or the antigen-binding fragment thereof according to any one of claims 1-6, the nucleic acid molecule according to claim 7, the vector according to claim 8, the host cell according to claim 9 or 10, or the conjugate according to claim 12, and a pharmaceutically acceptable carrier.

14. The pharmaceutical composition according to claim 13, further comprising a second therapeutic agent and/or an optional pharmaceutical auxiliary material, wherein the second therapeutic agent is selected from a cytokine, an antibody, a chemotherapeutic agent, and a small-molecule drug.

15. An antibody-drug conjugate, comprising the antibody or the antigen-binding fragment thereof according to any one of claims 1-6, and further comprising a cytotoxic drug and a linker.

16. The antibody-drug conjugate according to claim 15, wherein the cytotoxic drug is selected from DX8951, MMAE, and Dxd, and the linker is a cleavable linker.

17. Use of the antibody or the antigen-binding fragment thereof according to any one of claims 1-6, the nucleic acid molecule according to claim 7, the vector according to claim 8, the host cell according to claim 9 or 10, the conjugate according to claim 12, the pharmaceutical composition according to claim 13 or 14, or the antibody-drug conjugate according to claim 15 or 16 in preparing a medicament for treating a disease in a subject.

18. The use according to claim 17, wherein the disease is a DLL3-positive autoimmune disease or a DLL3-positive cancer.

19. The use according to claim 17 or 18, wherein the disease is a neuroendocrine tumor, lung cancer, prostate cancer, melanoma, or gastric cancer.

20. The use according to claim 19, wherein the lung cancer is small cell lung cancer, and the neuroendocrine tumor is castration-resistant neuroendocrine prostate cancer, large cell neuroendocrine carcinoma, or gastroenteropancreatic neuroendocrine neoplasm.

21. The antibody or the antigen-binding fragment thereof according to any one of claims 1-6, the nucleic acid molecule according to claim 7, the vector according to claim 8, the host cell according to claim 9 or 10, the conjugate according to claim 12, the pharmaceutical composition according to claim 13 or 14, or the antibody-drug conjugate according to claim 15 or 16 for use as a medicament or in therapy.

22. A method for preventing and/or treating a disease in a subject, comprising administering to a subject in need thereof an effective amount of the antibody or the antigen-binding fragment thereof according to any one of claims 1-6, the nucleic acid molecule according to claim 7, the vector according to claim 8, the host cell according to claim 9 or 10, the conjugate according to claim 12, the pharmaceutical composition according to claim 13 or 14, or the antibody-drug conjugate according to claim 15 or 16, wherein the disease comprises a tumor or an autoimmune disease;
preferably, the subject is a mammal;
more preferably, the subject is a human.

23. A kit, comprising the antibody or the antigen-binding fragment thereof according to any one of claims 1-6 or the conjugate according to claim 12.

24. A method for detecting the presence or level of DLL3 in a sample, comprising the steps of contacting the antibody or the antigen-binding fragment thereof according to any one of claims 1-6 or the conjugate according to claim 12 with the sample, and detecting whether the antibody or the antigen-binding fragment thereof or the conjugate forms a complex with DLL3.
